(19) Europäisches Patentamt
European Patent Office
Office européen des brevets

(11) Publication number: **0 508 792 A1**

# (12) EUROPEAN PATENT APPLICATION

(21) Application number: **92303187.6**

(22) Date of filing: **09.04.92**

(51) Int. Cl.$^5$: **C07D 498/22,** C07H 19/04, A61K 31/55, // (C07D498/22, 307:00, 273:00, 209:00, 209:00, 209:00)

(30) Priority: **11.04.91 US 683770**

(43) Date of publication of application: **14.10.92 Bulletin 92/42**

(84) Designated Contracting States: **PT**

(71) Applicant: **SCHERING CORPORATION**
**2000 Galloping Hill Road**
**Kenilworth New Jersey 07033 (US)**

(72) Inventor: **McCombie, Stuart W.**
**28 Hanford Place**
**Caldwall, New Jersey 07006 (US)**
Inventor: **Shankar, Bandarpalle B.**
**3405 Wellington Court, Hillsboro**
**Somerville, New Jersey 08876 (US)**
Inventor: **Kirkup, Michael P.**
**753 Cedarbrook Road**
**Bridgewater, New Jersey 08807 (US)**

(74) Representative: **Ritter, Stephen David et al**
**Mathys & Squire 10 Fleet Street**
**London EC4Y 1AY (GB)**

## (54) Anti-tumor and anti-psoriatic agents.

(57) Compounds of formula:

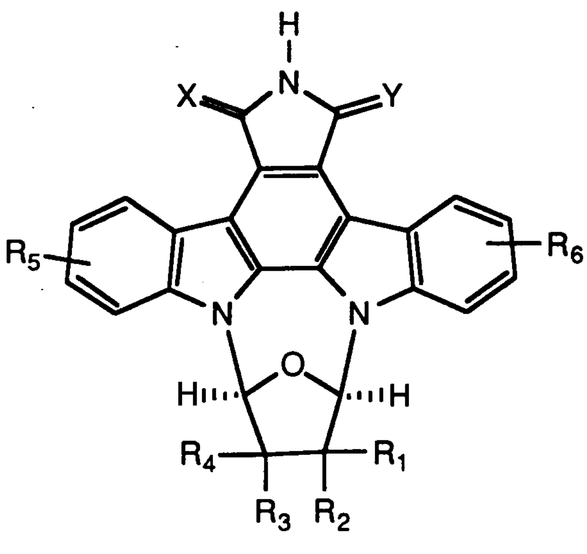

I

or pharmaceutically acceptable salts thereof wherein X, Y, $R_1$, $R_2$, $R_3$, $R_4$, $R_5$, and $R_5$, are as set forth herein are described. These compounds are useful as anti-tumor agents. These compounds are also useful as anti-psoriatic agents.

EP 0 508 792 A1

## SUMMARY OF THE INVENTION

The invention relates to compounds of the formula

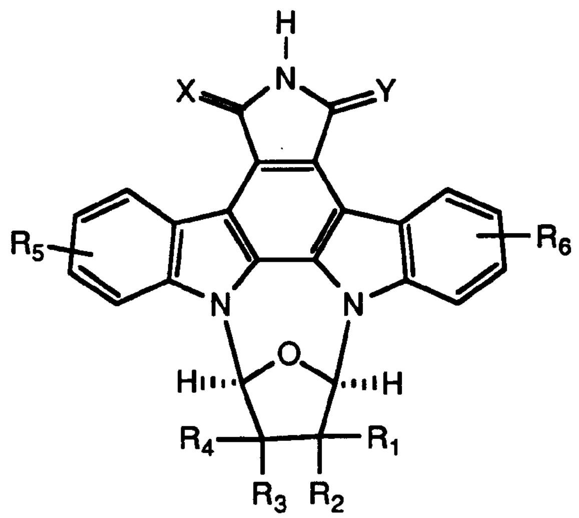

I

or a pharmaceutically acceptable salt thereof, wherein

X is O or S;

Y is O, NH, (H,H), (H,OH) or S; with the proviso that when X is S, Y is not NH, (H,H) or (H,OH);

$R_1$, $R_2$, $R_3$ and $R_4$ may be the same or different and each independently is H, $C_1$-$C_7$ alkyl, $C_1$-$C_7$ substituted alkyl, -CHO, CN, -CON($R_8$,$R_9$), -COO$R_7$, -CH=NO$R_8$, -CH=NNHCON$H_2$, F, Cl, Br, OH, $N_3$, -O$C_1$-$C_7$ alkyl, -O$C_1$-$C_7$ substituted alkyl, -OCO$R_9$, SH, S($C_1$-$C_7$)alkyl, S($C_1$-$C_7$)substituted alkyl, SCO$R_{10}$, S(O)$_n$$R_{11}$, $NH_2$, N($R_{12}$, $R_{13}$), and N ( acyl, $R_{14}$);

$R_1$, and $R_2$ taken together may be O, NOH, NO$R_8$, =$CH_2$ or NNHCON$H_2$;

$R_3$, and $R_4$ taken together may be O, NOH, NO$R_8$, =$CH_2$ or NNHCON$H_2$;

$R_1$ and $R_4$ taken together may be a carbon-carbon bond with the proviso that $R_2$ and $R_3$ may be the same or different and each independently is selected from the group consisting of H, $C_1$-$C_7$ alkyl, $C_1$-$C_7$ substituted alkyl, -CHO, CN, -COO$R_7$, -CON($R_8$, $R_9$), -CH=NO$R_{10}$, and -CHN=NNHCON$H_2$;

$R_8$ and $R_6$ are independently H, $C_1$-$C_7$ alkyl, $C_1$-$C_7$ substituted alkyl, F, Cl, Br, OH, $N_3$, -O$C_1$-$C_7$ alkyl, -O$C_1$-$C_7$ substituted alkyl, -OCO$R_{15}$, SH, S($C_1$-$C_7$)alkyl,S($C_1$-$C_7$) substituted alkyl, SCO$R_{16}$, S(O)$_n$$R_{17}$, $NH_2$, N($R_{18}$,$R_{19}$), and N ( acyl, $R_{20}$);

$R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, and $R_{20}$ may be the same or different and each independently is H alkyl or substituted alkyl; and

n is 1 or 2;

with the proviso that $R_1$ and $R_2$ may not both be directly attached to the 10-position via a heteroatom and $R_3$ and $R_4$ may not both be directly attached to the 11-position via a heteroatom ;

and with the still further proviso that the total number of carbon atoms for those $R_1$, $R_2$, $R_3$, and $R_4$ which are $C_1$-$C_7$ alkyl may not exceed 14;

and with the still further proviso that when X and Y are both O, then $R_1$, $R_2$, $R_3$, $R_4$, $R_5$, and $R_6$ cannot all be H.

Preferred are compounds of formula I wherein X and Y are both O.

Also preferred are compounds of formula I wherein one of $R_1$, $R_2$, $R_3$ or $R_4$ is selected from the group consisting of OH, $N_3$, O$C_1$-$C_7$ alkyl, O$C_1$-$C_7$ substituted alkyl, OCO$R_9$, SH, S($C_1$-$C_7$)alkyl, S(O)$_n$$R_{11}$, $NH_2$, N($R_{12}$,$R_{13}$), and N ( acyl, $R_{14}$).

Most preferred are compounds of formula I wherein $R_1$ is $C_1$-$C_3$ alkyl substituted with OH, and $R_2$ is H; or wherein $R_2$ is $C_1$-$C_3$ alkyl substituted with OH, and $R_1$ is H; and $R_3$, $R_4$, $R_5$, and $R_6$ are H.

Exemplary of compounds of formula I of the invention are

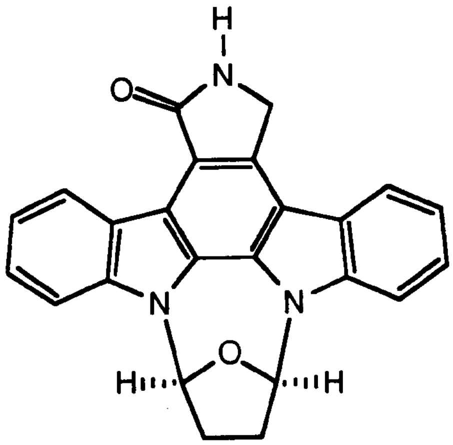

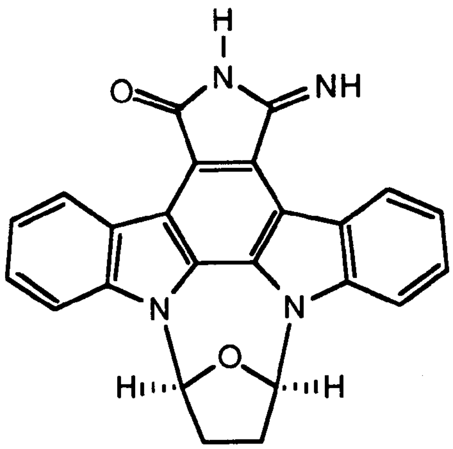

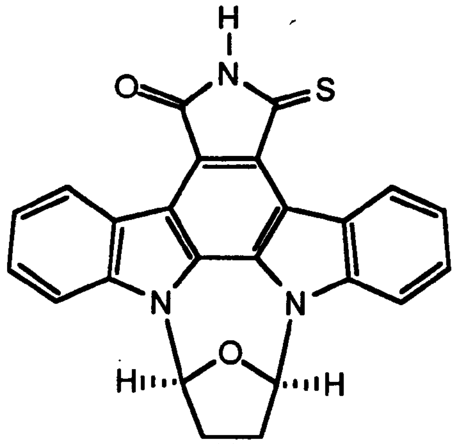

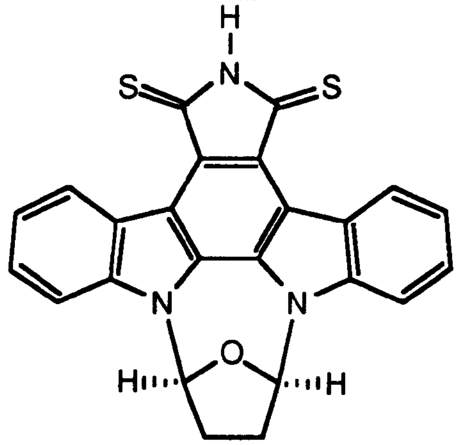

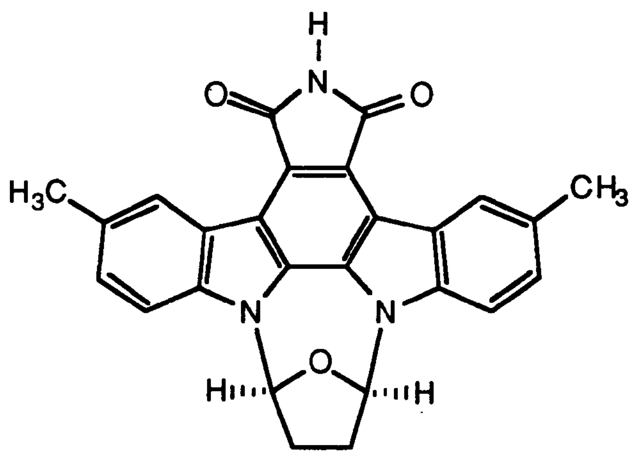

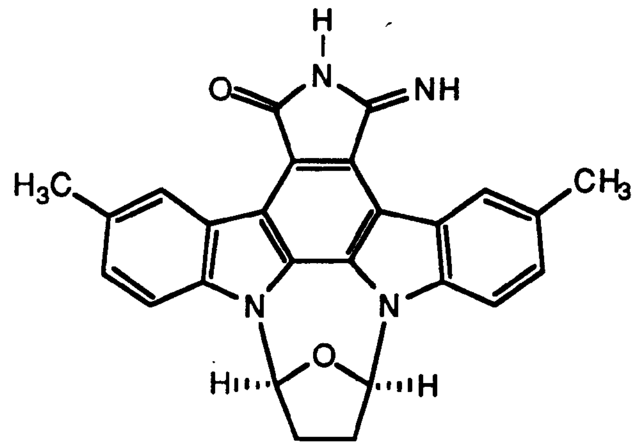

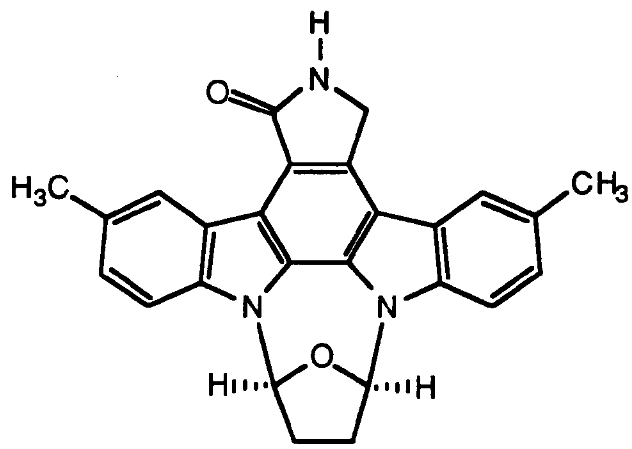

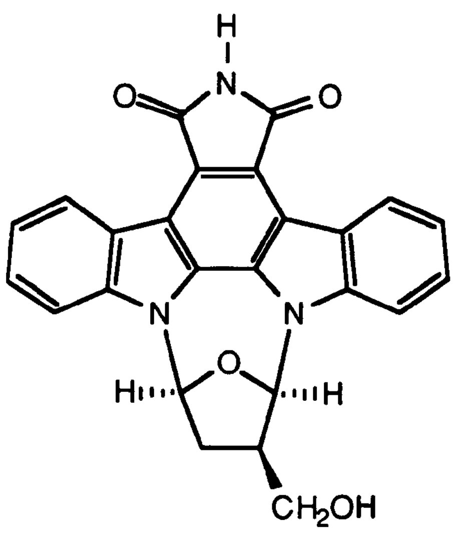

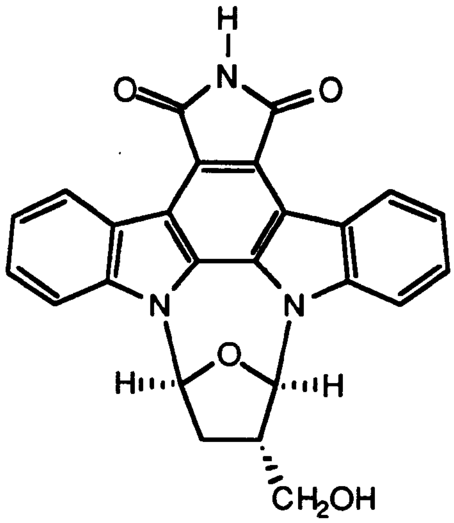

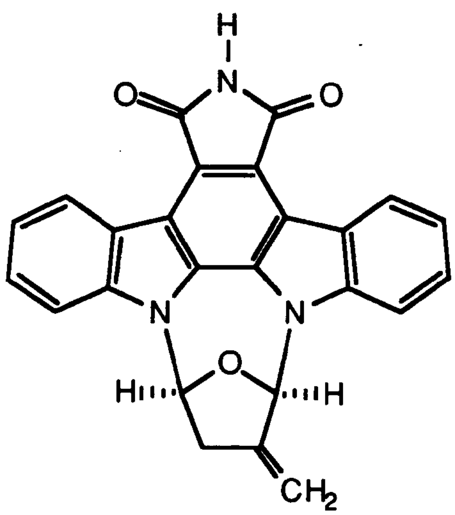

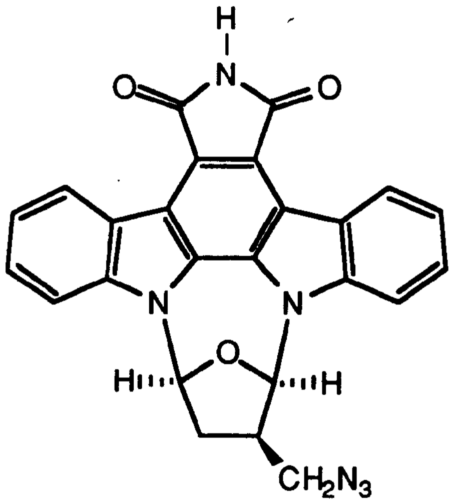

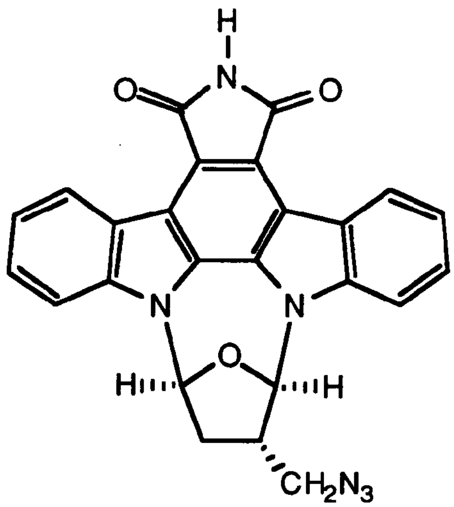

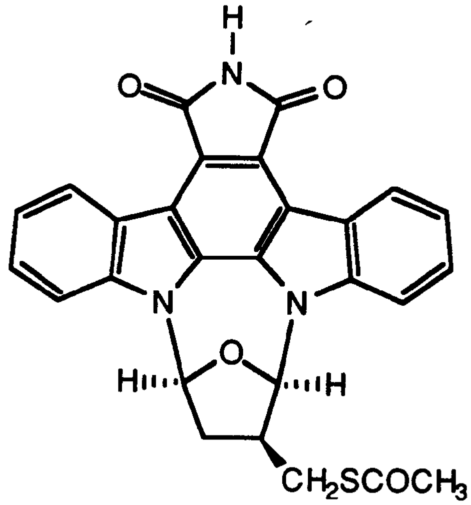

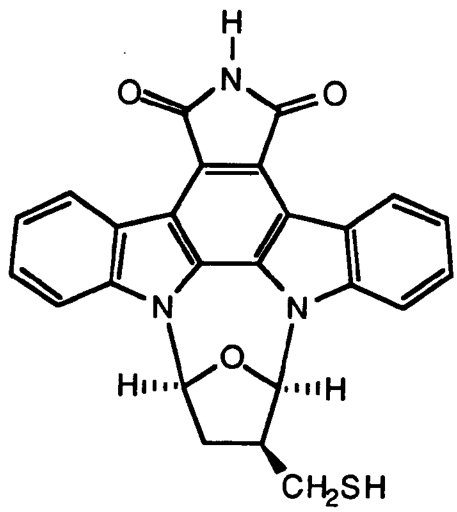

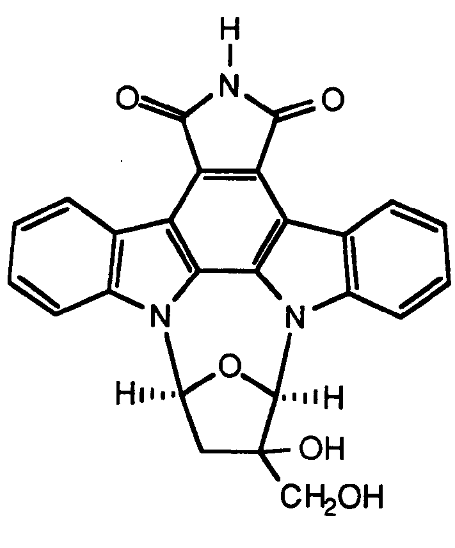

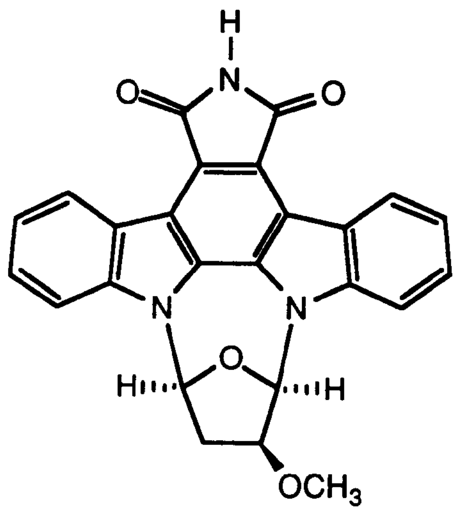

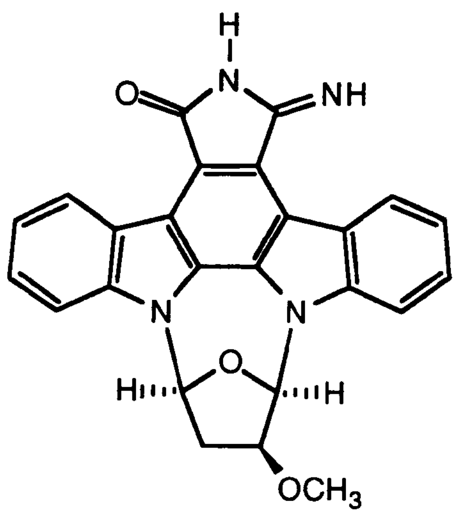

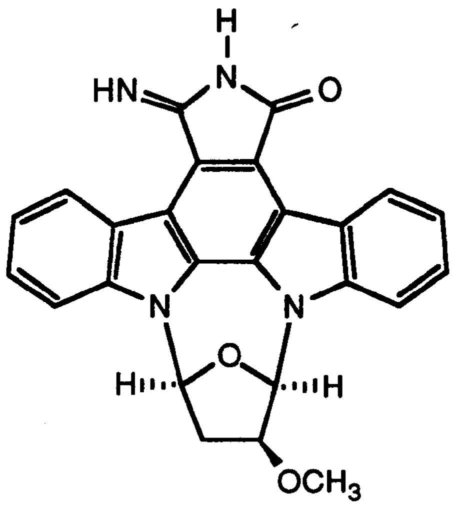

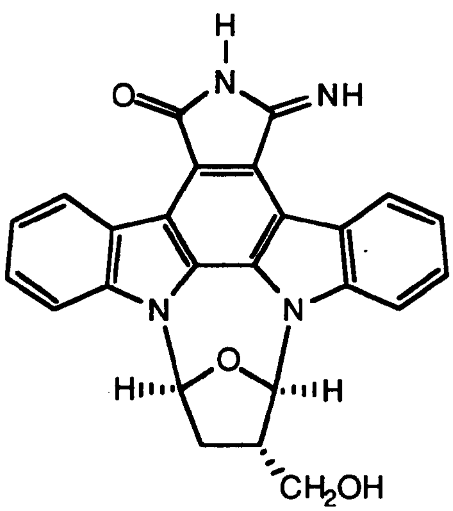

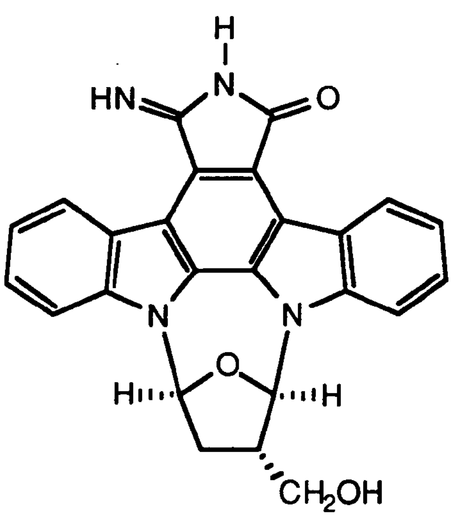

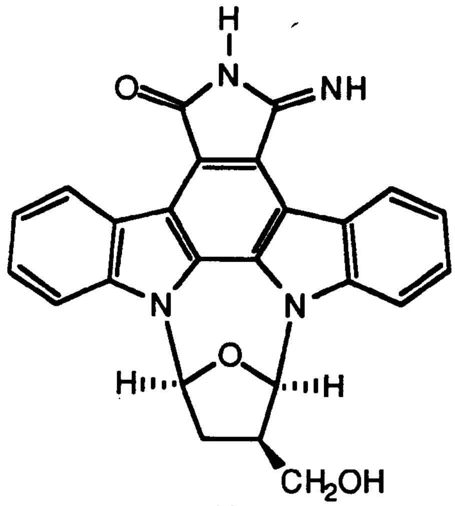

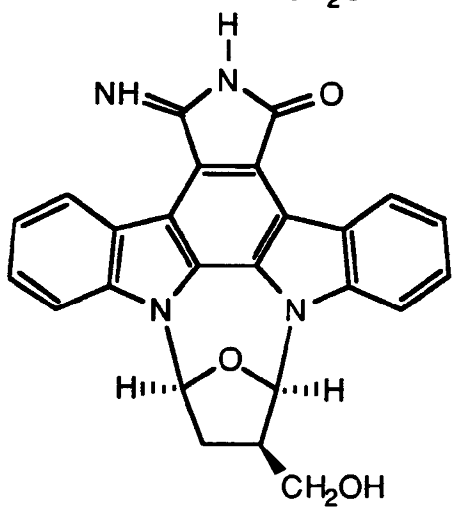

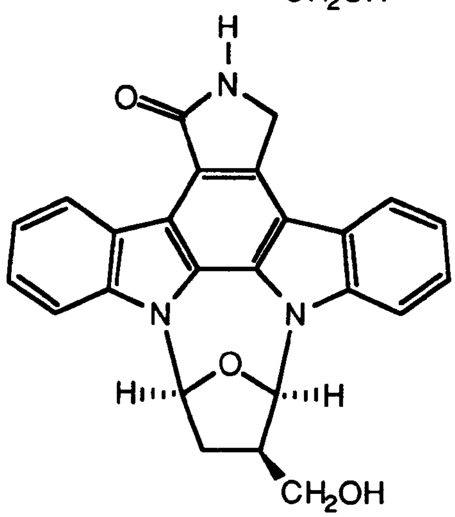

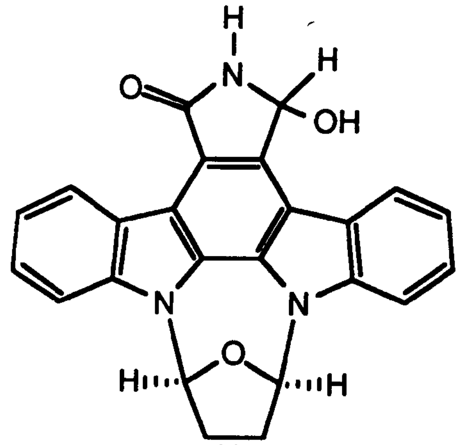

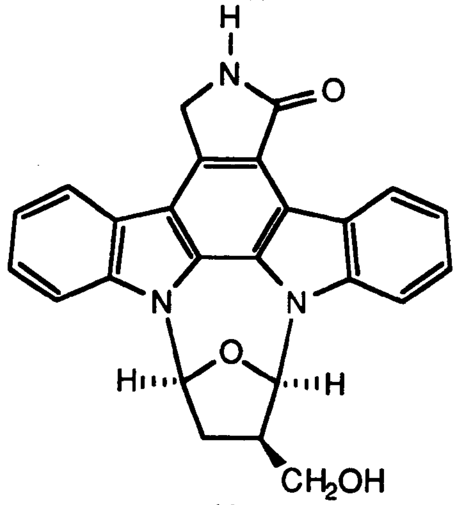

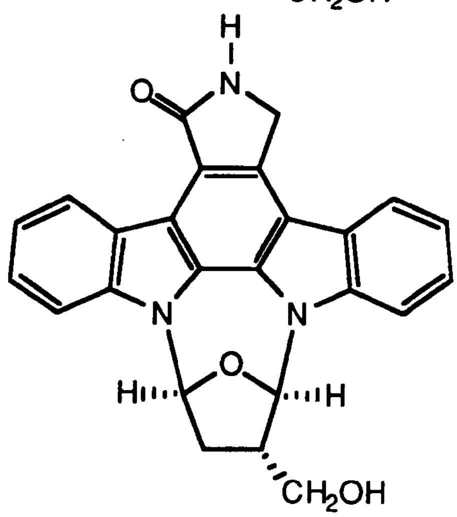

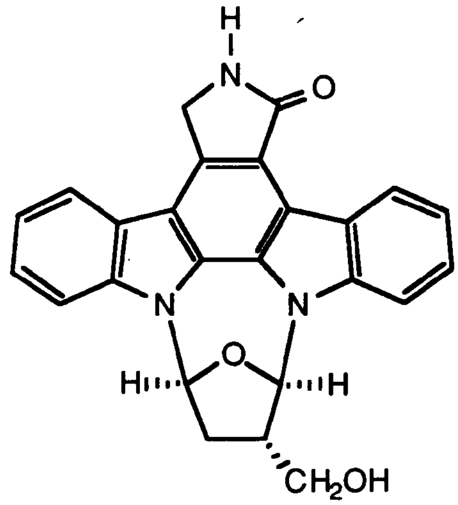

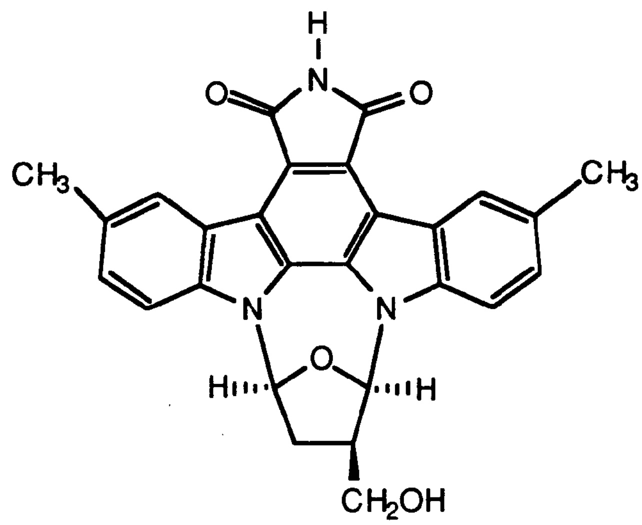

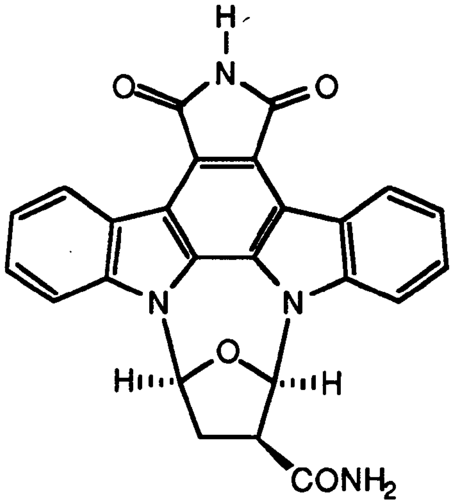

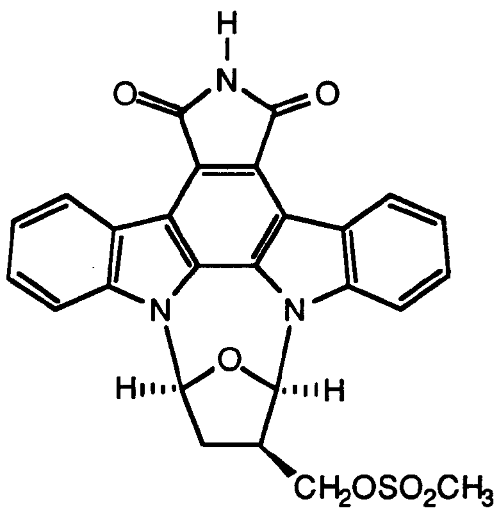

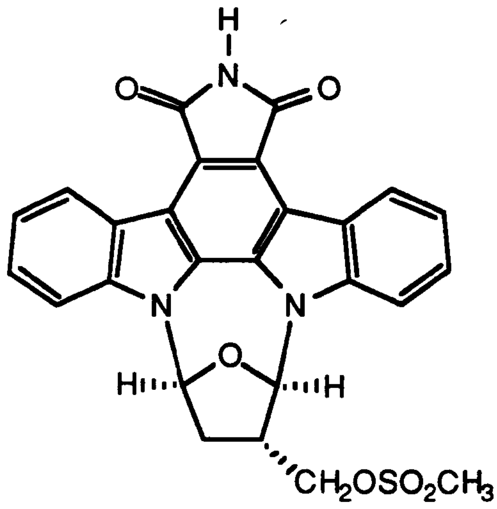

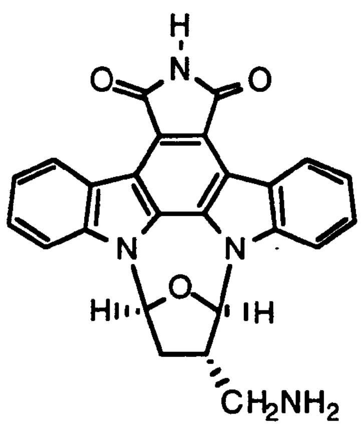

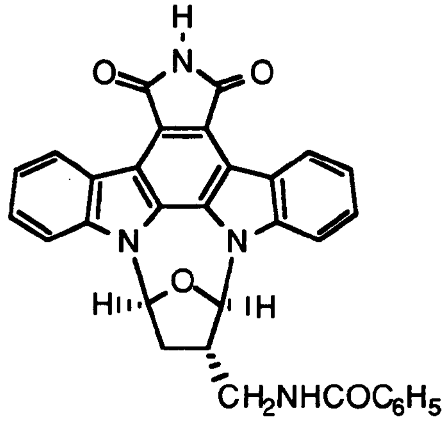

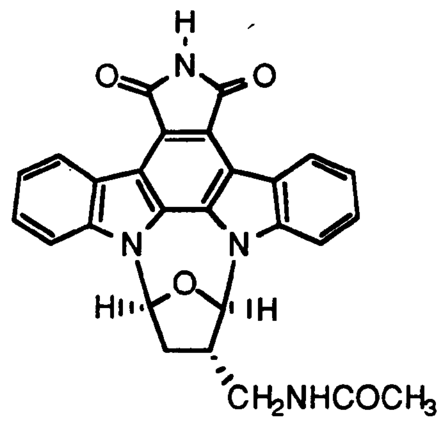

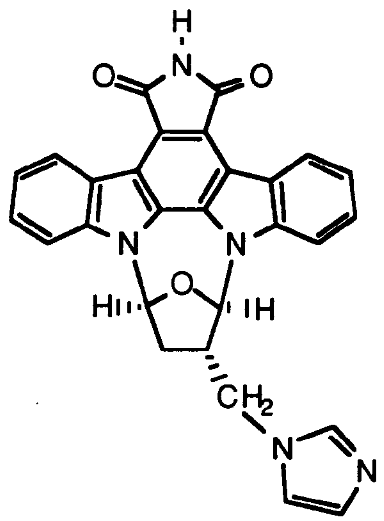

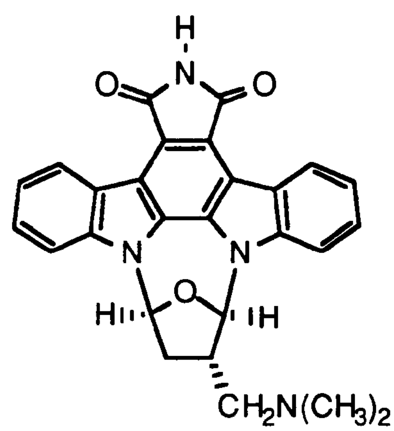

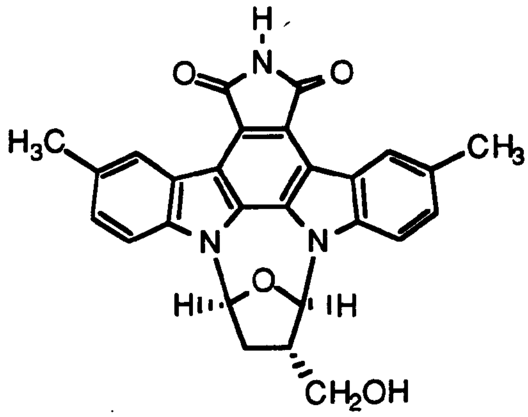

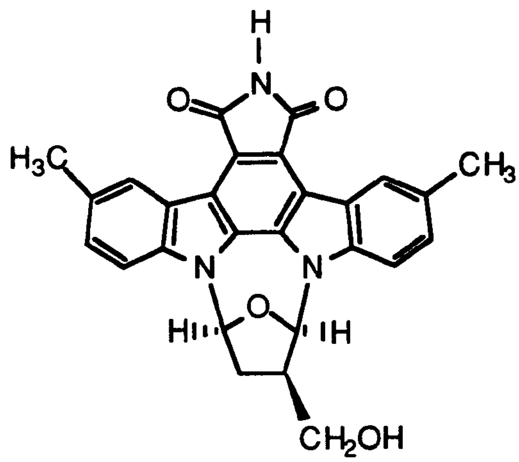

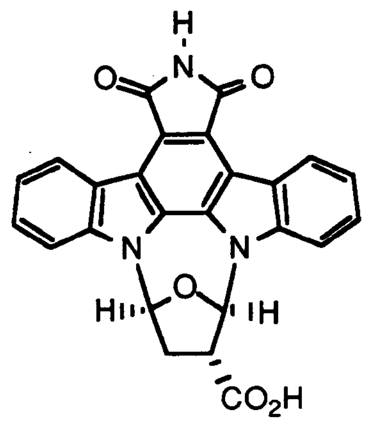

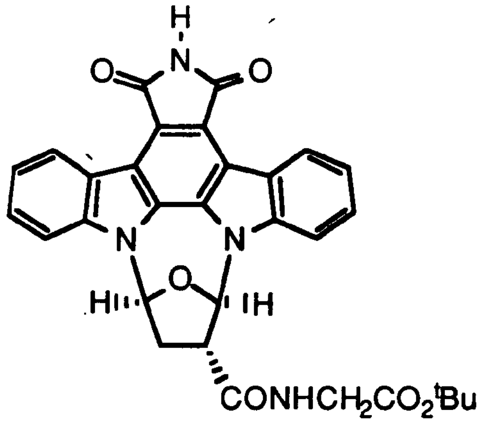

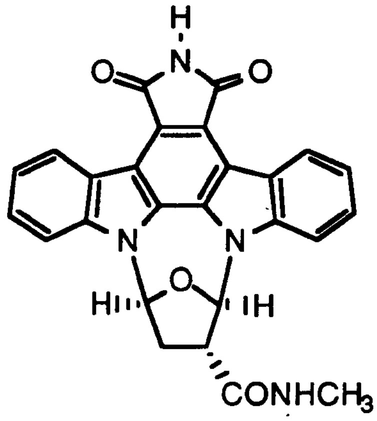

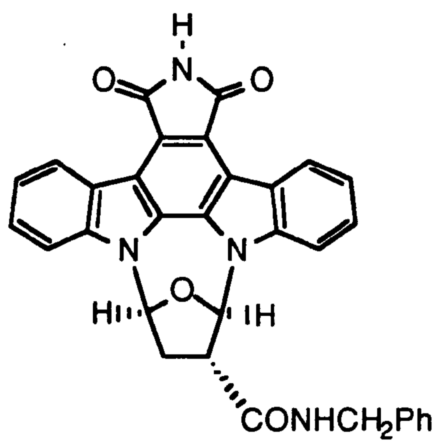

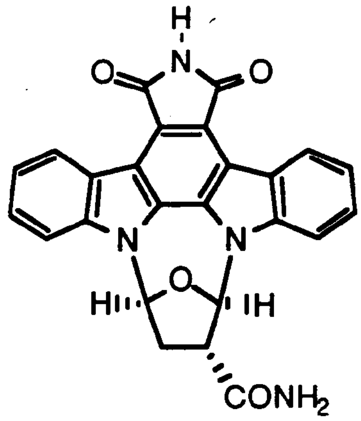

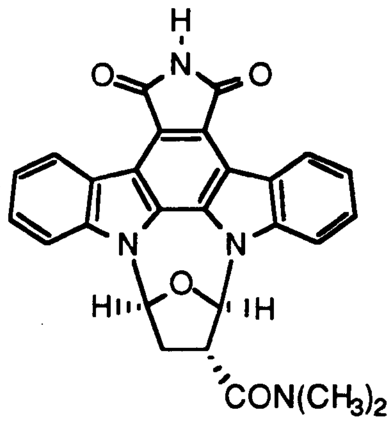

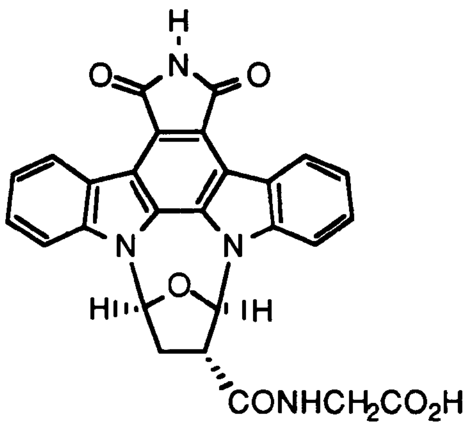

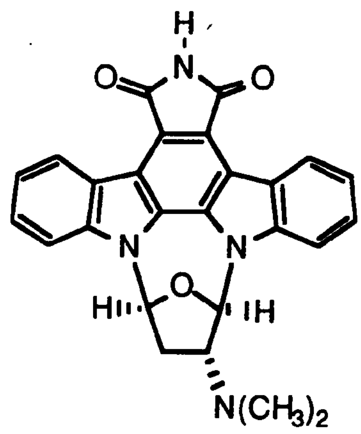

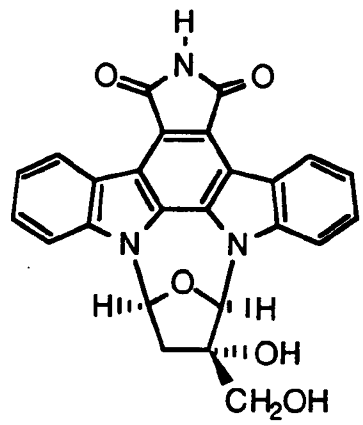

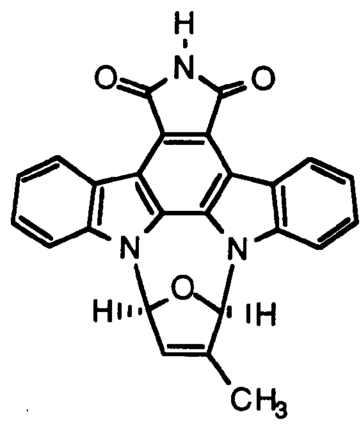

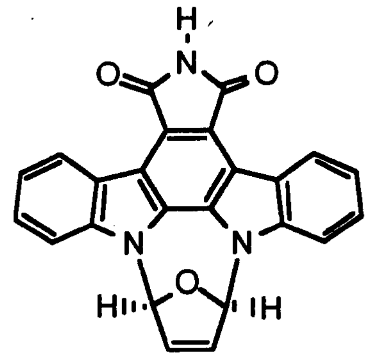

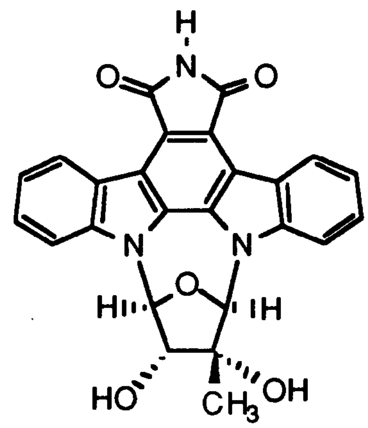

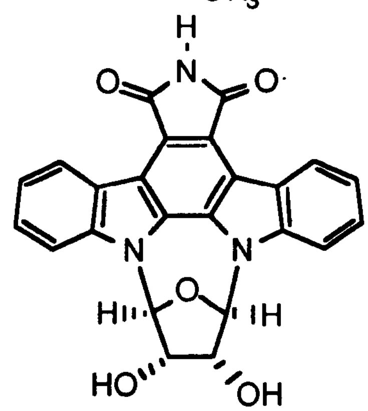

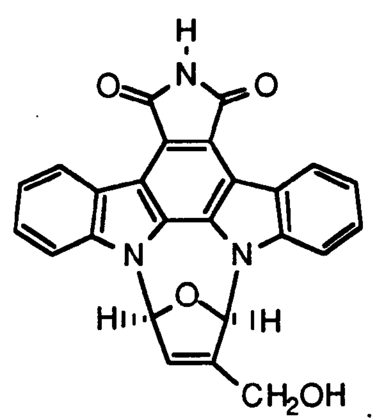
.

and

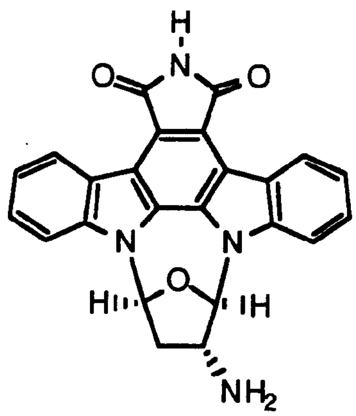

The most preferred compound of the invention is:

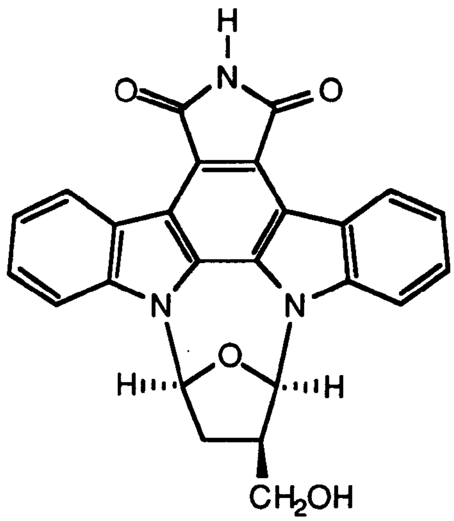

The invention also relates to a pharmaceutical composition comprising a thereapeutically effective amount of a compound of formula I in combination with a pharmaceutically acceptable carrier.

The invention also relates to a method of treating tumors which comprises administering to a mammal in need of such treatment an anti-tumor effective amount of a compound of formula I for such purpose.

The invention also relates to a method for treating psoriasis which comprises administering to a mammal in need of such treatment an anti-psoriatically effective amount of a compound of formula I for such purpose.

## DETAILED DESCRIPTION OF THE INVENTION

The numbering of positions on compounds of the invention is as follows:

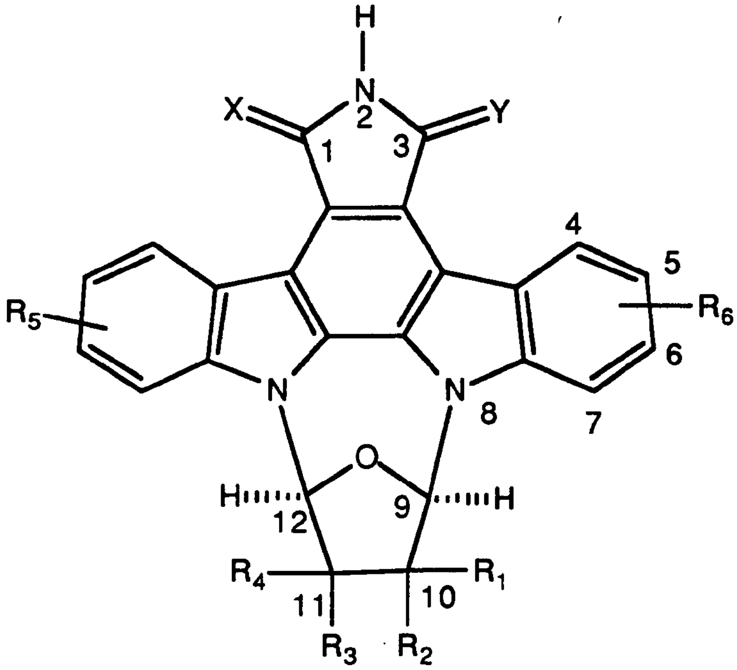

I

wherein $R_1$, $R_2$, $R_3$, $R_4$, $R_5$, $R_6$, X, and Y are as described above.

As used herein, a heavy dark line ( ▬ ) denotes a chemical bond coming above the plane of the page. A dashed line (''''''') denotes a chemical bond coming below the plane of the page. A wavy line ( ~~~~ ) denotes either a chemical bond whose stereochemistry is not known, or a mixture of compounds, one compound having the chemical bond going above the plane of the page and the other compound having the chemical bond going below the plane of the page.

Assymetric centers exist in compounds of formula I of the invention. Accordingly, compounds of formula I include stereoisomers.

All such isomeric forms and mixtures thereof are within the scope of the present invention. Unless otherwise indicated, the methods of preparation disclosed herein may result in product distributions which include all possible structural isomers, although it is understood that physiological response may vary according to stereochemical structure. The isomers may be separated by conventional means such as fractional crystallization, preparative plate or column chromatography on silica, alumina, or reversed phase supports or HPLC (high performance liquid chromatography).

Enantiomers may be separated, where appropriate, by derivatization or salt formation with an optically pure reagent, followed by separation by one of the aforementioned methods. Alternatively, enantiomers may be separated by chromatography on a chiral support.

An example of a particular isomer which is a compound of formula I of the invention is:

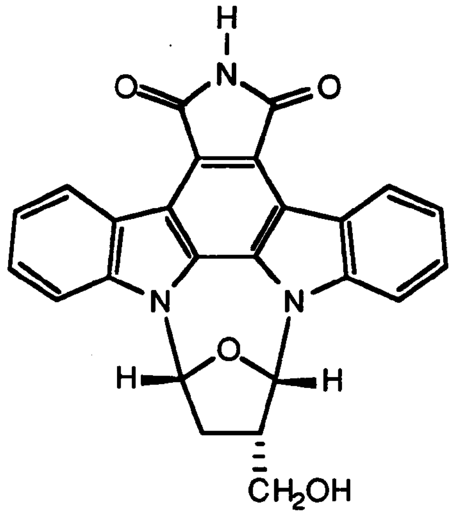

The enantiomer of the isomer just above is a compound of the formula:

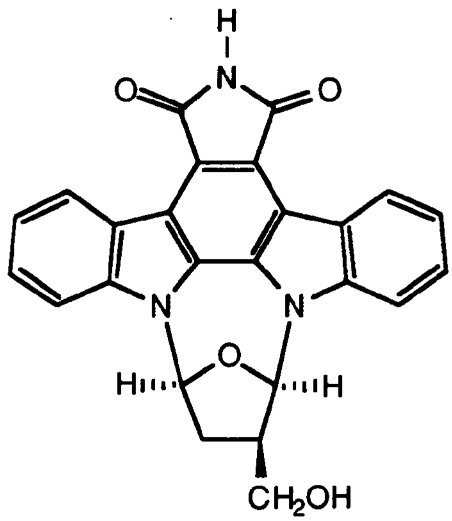

As pointed out above, both of the above compounds are compounds of the invention and such compounds and their racemic mixtures fall within the claimed invention.

In this disclosure, all structural formulas except those used in descriptions of stereochemistry, have been written with the bridgehead hydrogens of the sugar moiety pointing downward, in the following manner:

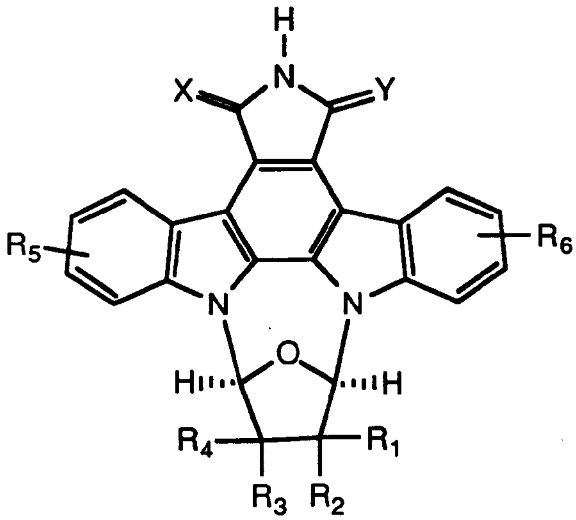

I

wherein $R_1$, $R_2$, $R_3$, $R_4$, $R_5$, $R_6$, X, and Y are as described above.

The above structure denotes compounds of the formula

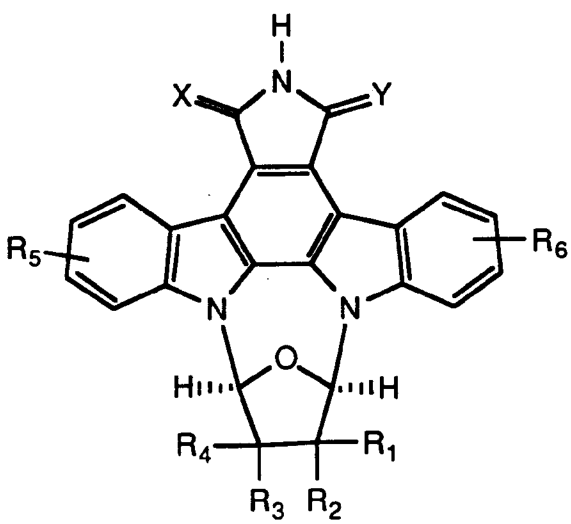

I

wherein $R_1$, $R_2$, $R_3$, $R_4$, $R_5$, $R_6$, X, and Y are as described above;

or

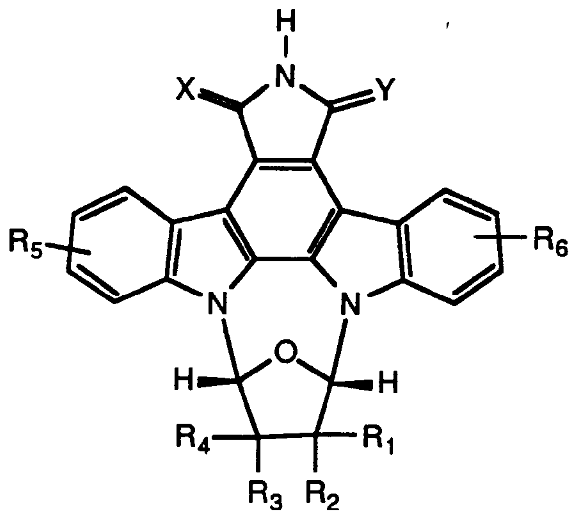

I

wherein $R_1$, $R_2$, $R_3$, $R_4$, $R_5$, $R_6$, X, and Y are as described above;
or a mixture of the above two enantiomers.

In certain structural formulas in this disclosure, substituents $R_1$, $R_2$, $R_3$, and $R_4$, on the sugar moiety have been written as follows

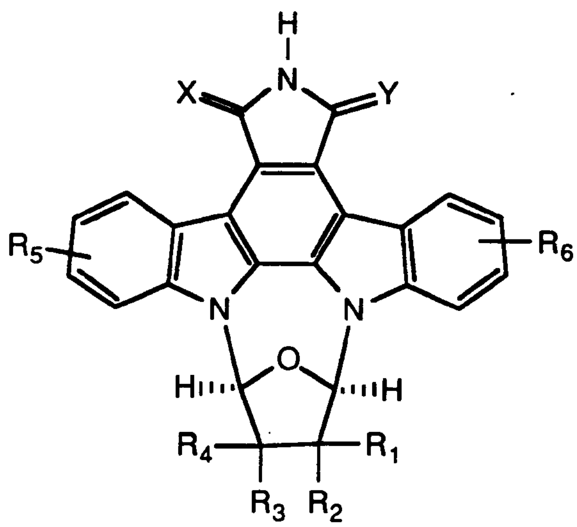

I

wherein $R_1$, $R_2$, $R_3$, $R_4$, $R_5$, $R_6$, X, and Y are as described above.

This structural formula denotes all stereoisomers, namely:

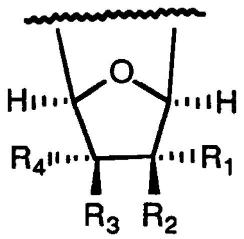

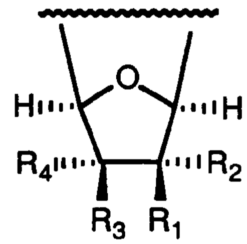

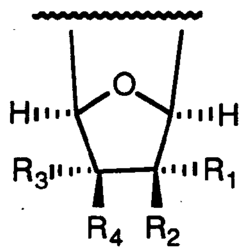

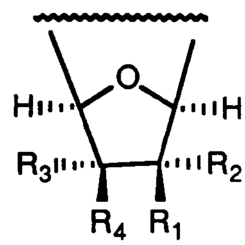

(The structural formulas of these compounds have been abbreviated by depicting only their bottom rings with wavy lines ( 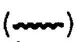 ) showing the bonds through which these rings are attached to the rest of the molecule. This abbreviation is used elsewhere in the specification.)

Two different compounds of the invention may also be diastereoisomers. For example

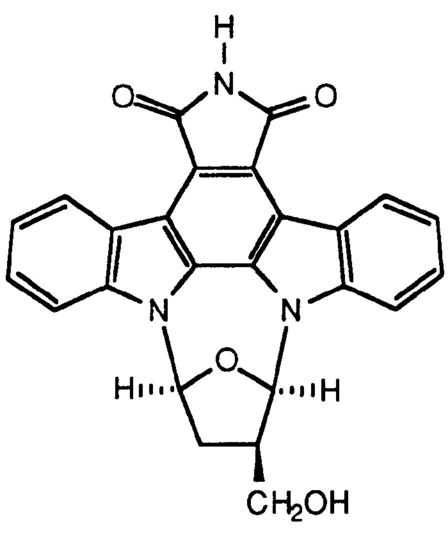

is a diastereoisomer of

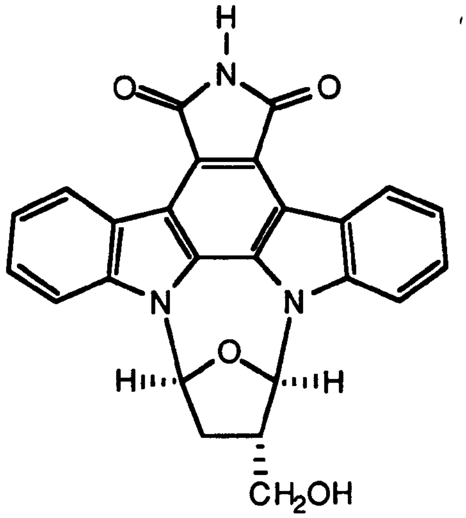

while

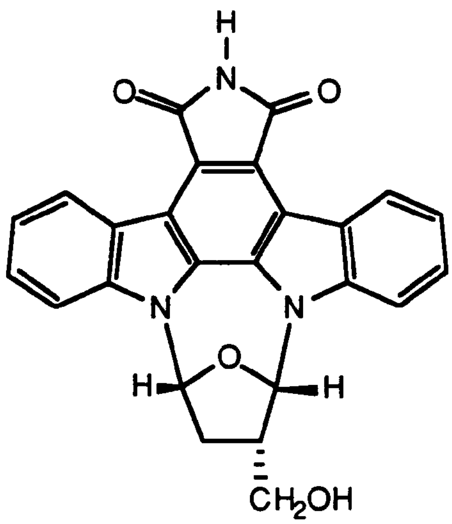

is a diastereoisomer of

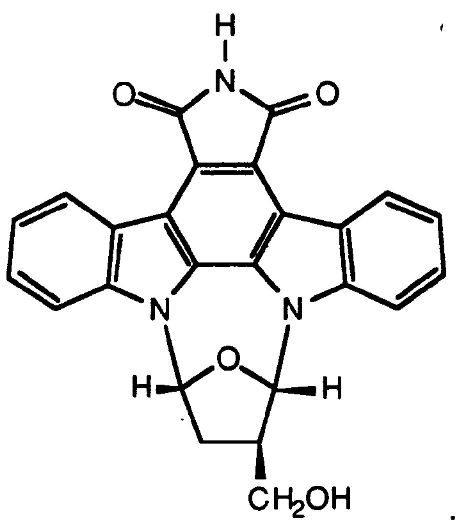

All of the above compounds are compounds of the invention.

The compounds of formula I can exist in unsolvated as well as solvated forms, including hydrated forms, e.g. the hemihydrate. In general, the solvated forms, with pharmaceutically acceptable solvents such as water, ethanol, and the like are equivalent to the unsolvated forms for the purposes of the invention.

Those compounds of formula I which contain a basic group such as $-CH_2NH_2$, form pharmaceutically acceptable salts. The preferred pharmaceutically acceptable salts are nontoxic acid addition salts formed by adding to a suitable compound of the invention about a stoichiometric amount of a mineral acid , such as HCl, HBr, $H_2SO_4$ or $H_3PO_4$ or of an organic acid such as acetic, propionic, valeric, oleic, palmitic, stearic, lauric, benzoic, lactic, para-toluenesulfonic, methane sulfonic, citric, maleic, fumaric, succinic and the like, respectively.

When utilized herein and in the appended claims, the following terms, unless otherwise specified have the following meanings

"alkyl" - (including the alkyl portions of alkoxy, etc) represents a straight or branched, saturated hydrocarbon chain having from 1 to 7 carbon atoms. The number of carbon atoms may be designated. For example, "$C_1$-$C_3$ alkyl" represents a straight or branched, saturated hydrocarbon having from 1 to 3 carbon atoms.

"substituted alkyl" -represents alkyl as defined above wherein one or more hydrogen atoms are replaced by a substituent selected from the group consisting of F, Cl, Br, OH, $OC_1$-$C_7$ alkyl, $OCOR^a$, -COOH, -CHO, $-COR^b$, -CN, SH, S($C_1$-$C_7$)alkyl, $S(O)_nR^c$, $-O(CH_2CH_2O)_qCH_2CH_2OH$, $-O(CH_2CH_2O)_pCH_2CH_2OCH_3$,$SCOR^d$, $OS(O)_2R^e$, $N_3$, $NH_2$, $N(R^f,R^g)$, 1-imidazolyl, and N ( acyl, $R^h$), wherein $R^a$, $R^b$ $R^c$, $R^d$, $R^e$, $R^f$, $R^g$ and $R^h$ are H or alkyl; n is 1 or 2; and q and p are 1 to 3; provided that the two heteroatoms cannot be directly bonded to the same carbon atom.

"acyl" - represents a CO-alkyl, a CO-substituted alkyl, a CO-aryl or a CO-aralkyl wherein alkyl, substituted alkyl, aryl and aralkyl are as defined herein .

"aryl" -represents a mono or bi-cyclic aromatic system. Examples of preferred aryl groups include those having from 6 to 14 carbon atoms. Representative examples include phenyl, 1-naphthyl, and 2-naphthyl. The aryl group may contain additional substituents selected from the group consisting of: halogen atoms (e.g., Cl, Br, F, and/or I), alkoxy, alkyl, and amino.

"aralkyl"-represents an alkyl group as defined above in which an aryl group as defined above replaces one of the alkyl hydrogen atoms. Representative examples include $-CH_2$phenyl, - $CH_2CH_2$phenyl, 4-hydroxybenzyl, 4-t-butyldimethylsilyloxybenzyl, and the like.

"heteroatom" -represents any atom other than carbon or hydrogen.

The compounds of formula I above may be prepared by the methods described below with reference to Schemes 1, 2, 3, 4, 5, 6 and 7, wherein $R_1$, $R_2$, $R_3$, $R_4$, $R_5$, and $R_6$ are as described above unless designated otherwise.

FORMULA SCHEME 1

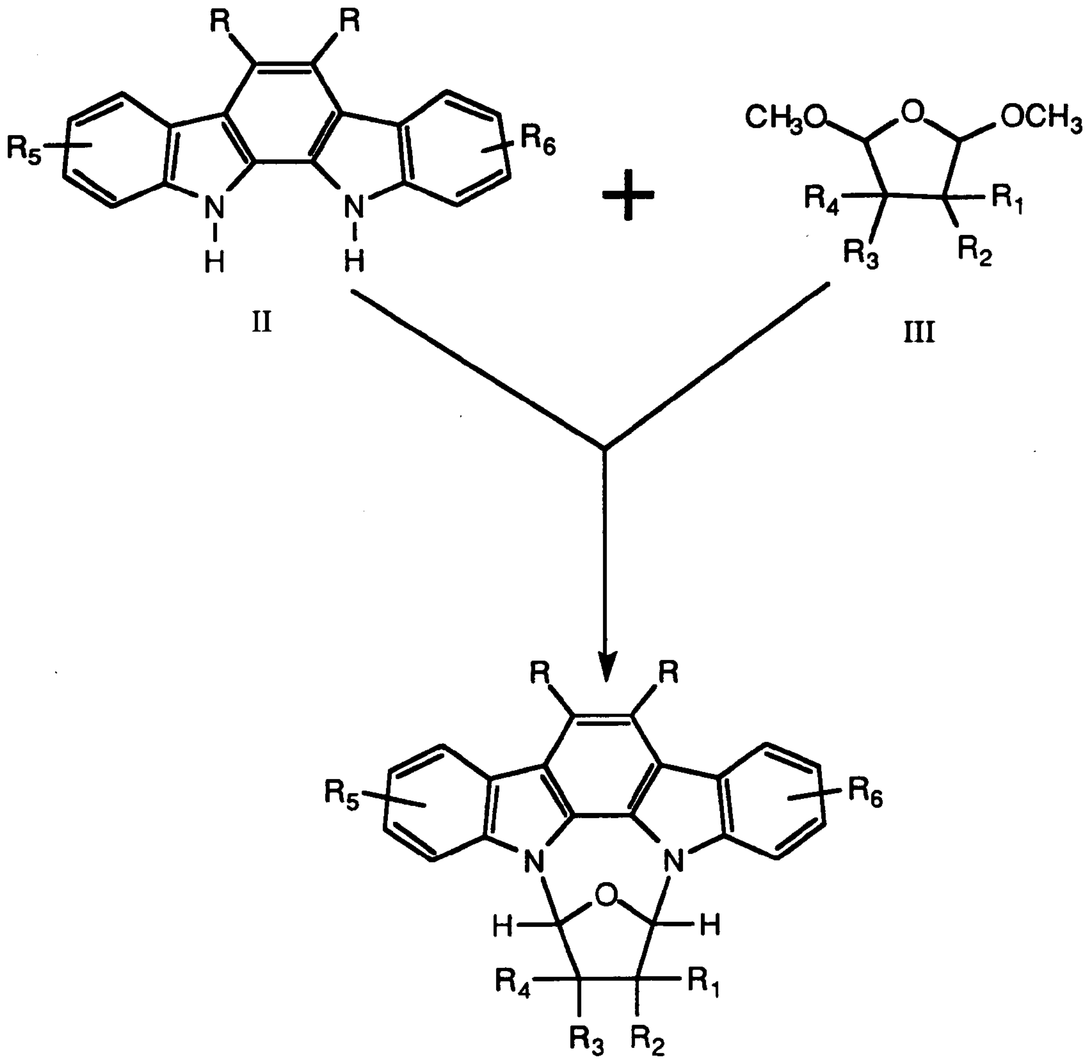

II

III

IV

wherein R is H, Br or $CO_2R_{21}$ and $R_{21}$ is alkyl or, aryl or aralkyl.

FORMULA SCHEME 2

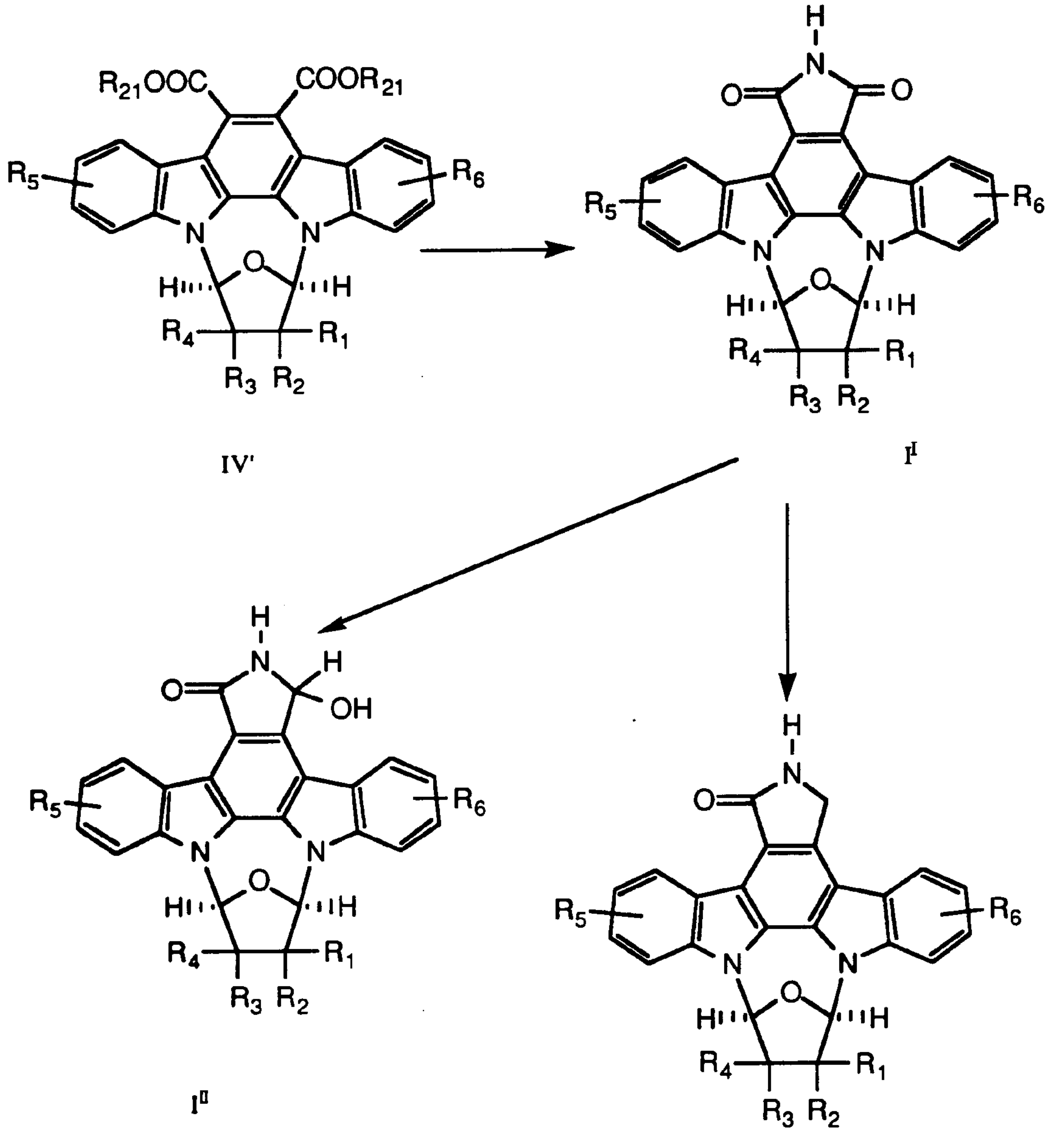

IV'

$I^{I}$

$I^{II}$

$I^{III}$

wherein $R_{21}$ is as described above.

FORMULA SCHEME 3

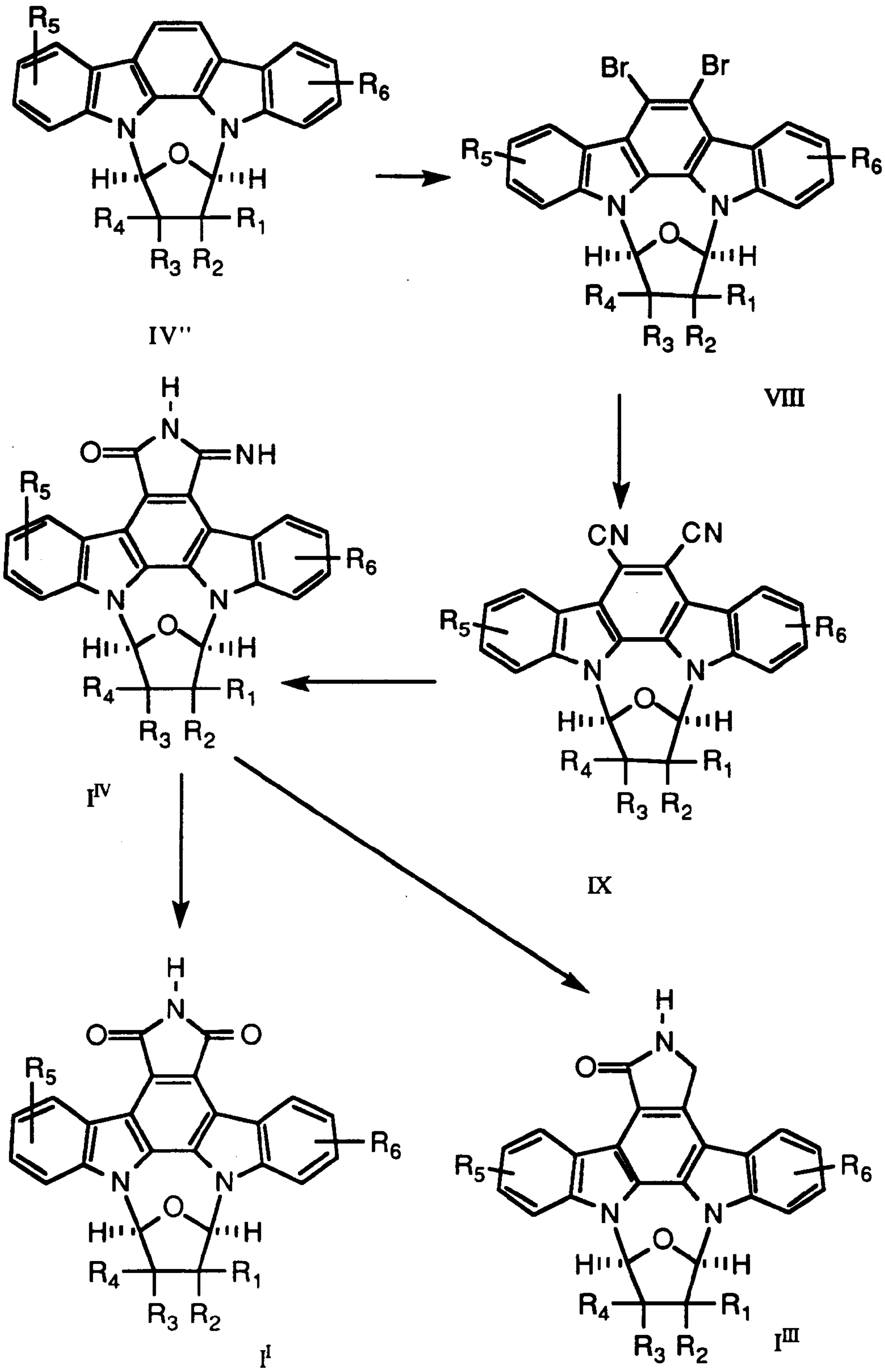

IV''

VIII

IX

$I^{IV}$

$I^{I}$

$I^{III}$

FORMULA SCHEME 4

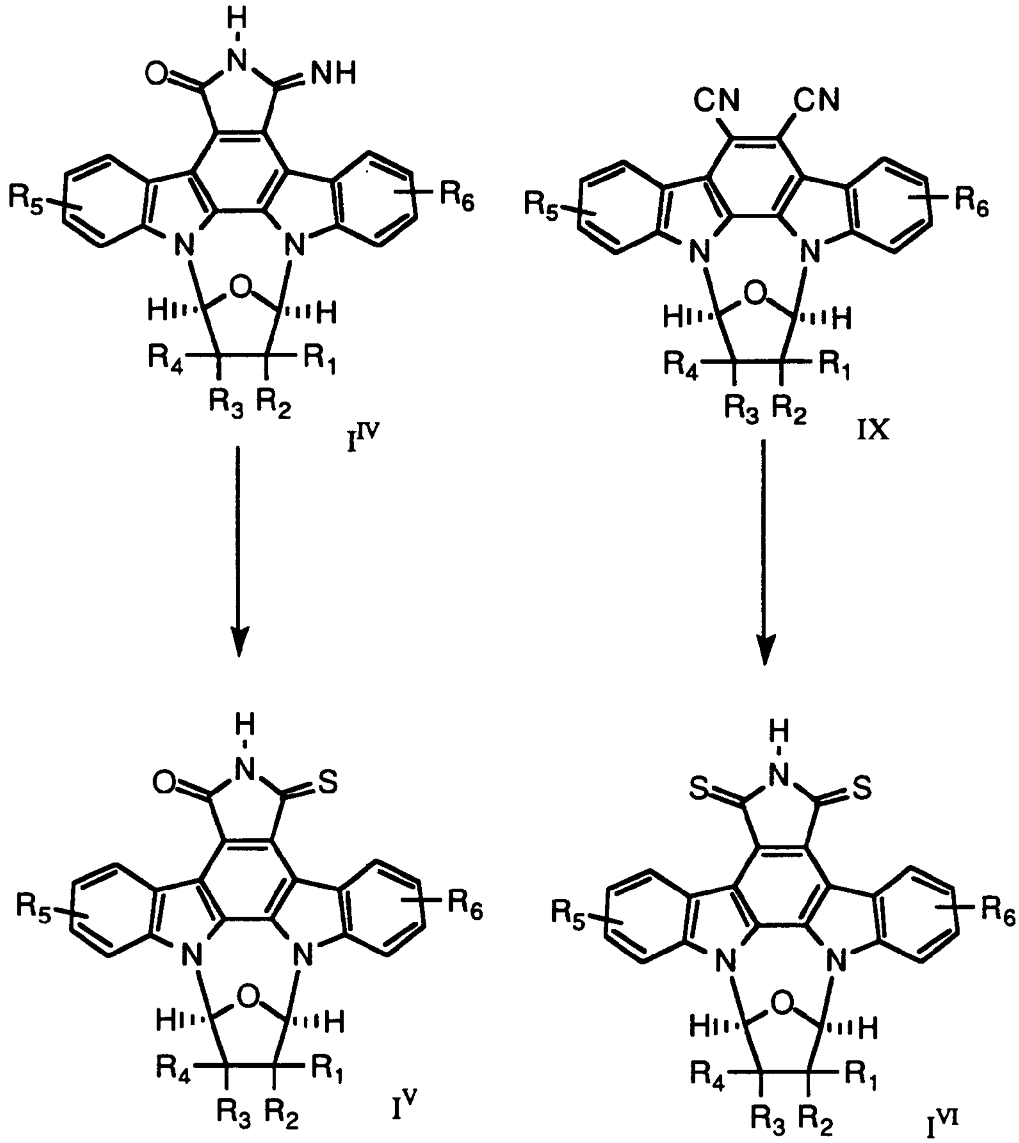

$I^{IV}$ IX

$I^{V}$ $I^{VI}$

## FORMULA SCHEME 5

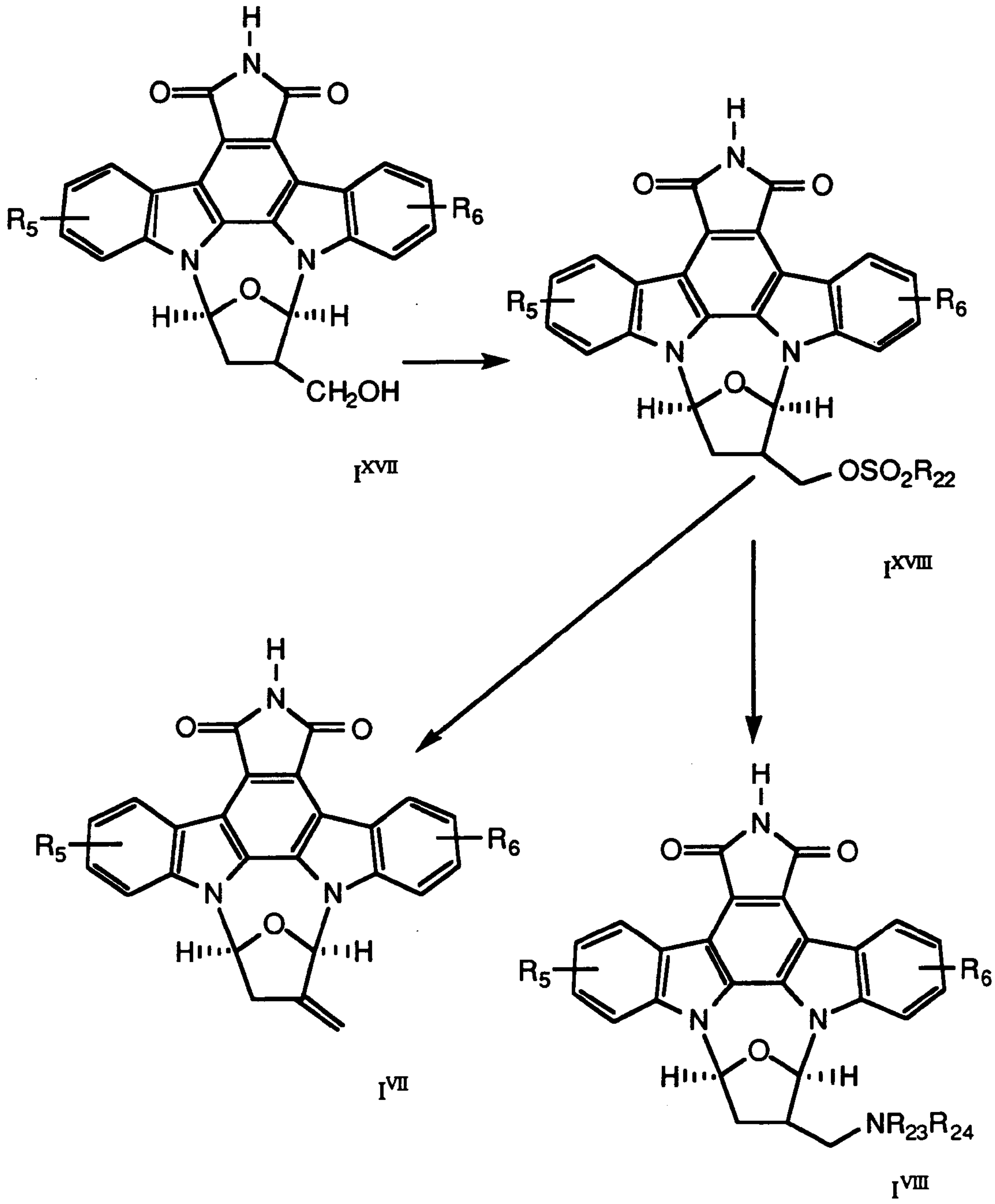

$I^{XVII}$

$I^{XVIII}$

$I^{VII}$

$I^{VIII}$

wherein $R_{22}$, $R_{23}$ and $R_{24}$ are independently H and alkyl.

## FORMULA SCHEME 6

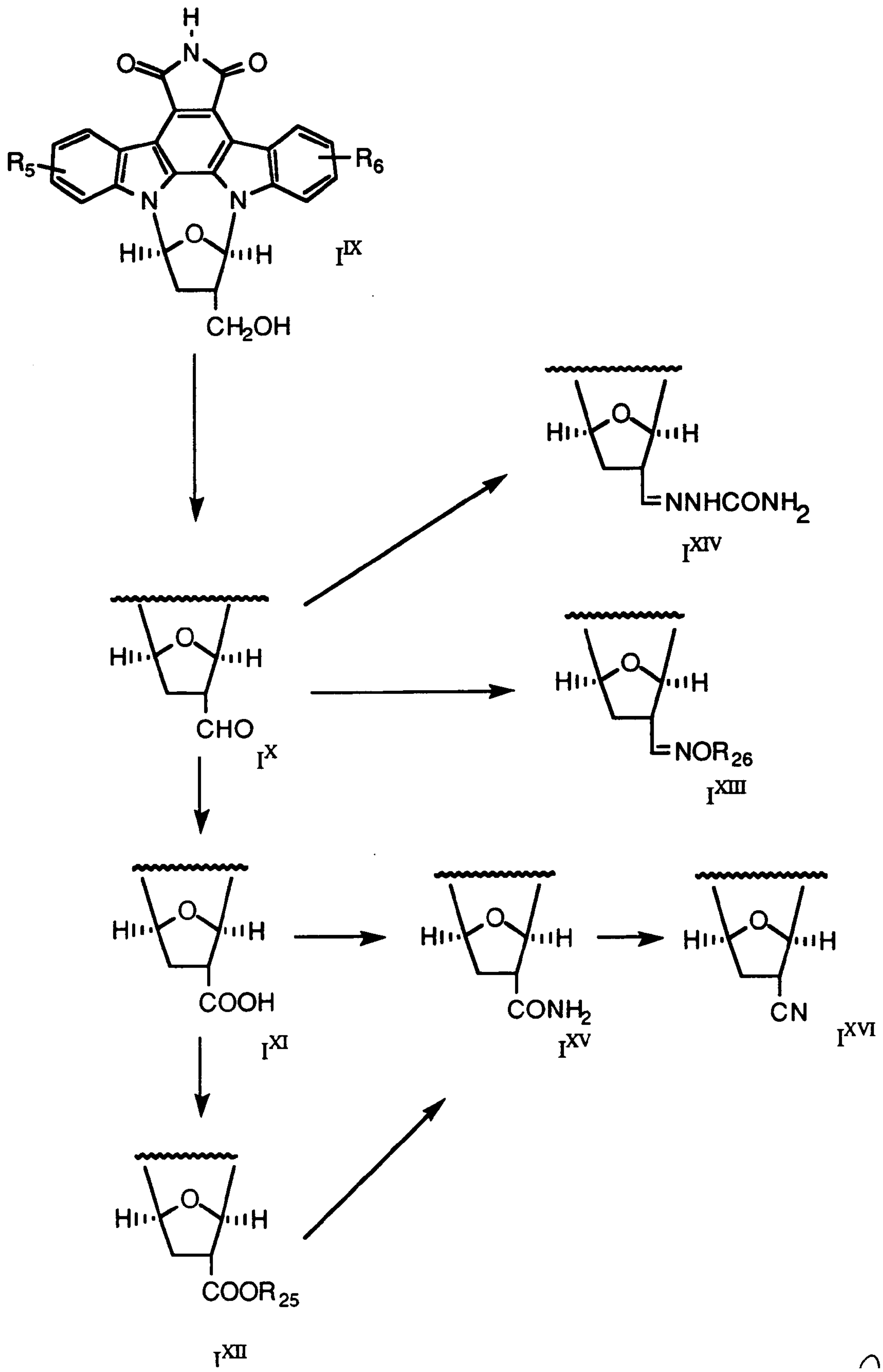

$I^{IX}$

$I^{XIV}$

$I^{X}$

$I^{XIII}$

$I^{XI}$

$I^{XV}$

$I^{XVI}$

$I^{XII}$

wherein $R_{25}$ is alkyl, aryl, or aralkyl and $R_{26}$ is H, methyl or benzyl.

FORMULA SCHEME 7

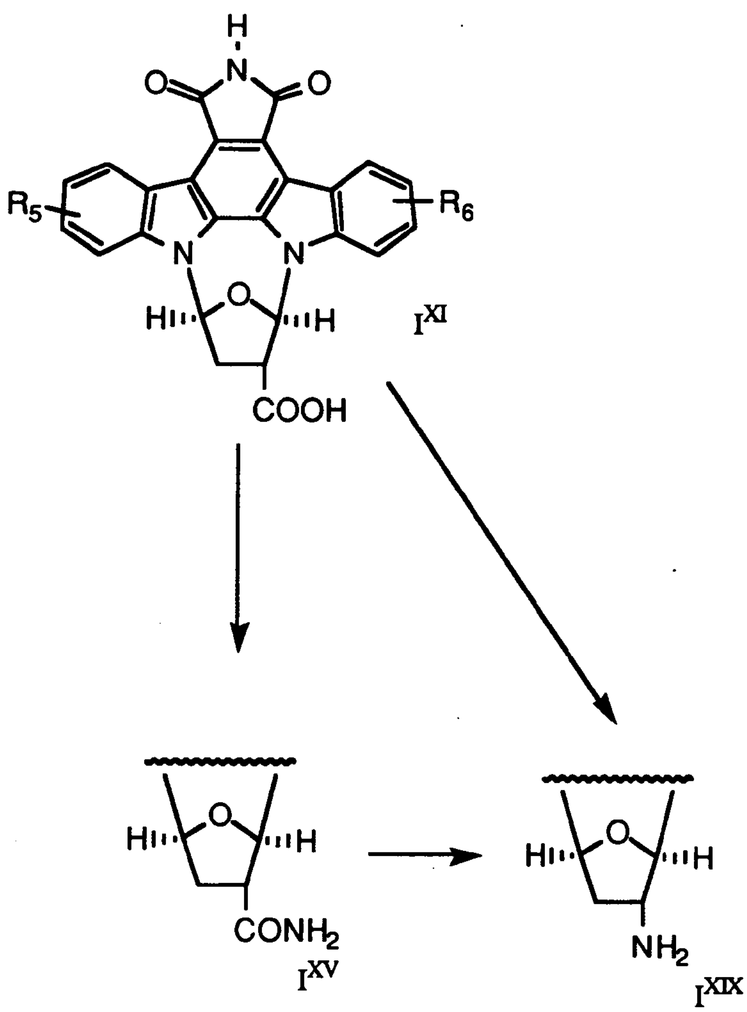

$I^{XI}$

$I^{XV}$

$I^{XIX}$

Starting materials of Formula Scheme 1 are known or may be prepared in accordance with known methods. For example, compounds of formula II may be prepared by methods set forth in Bhide et al Chem and Ind., 1957, 363; Mann and Wilcox J. Chem Soc., 1958, 1525; Moldenhauser and Simon, Chem. Bericht. 1969, 102, 1198; Bergmar and Pekman, Tet. Letts., 1987, 28, 4441; and Bergmar, Chemica Scripta, 1987, 27, 539. These five publications that have just been mentioned are hereby incorporated by reference.

Or compounds of formula II may be prepared by known methods from compounds whose preparation is described in the five just above mentioned publications.

Compounds of formula III are known or may be prepared by known methods.

As can be seen by the above formula schemes and the description below, compounds of formula $I^{I}$, $I^{II}$, $I^{III}$, $I^{IV}$, $I^{V}$, $I^{VI}$, $I^{VII}$, $I^{VIII}$, $I^{IX}$, $I^{X}$, $I^{XI}$, $I^{XII}$, $I^{XIII}$, $I^{XIV}$, $I^{XV}$, $I^{XVI}$, $I^{XVII}$, $I^{XVIII}$ and $I^{XIX}$ are encompassed by formula I of the invention.

As can be seen by the above formula schemes and the description below, compounds of formula IV′ and IV″ are encompassed by formula IV of the invention.

Unless otherwise indicated the reactions below are run under an inert atmosphere such as argon or nitrogen.

Unless otherwise indicated, degrees or "°" means degrees Celsius throughout the specification.

In Formula Scheme 1, a compound of formula II (the compound of formula II wherein R is H is preferred) may be reacted with a compound of formula III in the presence of an acid to obtain a compound of formula IV. The acid may be a protic acid selected from the group consisting of HCl, toluenesulfonic acid, camphor sulfonic acid and the like. Alternatively, the acid may be a Lewis acid selected from the group consisting of $BF_3$, $SnCl_4$,

$TiCl_4$ and the like. The most preferred acid for use in this reaction is camphor sulfonic acid. The reaction is carried out in a nonpolar solvent such as toluene, or more preferably $CH_2Cl_2$.The reaction may be conducted at a temperature in the range of about 0° to about 100°. The resulting compound of formula IV may be purified or used directly in the next step of the synthesis of a compound of formula I. Purification may be carried out by standard techniques such as column chromatography or crystallization.

It is noted that in Formula Scheme 1 a compound of formula III will often be reacted in the form of a racemic mixture such as, for example:

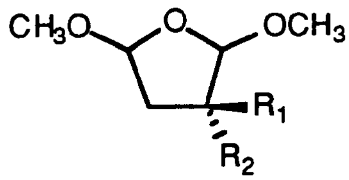

and

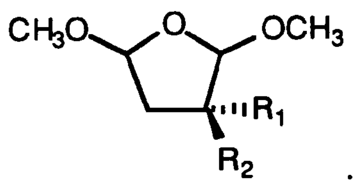

In such a case the reaction shown in Formula Scheme 1 will result, for example, in compounds of the formulas:

(A)

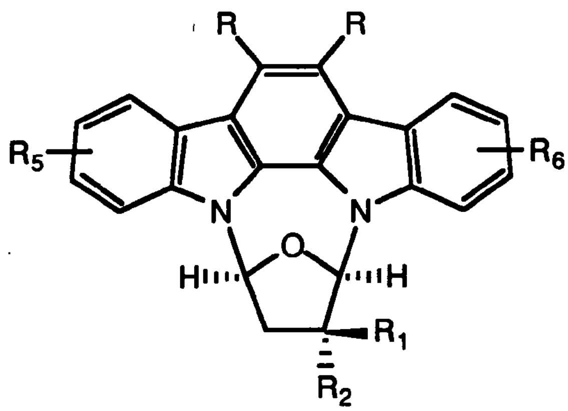

(B)

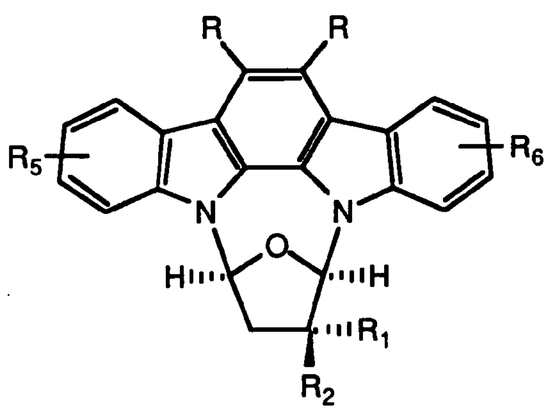

(C)

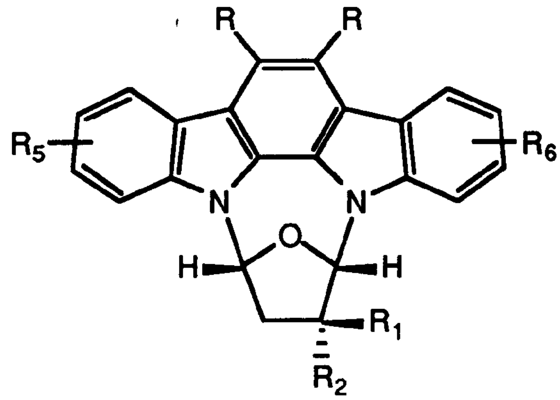

and

(D)

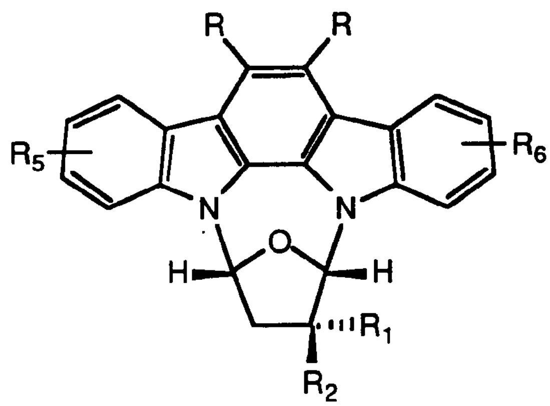

Compounds (A) and (B) above are diastereomers of each other. Similarly, compounds (C) and (D) above are diastereomers of each other. Compounds (A) and (D) above are enantiomers of each other. Similarly, compounds (B) and (C) above are enantiomers of each other. The above described stereochemistry is carried into the compounds of formula I of the invention. Other compounds of the invention may exist in similar stereoismeric forms.

In Formula Scheme 2, a compound of formula IV′ is reacted with an ammoniating agent such as $NH_3$, ammonium carbonate, or more preferably ammonia in a solvent such as dimethylformamide (DMF), or dimethylsulfoxide (DMSO); more preferably DMF at a temperature in the range of about 50° to about 150° to obtain a compound of formula $I^{I}$. The resulting compound of formula $I^{I}$ may be purified or used directly in the synthesis of another compound of formula

I. Purification may be carried out by standard techniques such as column chromatography or crystallization.

A compound of formula $I^{I}$ may be treated with a reducing agent such as $LiAlH_4$ or, more preferably $NaBH_4$ to obtain a compound of formula $I^{II}$.The reaction is carried out in a nonpolar solvent such as tetrahydrofuran(THF) or dioxan, or more preferably THF.The reaction may be conducted at a temperature in the range of about 0° to about 50°. The resulting compound of formula $I^{II}$ may be purified or used directly in the synthesis of another compound of formula I. Purification may be carried out by standard techniques such as column chromatography.or crystallization.

Alternatively, in Formula Scheme 2, a compound of formula $I^{I}$ may be treated with $BH_3$ to obtain a compound of formula $I^{III}$.The reaction is carried out in a nonpolar solvent such as THF or dioxan or, preferably THF.The reaction may be conducted at a temperature in the range of about 0° to about 100°. The resulting compound of formula $I^{III}$ may be purified. Purification may be carried out by standard techniques such as column chromatography or crystallization.

In Formula Scheme 3, a compound of formula IV″ may be treated with a brominating agent such as $Br_2$, or more preferably N-bromosuccinimide or phenyltrimethylammonium tribromide to obtain a compound of formula VIII.The reaction is carried out in a polar solvent such as dimethylformamide (DMF) or dimethylacetamide (DMAC) or more preferably DMF.The reaction may be conducted at a temperature in the range of about 0° to

about 30°. The resulting compound of formula VIII may be purified or used directly in the next step of the synthesis of a compound of formula I. Purification may be carried out by standard techniques such as column chromatography or crystallization.

A compound of formula VIII may be treated with copper(I) cyanide to obtain a compound of formula IX.The reaction is carried out in a polar, aprotic solvent such as DMF or dimethyl acetamide preferably DMAC.The reaction may be conducted at a temperature in the range of about 140° to about 200°. The resulting compound of formula IX may be purified or used directly in the next step of the synthesis of a compound of formula I. Purification may be carried out by standard techniques such as column chromatography or crystallization.

A compound of formula IX may be treated with a base such as sodium hydroxide, potassium carbonate, or more preferably potassium hydroxide to obtain a compound of formula $I^{IV}$. The reaction is carried out in a polar solvent such as DMSO. The reaction may be conducted at a temperature in the range of about 50° to about 150°. The resulting compound of formula $I^{IV}$ may be purified or used directly in the next step of a synthesis of compound of formula I of the invention. Purification may be carried out by standard techniques such as column chromatography or crystallization.

A compound of formula $I^{IV}$ may be treated with an aqueous acid such as HCl, $H_2SO_4$, or, more preferably trifluoroacetic acid to obtain a compound of formula II.The reaction is carried out in a polar solvent such as DMF or methanol, more preferably DMSO.The reaction may be conducted at a temperature in the range of about 0° to about 100°. The resulting compound of formula $I^{I}$ may be purified or used directly in the next step of the synthesis of another compound of formula I of the invention. Purification may be carried out by standard techniques such as column chromatography, or crystallization.

Alternatively, a compound of formula $I^{IV}$ may be treated with acetic acid and a reducing agent such as such as t-$BuNH_2.BH_3$, or more preferably $NaBH_3CN$ to obtain a compound of formula $I^{III}$.The reaction is carried out in a polar solvent such as DMF, ethanol, or more preferably acetic acid.The reaction may be conducted at a temperature in the range of about 25° to about 100°.The resulting compound of formula $I^{III}$ may be purified. Purification may be carried out by standard techniques such as column chromatography or crystallization.

The synthetic routes of Formula Scheme 3 are preferred means for preparing compounds of formula I of the invention.

In Formula Scheme 4, a compound of formula IX may be treated with a sulfurating agent such as $Na_2S$, $H_2S$-pyridine or, more preferably NaSH to obtain a compound of formula $I^{V}$.The reaction is carried out in a polar solvent such as DMF or more preferably DMSO,The reaction may be conducted at a temperature in the range of about 20° to about 100°. The resulting compound of formula $I^{V}$ may be purified. Purification may be carried out by standard techniques such as column chromatography or crystallization.

Alternatively, in Formula Scheme 4, a compound of formula $I^{IV}$ may be treated with a sulfurating agent such as $Na_2S$, $H_2S$-pyridine or, more preferably NaSH to obtain a compound of formula $I^{V}$ The reaction is carried out in a polar solvent such as DMF or more preferably DMSO. The reaction may be conducted at a temperature in the range of about 20° to about 100°. The resulting compound of formula $I^{V}$ may be purified. Purification may be carried out by standard techniques such as column chromatography.

In Formula Scheme 5, a compound of formula $I^{XVII}$ may be treated with a sulfonylating agent such as $CH_3SO_2Cl$, $C_6H_5SO_2Cl$, $(C_6H_5SO_2)_2O$ or, more preferably $R_{22}SO_2Cl$ (wherein $R_{22}$ is methyl, phenyl or p-toluene) to obtain a compound of formula XI.The reaction is carried out in a nonhydroxylic solvent such as $CH_2Cl_2$, $CHCl_3$, or THF containing a base such as pyridine, diisopropylethylamine, or triethylamine.The reaction may be conducted at a temperature in the range of about 0° to about 50°. The resulting compound of formula XI may be purified or used directly in the next step of the synthesis of a compound of formula I. Purification may be carried out by standard techniques such as column chromatography or crystallization. Preparation of the starting material of formula $I^{XVII}$ is described in the earlier formula schemes and the description accompanying them.

A compound of formula XI may be treated with an amine such as ammonia, methylamine, or dimethylamine to obtain a compound of formula $I^{VIII}$.The reaction is carried out in a polar solvent such as DMF, DMAC, or more preferably DMSO. The reaction may be conducted at a temperature in the range of about 25° to about 100°. The resulting compound of formula $I^{VIII}$ may be purified or used directly in the next step of the synthesis of another compound of formula I. Purification may be carried out by standard techniques such as crystallization.

Alternatively, in Formula Scheme 5, a compound of formula XI may be treated with a base such as triethylamine or, more preferably, diazabicycloundecene (DBU) to obtain a compound of formula $I^{VII}$.The reaction is carried out in an aprotic solvent such as THF or more preferably DMSO.The reaction may be conducted at a temperature in the range of about 20° to about 120°. The resulting compound of formula $I^{VIII}$ may be purified or used directly in the next step of the synthesis of another compound of formula I. Purification may be carried out by a standard technique such as crystallization.

In Formula Scheme 6, a compound of formula $I^{IX}$ is reacted with an oxidizing agent such as pyridine-sulfur trioxide in dimethylsulfoxide at a temperature in the range of about 0° to about 50° to obtain an aldehyde of

formula $I^X$. The resulting compound of formula $I^X$ may be purified or used directly in the synthesis of another compound of formula I. Purification may be carried out by standard techniques such as column chromatography or crystallization. A starting materiel of formula $I^{IX}$ may be obtained by following the procedures set forth in Formula Scheme 2.

A compound of formula $I^X$ is reacted with an oxidizing agent such as silver oxide in an aqueous organic solvent such as DMSO-water, or DMF-water at a temperature in the range of about 0° to about 50° to obtain a carboxylic acid of formula $I^{XI}$. The resulting compound of formula $I^{XI}$ may be purified or used directly in the synthesis of another compound of formula I. Purification may be carried out by standard techniques such as column chromatography or crystallization.

A compound of formula $I^{XI}$ is esterified with methyl or ethyl iodide in a polar solvent such as DMF or DMSO in the presence of a base such as potassium carbonate or cesium carbonate at a temperature in the range of about 0° to about 50° C obtain the compound of formula $I^{XII}$. The resulting compound of formula $I^{XII}$ may be purified or used directly in the synthesis of another compound of formula I. Purification may be carried out by standard techniques such as column chromatography or crystallization.

A compound of formula $I^{XII}$ may also be reacted with ammonia in a polar solvent such as DMSO at a temperature in the range of about 100° to about 150° to obtain an amide of formula $I^{XV}$. It is preferred that this reaction be run under inert atmosphere such as argon or nitrogen.Alternatively, a compound of formula $I^{XII}$ may be reacted ammonia and trimethylaluminum in a nonpolar solvent such as methylene chloride or toluene at a temperature in the range of about 25° to about 100° to obtain an amide of formula $I^{XV}$.

As shown in Formula Scheme 7, a compound of formula $I^{XI}$ or $I^{XV}$ may also be subjected to known degradations of a carboxylic acid or carboxamide function, such as the Curtius and Hoffmann degradations, to provide the corresponding amino compound of formula $I^{XIX}$.

The resulting compound of formula $I^{XV}$ may be purified or used directly in the synthesis of another compound of formula I. Purification may be carried out by standard techniques such as column chromatography or crystallization.

Alternatively, and preferably, a compound of formula 19 may be converted to an amide of formula $I^{XV}$ by reaction with an activating agent such as ethylchloroformate or pivaloyl chloride in a nonhydroxylic solvent such as $CH_2Cl_2$ or $CHCl_3$ at a temperature in the range of about -20° to about 0° in the presence of triethylamine followed by reaction with ammonia at a temperature in the range of about 0° to about 25°. The resulting compound of formula $I^{XV}$ may be purified or used directly in the synthesis of another compound of formula

I. Purification may be carried out by standard techniques such as column chromatography or crystallization.

A compound of formula $I^{XV}$ may be reacted with a dehydrating agent such as phosgene-triethlamine or benzene-sulfonylchloride-pyridine in a solvent such as $CH_2Cl_2$ or $CHCl_3$ at a temperature in the range of about 0° to about 50° to obtain a cyano compound of formula $I^{XVI}$. The resulting compound of formula $I^{XVI}$ may be purified by standard techniques such as column chromatography or crystallization or used directly in the synthesis of another compound of formula I.

Alternatively, compound of formula $I^X$ may be converted to an oxime of formula $I^{XIII}$ by reaction with a compound such as $NH_2OR_{26}$ wherein $R_{26}$ is hydrogen, methyl or benzyl. The reaction is run in a polar solvent such as ethanol, DMF or DMSO at a temperature in the range of about 0° to about 50° to obtain a compound of formula $I^{XIII}$. The resulting compound of formula $I^{XIII}$ may be purified by standard techniques such as column chromatography or crystallization.

A compound of formula $I^X$ may also be converted to a semicarbazone of formula $I^{XIV}$ by reaction with $NH_2NHCONH_2$ in a polar solvent such as methanol, ethanol,or DMF, at a temperature in the range of about 0° to about 50°, to obtain a compound of formula $I^{XIV}$. The resulting compound of formula $I^{XIV}$ may be purified by standard techniques such as column chromatography or crystallization.

As can be seen from Formula Scheme 6, the chemical transformations are carried out at C-10 of the sugar moiety. It is recognized that similar transformations may be carried out on C-11 or on both C-10 and C-11 of the same compound, to provide additional compounds of the invention. The only requirement for carrying out such chemical transformations on C-10, or both C-10 and C11 is to start with a compound having $-CH_2OH$ at the appropriate position(s) on the sugar moiety. Preparation of such compounds is described throughout the specification.

The compounds of formula I of the invention are useful as agents for treating tumors. For example, they are useful as agents in the treatment of tumors of the skin, colon and breast. The compounds of the invention reduce colony formation in HT-24 human breast carcinoma and HT-29 human colon carcinoma cells.

Moreover, Meyer et al, Int. J. Cancer, 43, 851 -856 (1989) and Takahashi et al, The Journal of Antibiotics, vol. XL, No. 12, 1782-1783 (1987) have recently demonstrated that other compounds which are indolocarbazoles and which inhibit PKC also inhibit murine lymphotic leukemia P388; and HL-60 human promyelocytic leukemia, as well. On the basis of the two publications mentioned just above, the compounds of the invention are expected to inhibit the P388 and HL60 leukemia cell lines.

The potential for compounds of formula I of the invention to have anti-tumor activity, may be demonstrated by their ability to inhibit protein Kinase C ("PKC") in vitro as shown in the test protocol just below.

## TEST METHODS

The below listed Test Methods A and B were run in accordance with the procedures described in Bishop, W.R., Petrin, J., Wang, L., Ramesh, U., and Doll, R.J. (1990) Biochemical Pharmacology 40: No. 9, pp. 2129-2135 and Hannun et al, J. Biol. Chem. 260, 10039-10043, (1985), which are herein incorporated by reference.The following modifications were made in this procedure:

### A. **Protein Kinase C ("PKC") Protocol**

Mixed micellular protein kinase C assays were conducted by the methods described in the two articles cited just above, (namely, Bishop et al, and Hannun et al), in a reaction mixture (0.25 mL total volume) containing: 20 mM Tris-HCl (pH 7.5); 200 mg/mL histone III-S; 10 mM $MgCl_2$; 5 mM [g-32P]ATP (4 x 106 cpm/nmol); 200 mM $CaCl_2$; 32 mg/mL of phosphatidylserine; 1.6 mg/mL of 1-oleoyl-2-acetyl-glycerol and various concentrations of compounds of this invention added from a dimethyl-sulfoxide (DMSO) stock solution. The final concentration of DMSO in the reaction was 2% w/v. Reactions were started by addition of approximately 7 mg of partially purified rat brain PKC and allowed to proceed at 23°C for 15 minutes. Reactions were terminated by collection of trichloroacetic acid-precipitated phosphorylated histone on GF/C filters using a Brandel Cell Harvester (Model No. M-24-R).

The following data demonstrate the activity of the compounds of the invention in the above protocol.

## IN VITRO INHIBITION OF PKC

### TABLE I

| COMPOUND FROM EXAMPLE NO. | $IC_{50}$ (nanomolar) |
|---|---|
| 25 | 13 |
| 26 | 19 |
| 27 | 18 |
| 28 | 58 |
| 29 | 42 |
| 30 | 50 |
| 33 | 180 |
| 34 | 18 |
| 35 | 7.5 |
| 36 | 10 |
| 37 | 115 |
| 38 | 1.4 |
| 39 | 5 |
| 40 | 15 |
| 41 | 7.5 |
| 47 | 5 |
| 45 | 13 |
| 46 | 230 |

| | |
|---|---|
| 47 | 11 |
| 48 | 7 |
| 49 | 4 |
| 57 | 23 |
| 58 | 23 |
| 63 | 20 |
| 64 | 19 |
| 67 | 0.6 |
| 66 | 20 |
| 69 | 0.8 |
| 68 | 1.0 |
| 65 | 0.5 |

The anti-psoriatic activity of the compounds of formula I of the invention may be demonstrated in the following experimental protocol.

**$^3H$ Thymidine Uptake in Normal Human Keratinocytes (NHEK): Effects of Compounds of the Invention**

Second passage NHEK cells grown in MCDB 153 media with a 5% $CO_2$ air mixture at 37° were used for experiments.

Compounds of the invention which were tested were prepared at 5mM stock solutions in DMSO. Cells were grown to 60-80% confluency in 6 well trays. Compounds were added to give a concentration of $5x10^{-6}$ molar and cultures were simultaneously pulsed with 1 microCurie/mL $^3H$ thymidine for 20 hours. Cells were then harvested and tritium was counted using a liquid scintillation counter; results are the average of 8 wells per compound. In the control, when no compound is employed, there is 0% inhibition of thymidine uptake.

## Results

| Compound (Ex No.) | % Inhibition of Control | Significance Level |
|---|---|---|
| | | |
| 22 | 77.3 | p=0.01 |
| 35 | 99.2 | p=0.03 |
| 23 | 78.8 | N.S. |

N.S. means not statistically significant.

**Dose Response Study**

A dose response study of the compound from Example 35, using the just above described test was conducted. Concentrations of the compound from Example 35 ranging from $5x10^{-5}$ to $5x10^{-6}$ M in tenfold dilutions were conducted. The compound from Example 35 had an $IC_{50}$ of 1.72 $x10^{-6}$M.

The compounds of this invention can be administered in any number of conventional dosage forms, e.g., topical, oral, parenteral, rectal, transdermal, and the like. Oral or rectal dosage forms include capsules, tablets, pills, powders, cachets, and suppositories. Liquid oral dosage forms include solutions and suspensions. Parenteral preparations include sterile solutions and suspensions. Topical dosage forms can be creams, ointments, lotions, transdermal devices (e.g., of the conventional patch or matrix type) and the like.

The formulations and pharmaceutical compositions contemplated by the above dosage forms can be prepared with conventional pharmaceutically acceptable excipients and additives, using conventional techniques. Such pharmaceutically acceptable excipients and additives are intended to include carriers, binders, flavorings, buffers, thickeners, coloring agents, stabilizing agents, emulsifying agents, dispersing agents, suspending agents, perfumes, preservatives lubricants, etc.

Suitable pharmaceutical acceptable solid carriers are magnesium carbonate, magnesium stearate, talc, sugar, lactose, pectin, dextrin, starch, gelatin, tragacanth, methylcellulose, sodium carboxymethylcellulose, low melting waxes, cocoa butter and the like. Capsules can be made wherein the active compound is inserted into the capsules along with a pharmaceutically acceptable carrier, The active compounds of this invention can be mixed with pharmaceutically acceptable excipients or be used in finely divided powder form without excipients for inclusion into the capsules. Similarly, cachets are included.

Liquid form preparations include solutions, suspensions and emulsions such as water or water-propylene glycol solutions for parenteral injection. Liquid preparations can also be formulated in solution in polyethylene glycol and/or propylene glycol, which may contain water. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the active component in finely divided form in water with viscous material, i.e., pharmaceutically acceptable natural or synthetic gums, resins, methylcellulose, sodium carboxymethylcellulose and other well-known suspending agents.

Formulations for topical application may include the above liquid forms, as well as creams, aerosols, sprays, dusts, powders, lotions and ointments which are prepared by combining an active ingredient according to this invention with conventional pharmaceutical acceptable diluents and carriers commonly used in topical, dry, liquid, cream and aerosol formulations. Ointment and creams may, for example, be formulated with an aqueous or oily base with the addition of suitable thickening and/or gelling agents. Such bases may, thus, for example, include water and/or an oil such as liquid paraffin or a vegetable oil such as peanut oil or castor oil. Thickening agents which may be used according to the nature of the base include soft paraffin, aluminum stearate, cetostearyl alcohol, propylene glycol, polyethylene glycols, woolfat, hydrogenated lanolin, beeswax, etc.

Lotions may be formulations with an aqueous or oil base and will, in general, also include one or more of pharmaceutically acceptable stabilizing agents, emulsifying agents, dispersing agents, suspending agents, thickening agents, coloring agents, perfumes and the like.

Powders may be formed with the aid of any suitable pharmaceutically acceptable powder base, e.g., talc, lactose, starch, etc. Drops may be formulated with an aqueous base or nonaqueous base also comprising one or more pharmaceutically acceptable dispersing agents, suspending agents, solubilizing agents, etc.

The topical pharmaceutical compositions may also include one or more preservatives or bacteriostatic agents, e.g., methyl hydroxybenzoate, propyl hydroxybenzoate, chlorocresol, benzalkonium chlorides, etc.

The topical pharmaceutical compositions may also contain an active compound of this invention in combination with other active ingredients such as antimicrobial agents, particularly antibiotics, anesthetics, analgesics and antipruritic agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses under conditions which retard possible decomposition. The solid form preparations intended to be converted to liquid form may contain, in addition to the active material, pharmaceutically acceptable flavorants, colorants, stabilizers, buffers, artificial and natural sweeteners, dispersants, thickeners, solubilizing agents and the like. The solvent utilized for preparing the liquid form preparation may be water, isotonic water, ethanol, glycerine, propylene glycol and the like as well as mixtures thereof. Naturally, the solvent utilized will be chosen with regard to the route of administration, for example, liquid preparations containing large amounts of ethanol are not suitable for parenteral

use.

The compounds of this invention may also be deliverable transdermally for systemic distribution. The transdermal compositions can take the form of creams, lotions and/or emulsions and can be included in a transdermal patch of the matrix or reservoir type as are conventional in the art for this purpose.

A composition of the invention comprises a therapeutically effective amount of a compound of the invention in combination with a pharmaceutically acceptable carrier material.

The compounds of this invention may be administered by any conventional mode of administration by employing a therapeutically effective amount of a compound of this invention for such mode. The dosages may be varied depending upon the requirements of the patient in the judgment of the attending clinician, the severity of the condition being treated and the particular compound being employed. Determination of the proper dosage for a particular situation is within the skill of the art. Treatment can be initiated with smaller dosages which are less than the optimum dose of the compound. Thereafter, the dosage should be increased by small increments until the optimum effect under the circumstances is reached. For convenience, the total daily dosage may be divided and administered in portions during the day if desired.

## Examples

Example 1

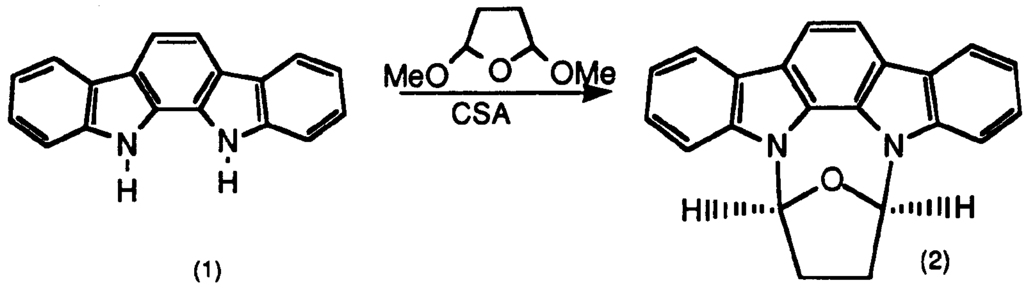


(1) (2)

A mixture of indolocarbazole (5.0 g), 2,5-dimethoxytetrahydrofuran (13 g) and camphorsulfonic acid (1.0 g) in dichloromethane (100 mL) was stirred at room temperature for 20 hours. Hexanes (50 mL) were added, and the product was filtered, washed with 1:1 dichloromethane-hexane, and dried at 25 ° in high vacuum to an off-white powder (4.51 g).
mp: 280-283°
Mass Spectrum: m/e = 324 ($M^+$)

Example 2

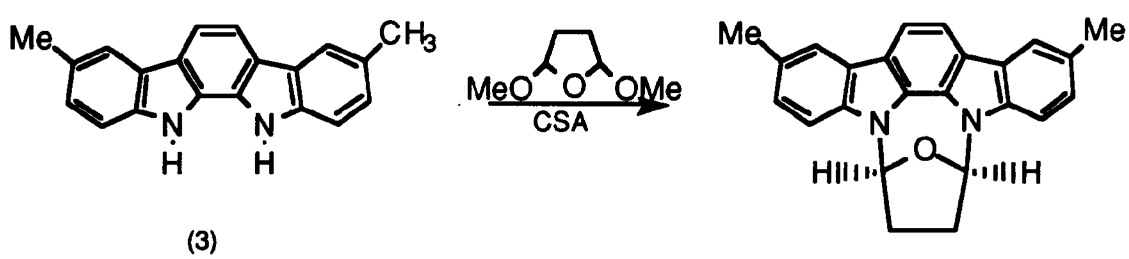


(3)

(4)

A mixture of 3,8-dimethyl indolocarbonazole (1.0 g), 2,5-dimethoxytetrahydrofuran (3.0 g) and camphorsulfonic acid (0.4 g) in dichloromethane (30 mL) was stirred at room temperature for 5 hours. The mixture was diluted with dichloromethane, washed with water, dried ($MgSO_4$), evaporated, and the product isolated by silica gel chromatography, eluting with dichloromethane-hexane, and crystallized from ether-hexane as a white powder (0.92 g).
mp: 268-720°
Mass Spectrum: m/e = 352 ($M^+$)

Example 3

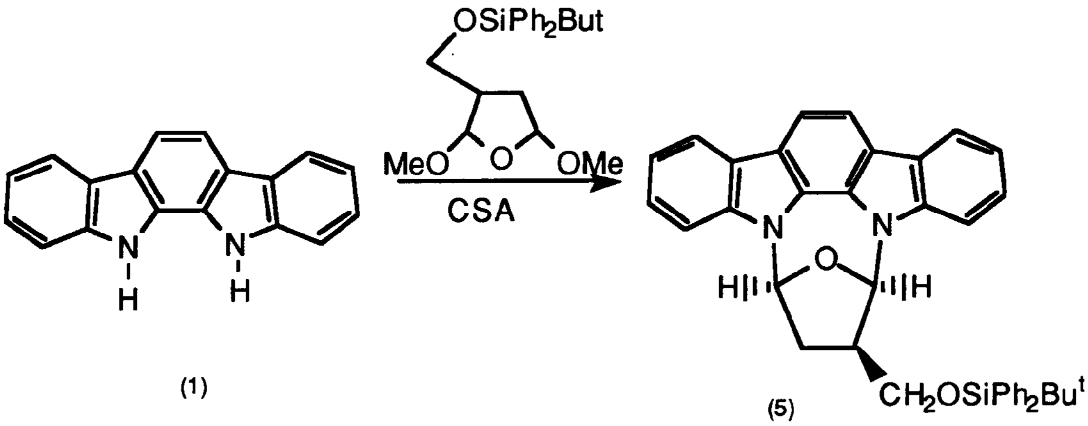


(1)

(5)

and

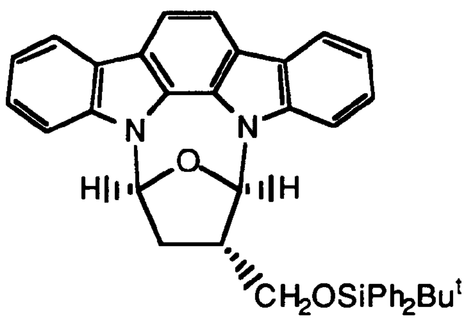

(6)

A mixture of indolocarbozole (7.0 g), 2,5-dimethoxy-5-(tert-butyldiphenyl silyloxymethyl)-tetrahydrofuran (34.2 g) and camphorsulfonic acid (1.15 g) in dichloromethane (250 mL) was stirred at room temperature for 48 hours. The dark solution was washed with water and the organic phase was filtered through a pad of silica gel, washing with dichloromethane. The eluates were evaporated, and the residue subjected to flash silica gel chromatography, eluting with dichloromethane-hexane mixtures. The first-eluted compound was the α-hydroxymethyl isomer (6) [5.0g], crystallized from ether-hexanes

mp: 150 - 153°

Mass Spec.: m/e = 592 ($M^+$)

later fractions gave the β-isomer (5) [6.0 g]

mp: 159-162°

Mass Spec.: m/e = 592 ($M^+$)

Example 4

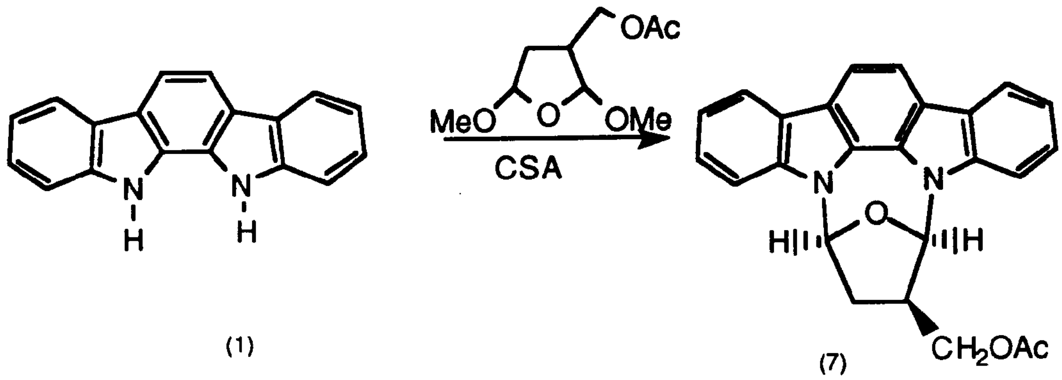


(1) (7)

and

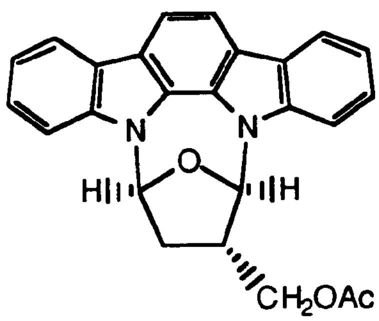

(8)

Following the procedure of Example 3, indolocarbazole (1.0 gm) was reacted with 2,5-dimethoxy-3-(acetoxymethyl)-tetrahydrofuran (3.5 gm) and camphorsulfonic acid (0.25 gm) in dichloromethane (20 mL). Final separation of the isomers was performed using hexane-ethyl acetate mixtures in silica gel flash chromatography.

The less polar compound (0.25 gm) was the α-acetoxymethyl compound (8) crystallized from dichloromethane-hexanes.
mp: 249-254°.
Mass Spec.: m/e = 396 ($M^+$).
The more polar compound (0.52 gm) was the β-isomer (7).
mp: 224-228°. Mass Spec.: m/e 396($M^+$)

Example 5

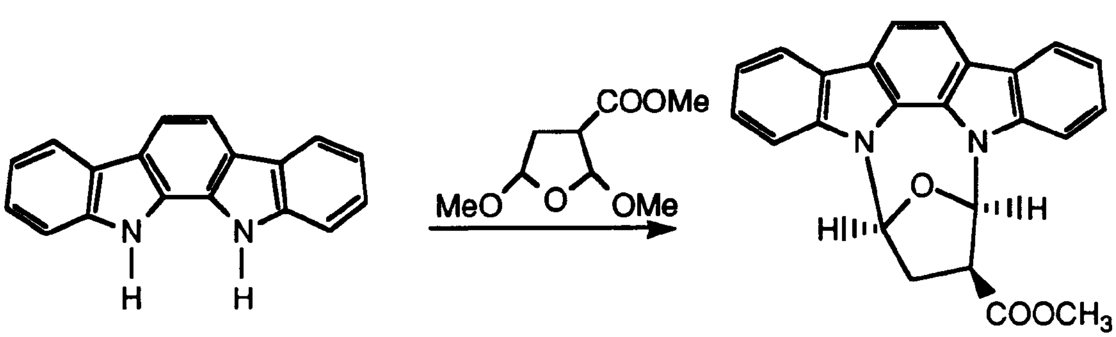

(1) (9)

To a dichloromethane (50 mL) solution containing (1) (1.0 g) and 2,5-dimethoxy-3-carbomethoxy tetrahydrofuran (3.7 g) was added camphorsulfonic acid (0.1 g). The resulting mixture was stirred under $N_2$ for 3 days. The solvent was removed under reduced pressure and then the crude was redissolved in ethyl acetate (60 mL) and washed with (2 x 20 mL) saturated sodium bicarbonate solution and brine (1 x 20 mL). The organic portion was dried ($MgSO_4$) and the solvent removed. The residue was subjected to silica-gel flash chromatography. The relevant fractions were pooled and concentrated to provide 0.52 g of product 9, an amorphous solid
Mass Spectrum: m/e=382 ($M^+$)

Example 6

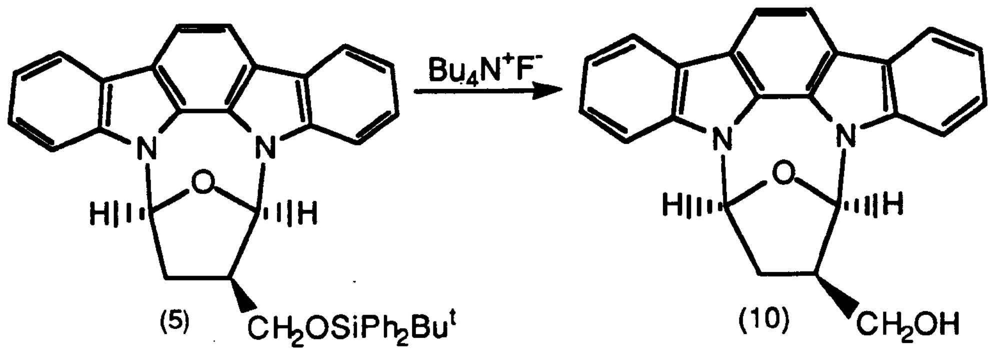

(5) (10)

A solution of compound (5) [Example 3] (5.1 g) and tetra-n-butylammonium fluoride trihydrate (2.8 g) in DMF (30 mL) was stirred at room temperature 1 hour, then diluted with water (200 mL). The products were extracted into dichloromethane, the solution washed with water, dried ($MgSO_4$) and evaporated. Silica gel chromatography (10% ether-dichloromethane) gave the product (10) as a white powder (2.25 g)
mp: 219-223°
Mass Spectrum: m/e = 354 ($M^+$)

Example 7

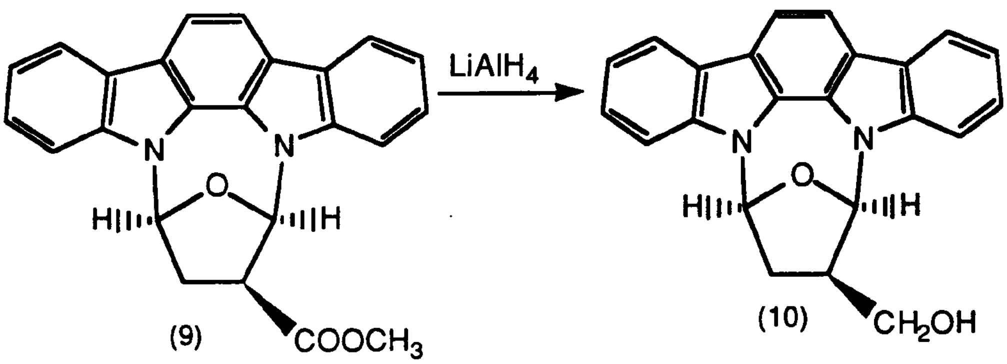

(9) (10)

A solution of compound (9) [Example 5] (0.15 gm) was stirred in dry THF (15 mL) and lithium aluminum hydride (0.1 gm) added. After 4 hours, the mixture was added to water, extracted with dichloromethane, and the extracts dried and evaporated. Chromatography on silica gel with etherdichloromethane gave (10); identical with material made by the procedure of Example 6.

Example 8

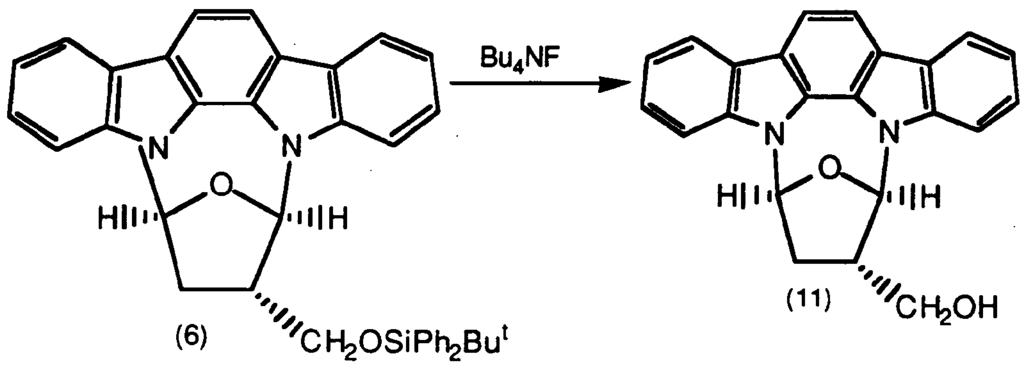


(6) (11)

The procedure of Example 6 was followed using compound (6) [Example 3] (5.0 g). Compound (11) was obtained as a white powder (2.2 g).
mp: 228-231°.
Mass Spectrum: m/e = 354 ($M^+$).

Example 9

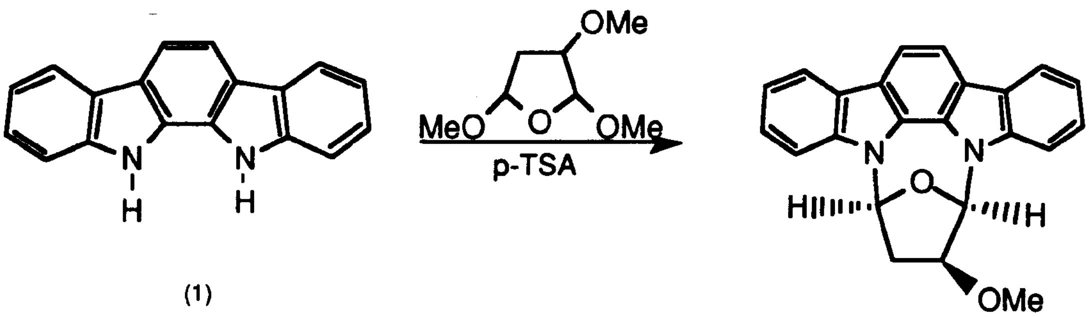


(1)

(12)

and

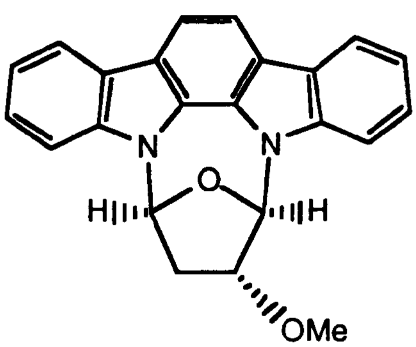

(13)

A mixture of indolocarbazole (0.26 g) 2,3,5-trimethoxytetrahydrofuran (0.6 g), dichloromethane (8 mL) and methanol (0.1 mL) was stirred for 24 hours at room temperature with p-toluenesulfonic acid (0.125 g). The solution was washed with water and evaporated, and the products separated by preparative thin-layer chromatography on silica gel, eluting with 3:1 dichloromethane: hexane. The least polar component (0.12 g) was the β-methoxy isomer (12), a white solid.
mp 235-237°

Mass Spectrum: m/e = 354 ($M^+$).

The mole polar isomer was the α-methoxycompound (13), obtained as an amorphous solid (0.06 g).
mp: indefinite.
Mass Spectrum (high resolution): Found = 354.1369. $C_{23}H_{18}N_2O_2$:
Calculated = 354.1368.

Example 10

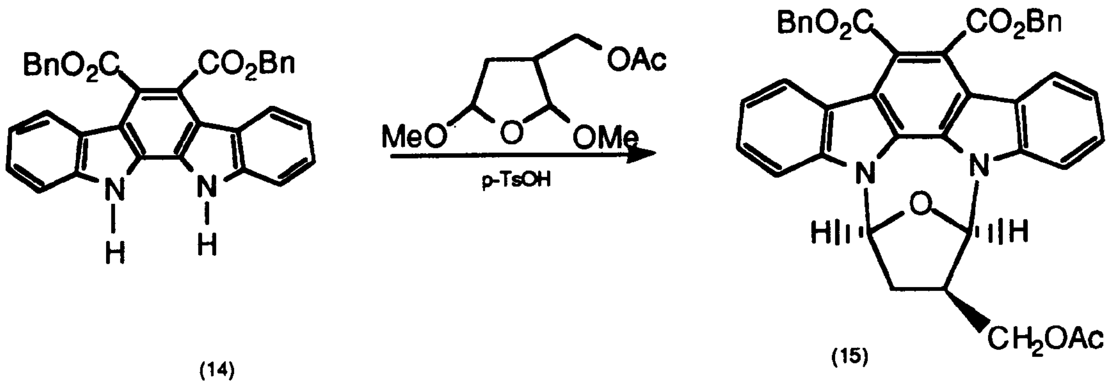


(14) (15)

To a suspension of (14) (0.131g) in dichloromethane (10 mL) was added 2,5-dimethoxy-5-acetoxymethyltetrahydrofuran (0.102 g; 0.005 mole) and 0.01 g of p-toluene sulfonic acid. The resulting mixture was stirred for 2 hours at room temperature and then refluxed for an additional 2 hours. After cooling, the reaction was diluted with 30 mL dichloromethane and washed with 2 x 15 mL saturated aqueous sodium bicarbonate and 2 x 15 mL brine. The organics were dried ($MgSO_4$) was isolated by silica-gel prep-plate chromatography in 3:7 ethyl acetate: hexane solvent system to give the product (15) (0.07g) as a foam. Bn in the above example and throughout the specification means benzyl.
Mass Spectrum: m/e=665 ($MH^+$)

Example 11

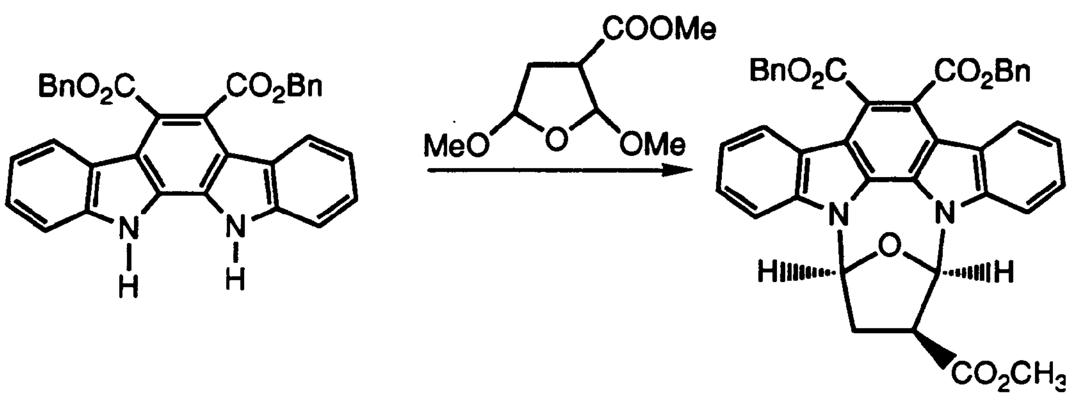

(14)

(16)

To a dry dichloromethane (10 mL) suspension of (14) (0.1 g), was added 2,5-dimethoxy-5-carbomethoxytetrahydrofuran (0.2 g) and p-tolunesulfonic acid (0.01 g). The suspension immediately turned into a clear dark solution. This mixture was stirred for 24 hours then the solvent was removed. The residue was redissolved in ethyl acetate (50 mL) and washed in sequence with saturated aqueous $Na_2CO_3$ ( 2 x 20 mL) and brine (25 mL). The organics were dried ($MagSO_4$), concentrated and the product was isolated by silica-gel prep. plate chromatography to provide 0.06 g of pure product, as a white foam.
Mass Spectrum: m/e=651 ($MH^+$).
FAB MS: $[M+1]^+$ = 651;; $[M]^+$=650.

Example 12

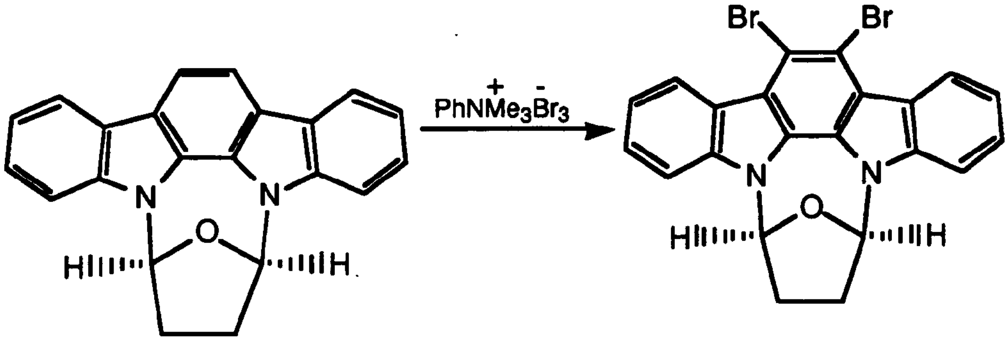


(17)

(2)

Compound (2) [Example 1] (1.00 g) was stirred at room temperature in dimethylformamide (DMF) (50 mL) and phenyltrimethylammonium tribromide (PTAB) (2.37 g was added in portions). After stirring for 2 hours, water (50 mL) was added slowly. The product was filtered, washed thoroughly with water and dried at 80° in high vacuum to give (17) as a white powder (1.43 gm). A sample was recrystallized from ethyl acetate.
mp: 222-224° (decomposes).
Mass Spectrum: m/e = 482/483/484 ($M^+$).

Example 13

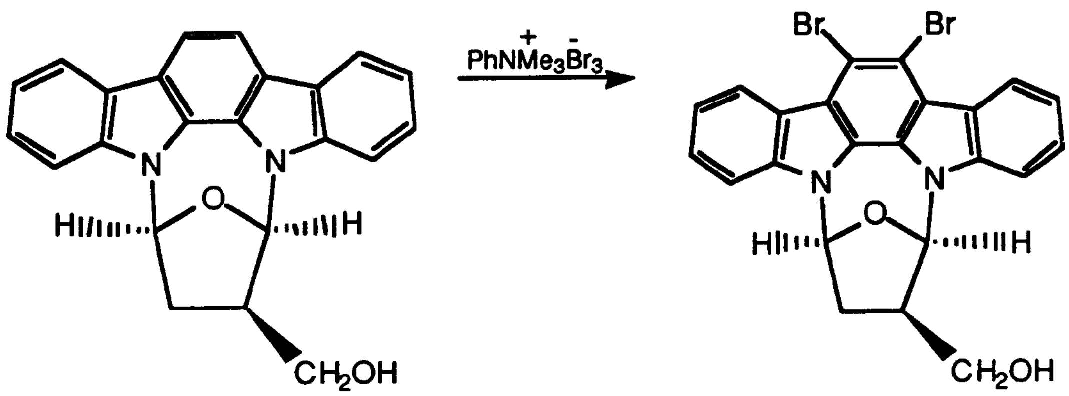


(18)

(10)

Following the procedures of Example 12, compound (10) [0.70 gm, Example 6], was brominated with phenyltrimethylammonium tribromide (PTAB) (1.5 gm) in DMF (25mL) to yield (18) as a white powder (1.1 gm), which was estimated to contain ~90% of the dibromocompound by PMR spectroscopy, and was used directly in the next step.

Example 14

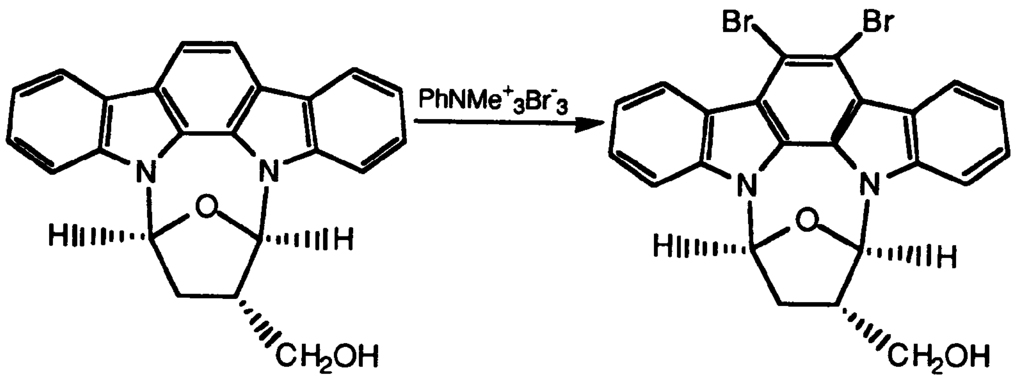


(11) (19)

Following the procedure of Example 12, compound (11) [0 .70 gm; Example 8] was brominated with phenyltrimethylammonium tribromide (PTAB) ( 1.5 gm) in DMF (25 mL) . The crude dibromocompound (18) was obtained as a white powder
(1.1 gm), and used directly in the next step.
mp: 218-220° (decomp.).

Example 15

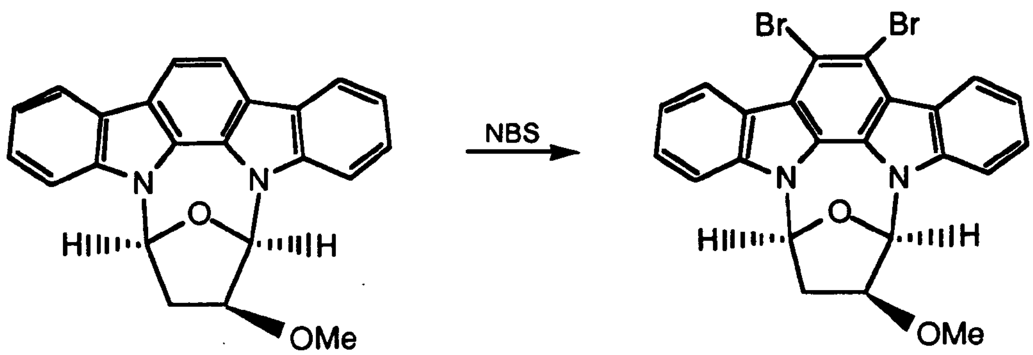


(12) (20)

A solution of compound (12) [0.35 gm; Example 9] was stirred at room temperature in DMF (20 mL) and N-bromosuccinimide (NBS) (0.36 g) was added. The mixture was stirred for 24 hours, water (20 mL) was added, and the solid was collected, washed with water and dried to a white powder (0.51 g) at 80° in high vacuum. This product was used directly in the next step, without purification.

Example 16

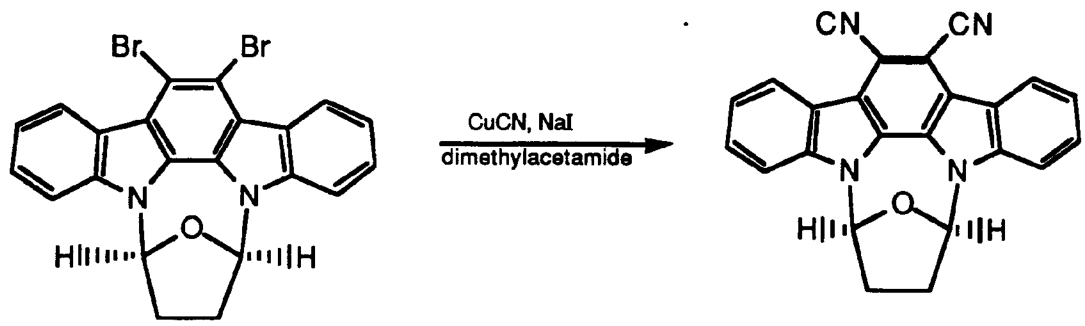


(17) (21)

A mixture of dibromocompound (17) [0.50 g; Example 12], copper (I) cyanide (1.0 g), sodium iodide (0.3 g) and N,N-dimethylacetamide (DMA; 15 ml) was heated and stirred at 180-190° for 6 hours. After cooling, the mixture was added to a stirred solution of ethylene diamine (5 mL) in water (75 mL), stirred 0.5 hour, and the crude product filtered, washed with water and dried in high vacuum. The solid was subjected to extraction with refluxing dichloromethane-methanol (20:1 v/v) in a Soxhlet apparatus for 3 days. The suspension was evaporated, and the residue triturated with a dichloromethane and filtered and dried at 80° to give the product (21) (0.29 g) as a pale yellow solid
mp >325°.
Mass Spec.: m/e = 374 ($M^+$).

Example 17

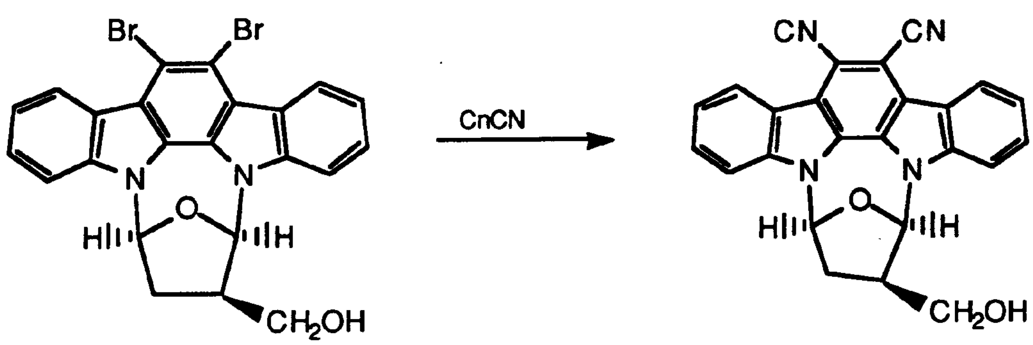


(22)

(18)

Following the procedure of Example 16, dibromocompound (18) [0.80 gm; Example 13] was converted to the corresponding dicyanocompound (22) (0.51 gm), which in turn was isolated by Soxhlet extraction with acetone.
mp: >325° (decomposes).
Mass Spec.: m/e = 404 ($M^+$).

Example 18

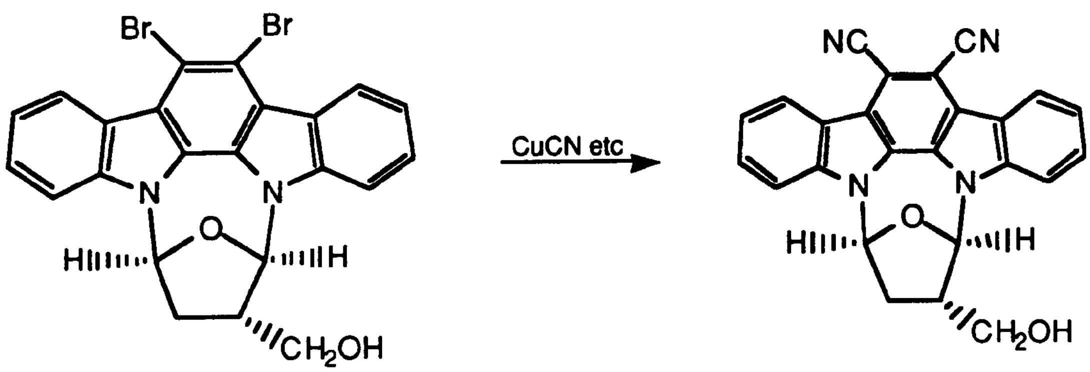


(19) (23)

Following the procedure of Example 16, dibromocompound (19) [3.30 gm; Example 14] was converted to the corresponding dicyanocompound (23) (2.01 gm), an off-white powder.
mp: 334-340° (with decomposition).
Mass Spec.: m/e = 404 ($M^+$).

Example 19

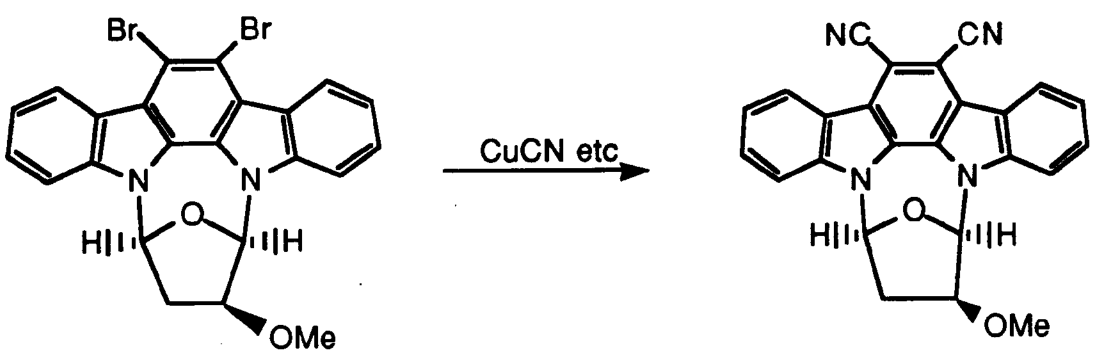


(24)*

(20)

Following the procedure of Example 16, compound (20) [0.48 gm; Example 15] was converted to the corresponding dicyanocompound (24). The final product was isolated by silica gel column chromatography and obtained as a pale yellow solid ) (0.14 gm).
mp: >350°.
Mass Spec.: m/e = 404 ($M^+$).

Example 20

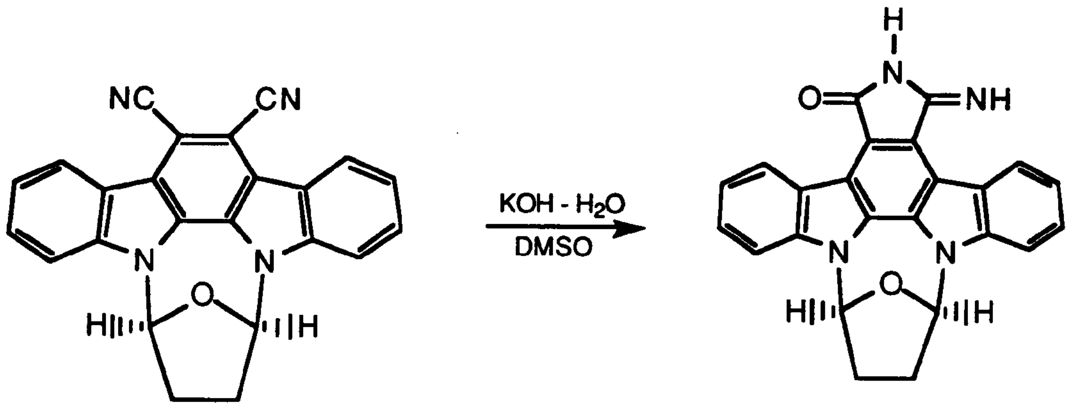


(21) (25)

The dinitrile (21) [0.35 g; Example 16] was heated and stirred for 1.25 hours at 120° in a mixture of DMSO (12 mL) and aqueous KOH (1 g KOH in 1.5 mL $H_2O$). Water (50 mL) was added, the mixture stirred without heating for 0.5 hours, and the product filtered, washed thoroughly with water and dried at 80° in high vacuum to a yellow powder (25) (0.34 g).
mp: 198-202°.
Mass Spec.: m/e = 392 ($M^+$).

Example 21

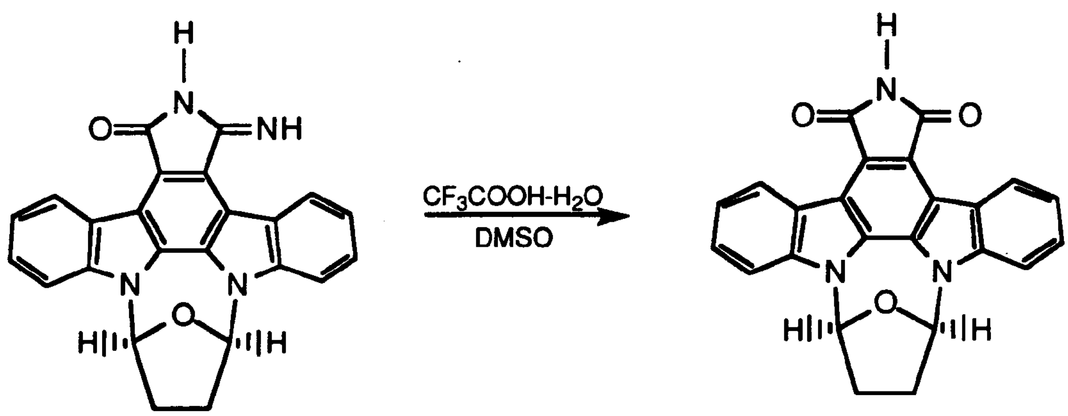


(25) (26)

A solution of compound (25) [0.25 g; Example 20] in DMSO (15 mL) was stirred for 4 hours at room temperature with the addition of water (0.25 mL) and trifluoroacetic acid (0.25 mL). Water (20 mL) was added dropwise with stirring; the precipitate was collected and washed well with water and dried at 80° in high vacuum to give the imide (26) (0.23 gm) as a bright yellow powder.
mp: >325°.
Mass Spec.: m/e = 393 ($M^+$).

Example 22

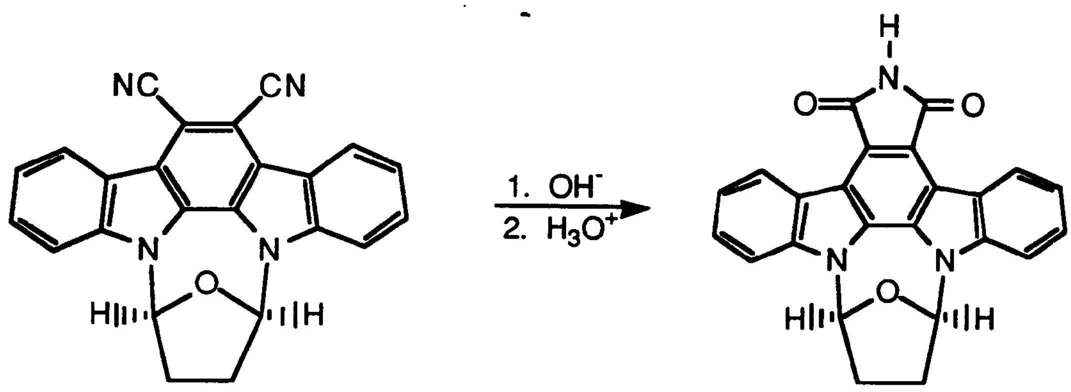


(21) (26)

This reaction was carried out in one reaction vessel.

Dicyanocompound (21) [0.65 g; Example 16] was heated at 150° for 1 hour in DMSO (30 mL), $H_2O$ (1.5 mL) and KOH (1 g). After cooling to room temperature, trifluoroacetic acid (1.5 mL) was added, and the mixture stirred for 2 hours. Water (60 mL) was added slowly, stirring continued for 0.35 hour, and the product was collected, washed with water, and dried to afford 0.66 g, identical with material (26) from the foregoing example.

Example 23

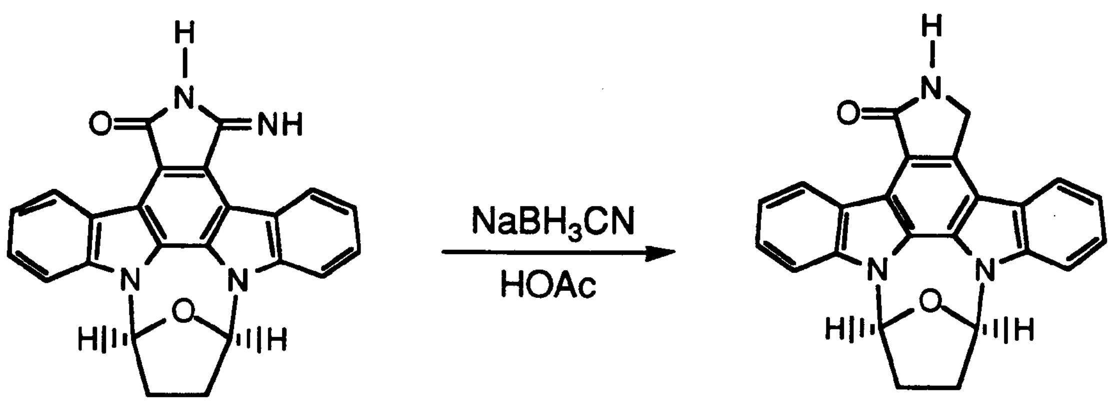


(25) (27)

Compound (25) [0.15 g; Example 20] was heated at 90-100° in acetic acid (5 mL) and sodium cyanoborohydride (0.20 g) was added in 4 portions over 2-3 minutes. After heating for 10 minutes, water was added, the precipitate was collected, washed with water and dried at 80° in vacuum to give the lactam (0.12 g) as a cream powder.

mp: >325°.

Mass Spectrum: m/e - 379 ($M^+$).

Example 24

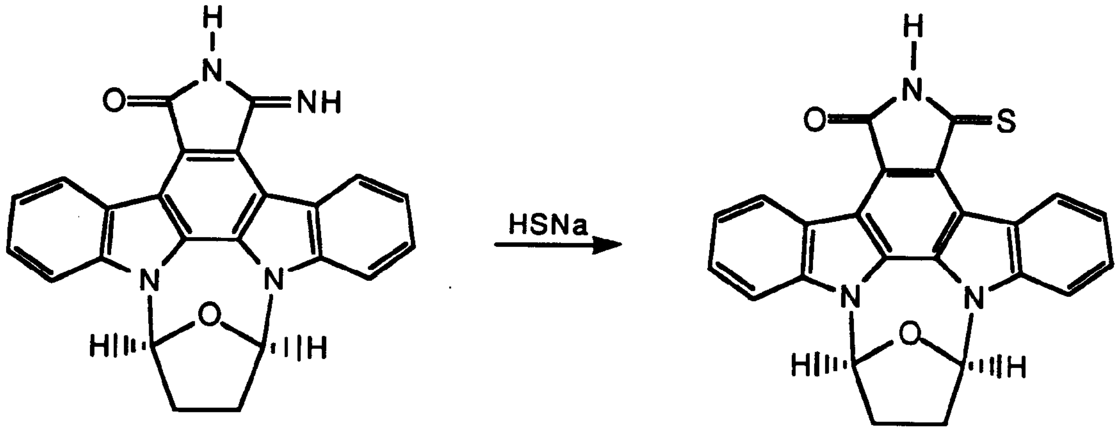


(25) (28)

The iminocompound (25) [0.05 g; Example 20] was stirred for 20 hours at room temperature in DMSO (3 mL) with powdered sodium hydrogen sulfide (0.25 g) and acetic acid (2 drops). The mixture was diluted with water and extracted with dichloromethane, and the extract was chromatographed on silica gel with dichloromethane. The product (28) eluted as a brown band, orange in solution. Evaporation and crystallization with dichloromethane-hexanes gave a bright red powder (0.003 g)
mp: decomposed above 250°.
Mass Spectrum: m/e = 409 ($M^+$).

Example 25

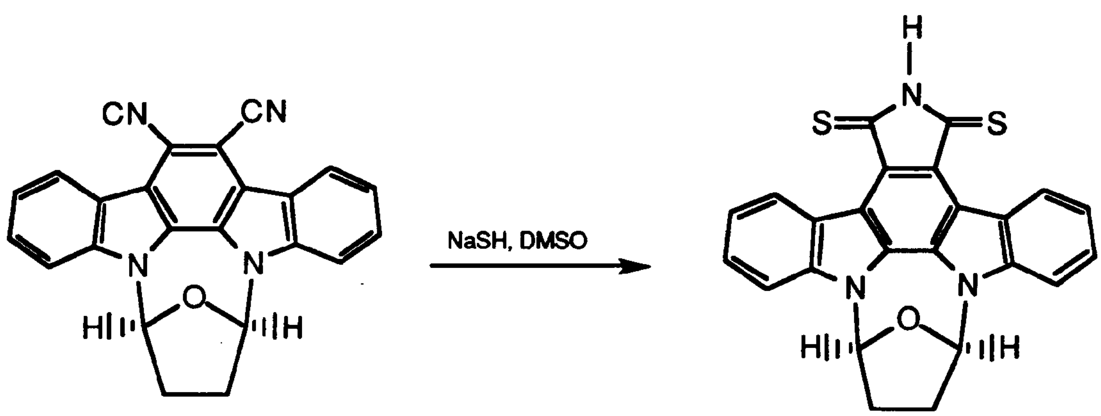


(21) (29)

Dicyanocompound (21) [0.05 g; Example 16] was heated at 150° for 10 minutes in DMSO (4 ml) with sodium hydrogen sulfide (0.2 g). The mixture was diluted with $H_2O$ and extracted with dichloromethane, and the extract washed ($H_2O$) and chromatographed on deactivated (Grade III) neutral alumina, eluting with dichloromethane.

The purple band was collected, evaporated, and the residue triturated in hexanes and vacuum dried to give the dithioimide as a black powder (29) (0.01 g)
mp: decomposed at 300-310°.
Mass spectrum: m/e'425 ($M^+$).

Example 26

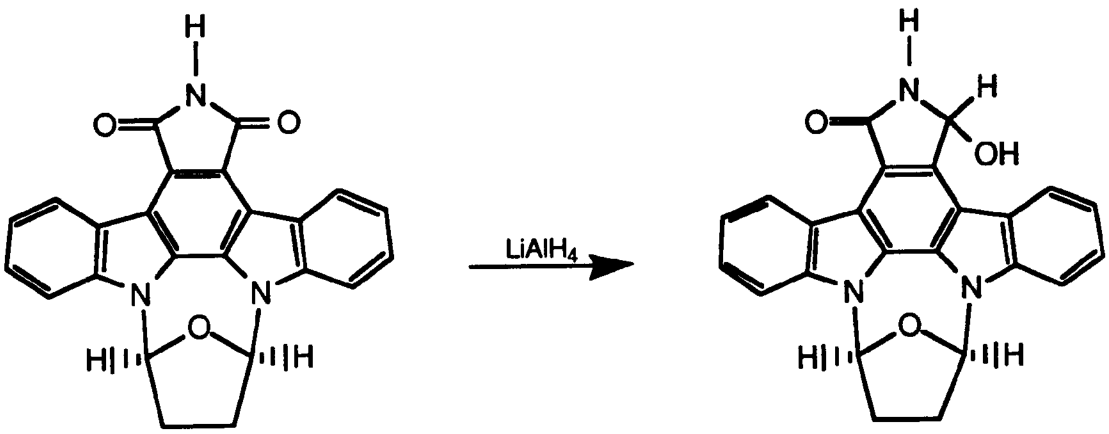


(26) (30)

The imide (26) [0.05 g; Example 22] was stirred at room temperature in anhydrous THF (5 mL) and 1 M lithium aluminum hydride-ether (1.0 mL) added. After 0.75 hours, acetic acid (0.2 mL) was added dropwise, and the reaction worked up in water-dichloromethane. The extract was dried and evaporated and the residue triturated with 1:2 dichloromethane: ether, filtered and dried to an off-white powder (30) (0.025 g).
mp: >325° (decomposes)
Mass Spec: M/e = 395 ($M^+$).

Example 27

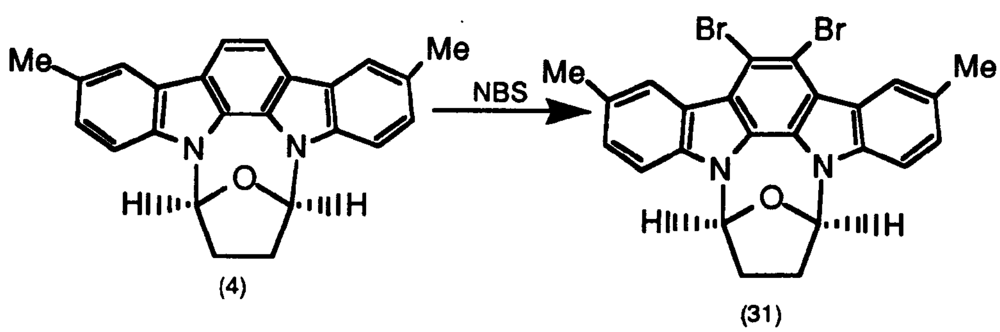


(4) (31)

The dimethyl compound (4) [0.53 gm; Example 2] was stirred for 24 hours at room temperature in DMF (150 mL) with N-bromo-succinimide (0.72 g). Water (300 mL) was added, and the solid filtered off, washed with water and dried at 80° in vacuum to a pale greenish powder (3) (0.71 g).
mp: Decomposes above 250°.
Mass Spectrum: m/e = 510/511/512 ($M^+$).

Example 28

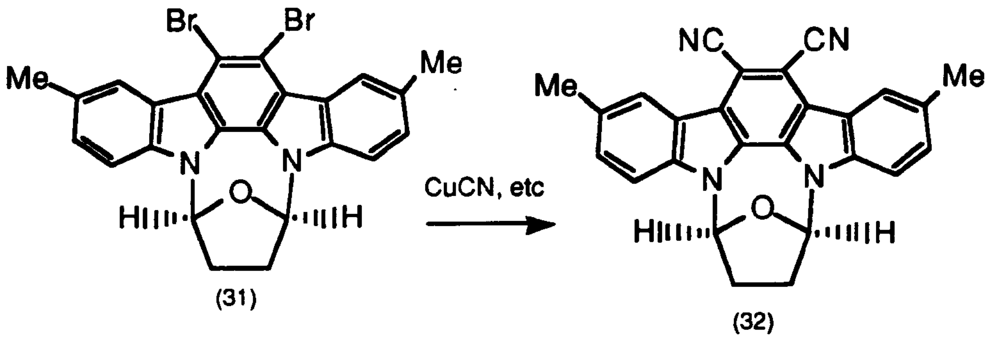


(31) (32)

The dibromocompound (31) [0.64 g; Example 27] was stirred and heated for 20 hours at 180° in DMAC (10 mL), copper (I) cyanide (0.6g) and sodium iodide (0.2 g). The mixture was added to ethylenediamine (5 mL) in water (100 mL), and stirred for 1 hour at room temperature. The crude product was collected and washed well with water, then methanol, and dried at 80° to a pale brown powder (0.48 g), sparingly soluble in all solvents.
Mp: >350°.
Mass Spectrum: m/e = 402 ($M^+$).

Example 29

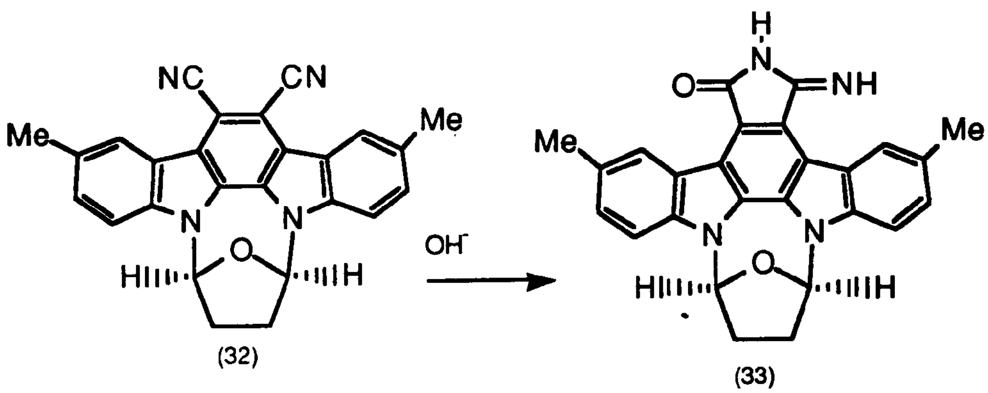


(32) (33)

The dicyanocompound (32) [0.45g; Example 28] was heated at 125° for 1.5 hours in dimethylsulfoxide (DMSO) (15 mL) with potassium hydroxide (1 g) in water (1.5 mL). Water (50 mL) was added slowly, and the solid was collected by filtration, washed with excess water, then with methanol and ether, and dried at 80° to a bright yellow powder (33) (0.41 g).
mp: >350°.
Mass Spec.: m/e = 420 ($M^+$).

Example 30

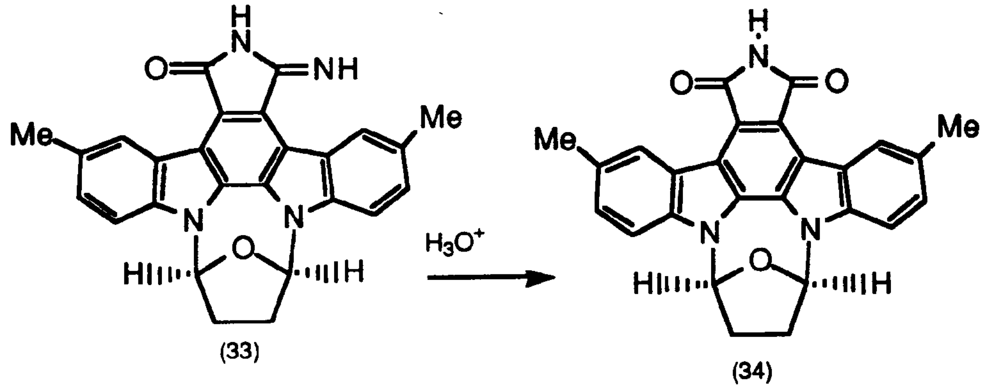


(33) (34)

A solution of the imino compound (33) [0.15 g; Example 29] in DMSO (12 mL), $H_2O$ (0.4 mL) and trifluoroacetic acid (0.2 mL) was stirred for 20 hours at room temperature, then diluted with water. The product was filtered, washed well with water and dried at 80° to a yellow powder (34) (0. 12 g).
m.p. >350°.
Mass Spec.: m/e = 421 ($M^+$).

Example 31

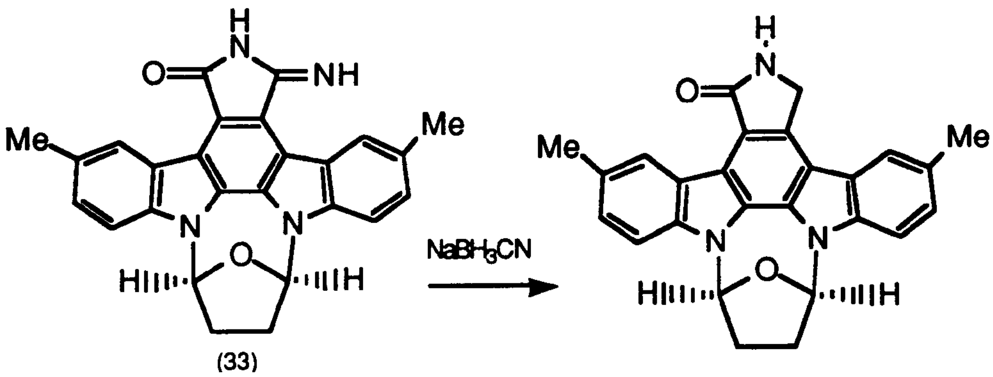


(33)

A solution of (33) [0.10g; Example 29] in acetic acid (7 mL) was stirred at 90-100° and sodium cyanoborohydride (0.15 gm) was added in three portions over 2 minutes. After a further 5-10 minutes, the product was precipitated with water, filtered, washed with water and dried at 80° to a buff powder (0.095 g).
mp: 342-346°, with decomposition.
Mass Spec.: m/e = 407 ($M^+$).

Example 32

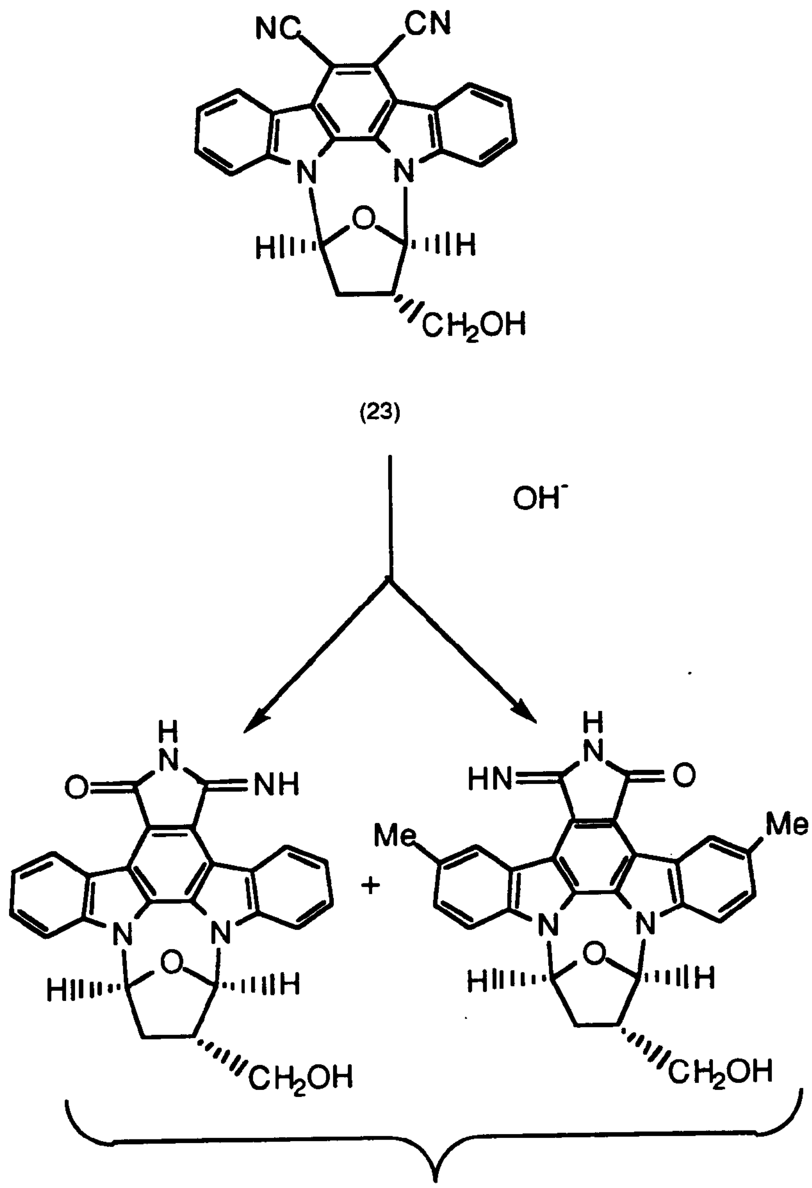


(23)

(36)

The dicyanocompound (23) [0.20 g; Example 18] was heated at 100-120° for 1 hour in DMSO (10 mL) with KOH (0.8 g) in $H_2O$ (1.2 mL), cooled, and $H_2O$ (25 mL) added dropwise. The precipitate was collected, washed well with water, and dried at 80° in vacuum to give the mixture of isomeric iminocompounds (36) as a yellow powder mp: 195-208°, with decomposition. Mass Spec.: m/e = 422 ($M^+$).

Example 33

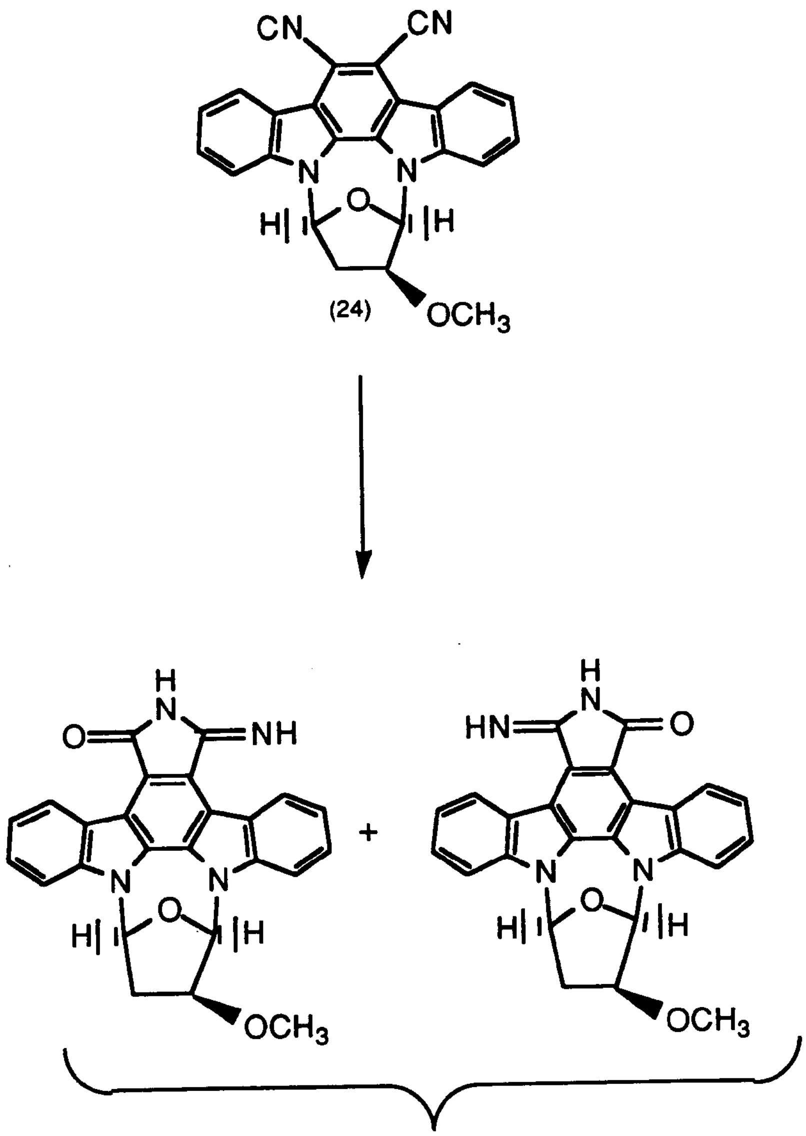

(24)

+

(37)

Dicyanocompound (24) [0.25 gm; Example 19] was hydrolyzed according to the procedure of Example 37, to give the iminoimide mixture (37) (0.22 gm), a yellow solid.
mp: decomposes at 220-230°.
Mass Spec.: m/e - 422 ($M^+$).

Example 34

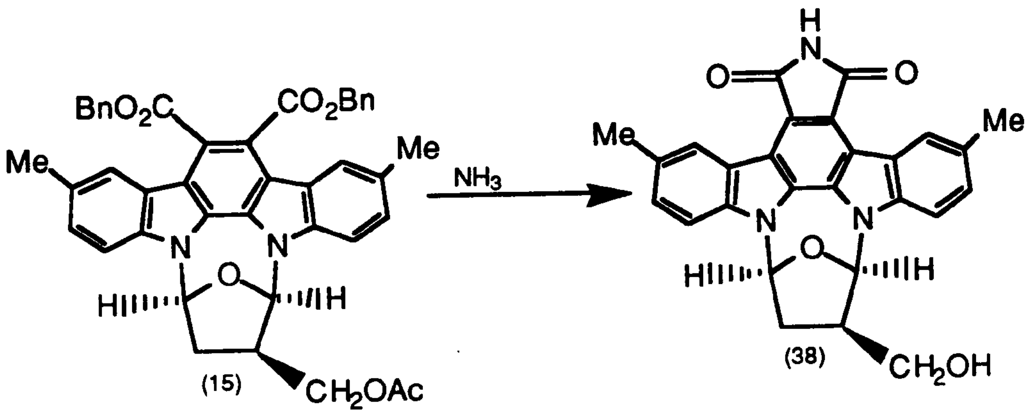


(15) (38)

The dibenzylester (15) [0.04 gm; Example 10] was heated for 4 hours at 120° in a mixture of DMSO (0.5 mL) and concentrated aqueous ammonia (0.5 mL). After cooling and diluting with water, the product was extracted with ethyl acetate, the extract evaporated, and the residue purified by chromatography on silica gel in hexanes-ethyl acetate to give the imide as a yellow powder (38) (0.02 gm).
mp: 215-220° with decomposition.
Mass Spec.: m/e = 423 ($M^+$).

Example 35

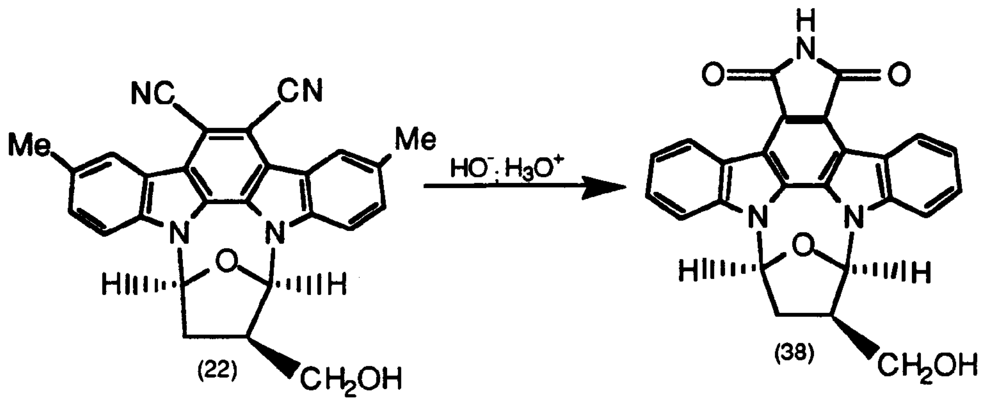


(22) (38)

The dicyanocompound (22) [1.00 gm; Example 17] was heated at 150°C for 1.5 hours in DMSO (30 mL) and KOH (2.7 g) in $H_2O$ (7 mL). After cooling, trifluoroacetic acid (3.3 mL) was added, and the mixture stirred overnight at room temperature. Water (100 mL) was added, and the product filtered, washed well with water and dried at 80° in vacuum to afford 0.74 gm, of (38), identical with material from the preceding example.

Example 36

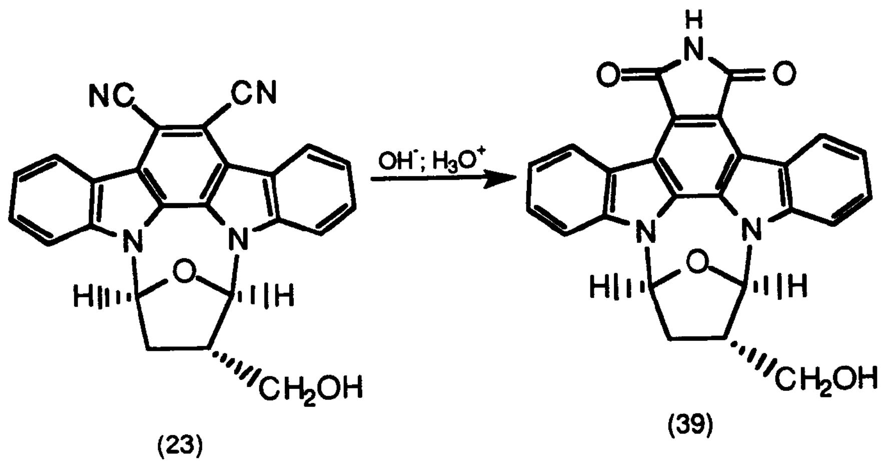


(23) (39)

Following the procedure of Example 35, a mixture of the dicyanocompound (0.65 gm; Example 18), DMSO (30 mL) and KOH (1.0 gm) in $H_2O$ (1.5 mL) was heated at 100°C for 3/4 hour, cooled, and stirred at room temperature for an hour with the addition of trifluoroacetic acid (2.5 mL). The product was precipitated, washed and dried to a yellow powder (39) (0.60 gm).
mp: 330-335° with decomposition.
Mass Spectrum: m/e = 423 ($M^+$).

Example 37

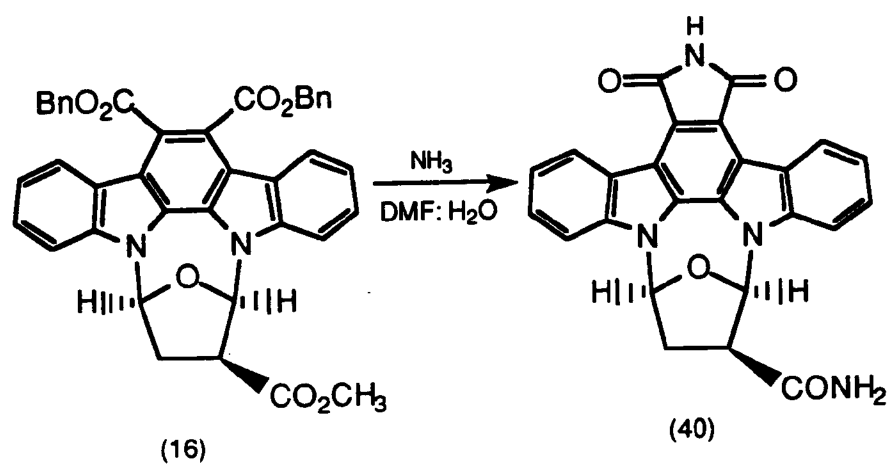


(16) (40)

Triester (16) [0.04 gm; Example 11] was heated at 120° for 12 hours in DMF (2 mL) and concentrated aqueous ammonia (2 mL), in a sealed reaction vial. After cooling to room temperature the reaction was diluted with ethyl acetate (25 mL) and washed with water (2 x 10 mL) and brine (1 x 15 mL) and then dried (Mg $SO_4$) and concentrated to yield a crude residue. The product (40) was isolated by preparative thin-layer, silica-gel chromatography.
Mass Spec.: FAB [$M^+$] = 436.
mp: >350°C.

Example 38

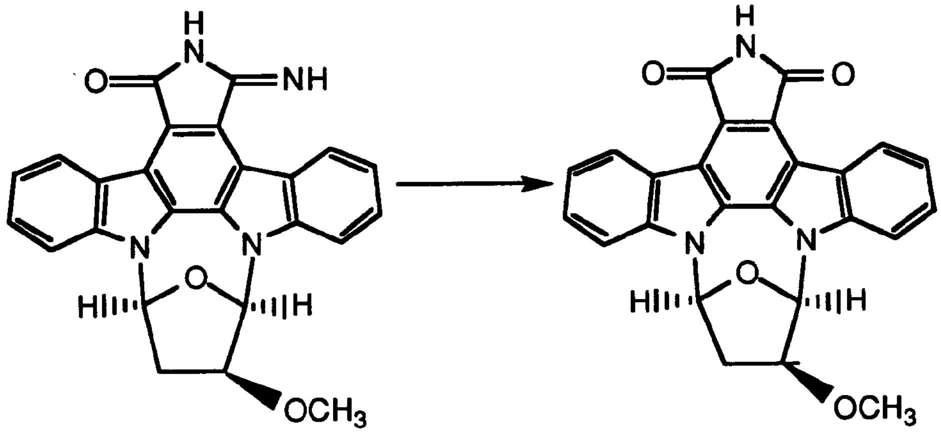

(37) (41)

Iminoimide (37) [0.09 gm; Example 33] in DMSO (4 mL) was stirred for 2.5 hours at room temperature with water (0.2 mL) and trifluoroacetic acid (0.1 mL), then diluted with water (20 mL). The precipitate was filtered and washed with water, then dried at 80° in vacuum to give the imide as a bright yellow powder (41 ) (0.06 gm).
mp: >350°.
Mass Spectrum: m/e 423 ($M^+$).

Example 39

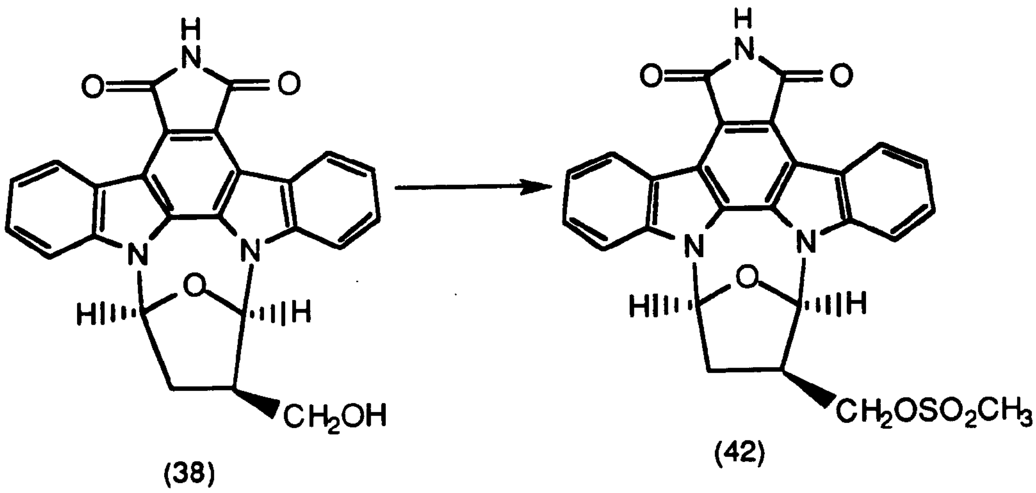

(38) (42)

The alcohol (38) [0.15 g m; Examples 34/35] was stirred with methanesulfonyl chloride (0.21 mL) and diisopropylethylamine (0.3 mL) in THF ( 15 mL) at room temperature for an hour. After workup in ethylacetate-water, the product was isolated by chromatography on silica gel, eluting with acetone-dichloromethane, to give the mesylate as a yellow amorphous solid (42) (0.10 gm).
mp: decomposes at 200°.
Mass Spectrum: m/e = 501 ($M^+$).

Example 40

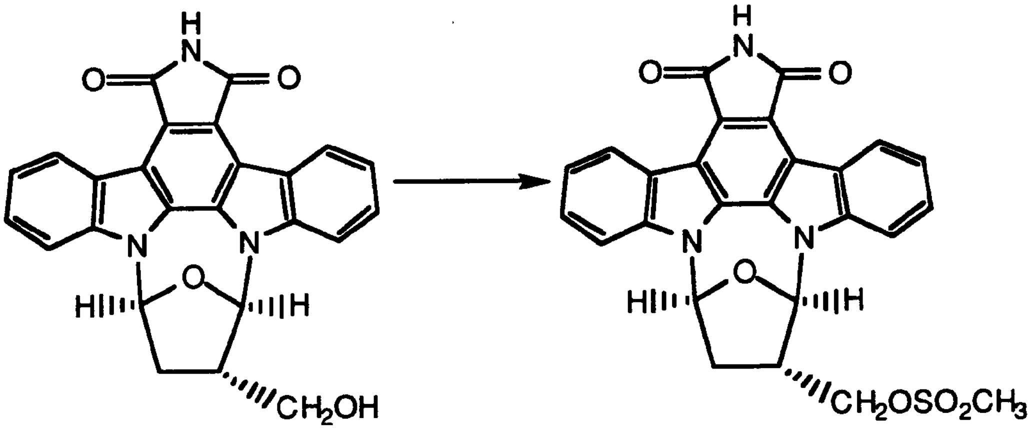

(39) (43)

Following the procedure of Example 39, compound (39) [0.15 gm; Example 36] was converted to the corresponding mesylate (43) (0.11 gm), a yellow solid.
mp: 173-180°, with decomposition.
Mass Spectrum: m/e = 501 ($M^+$).

Example 41

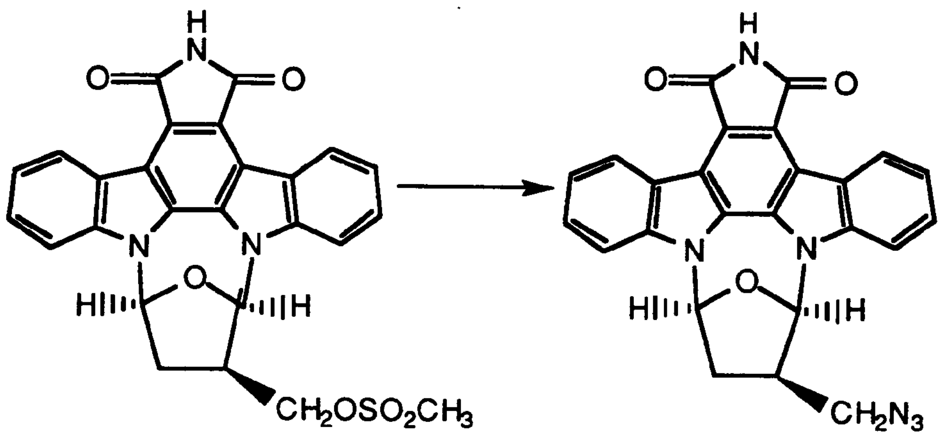

(42) (44)

Mesylate (42) (0.473 g) and $NaN_3$ (1.123 g) in DMF (20 mL) was heated to 90°C and stirred for 6 hours. The reaction was cooled and diluted with ethyl acetate (100 mL) and washed with water and brine. The organics were dried ($MgSO_4$), concentrated to provide a crude residue which was subjected to silica-gel flash chromatography (20% acetone/hexane) to afford (44) (0.25 g) of pure product.
M.S.:FAB [M+] = 448.
m.p$^+$: decomp + 300°C.

Example 42

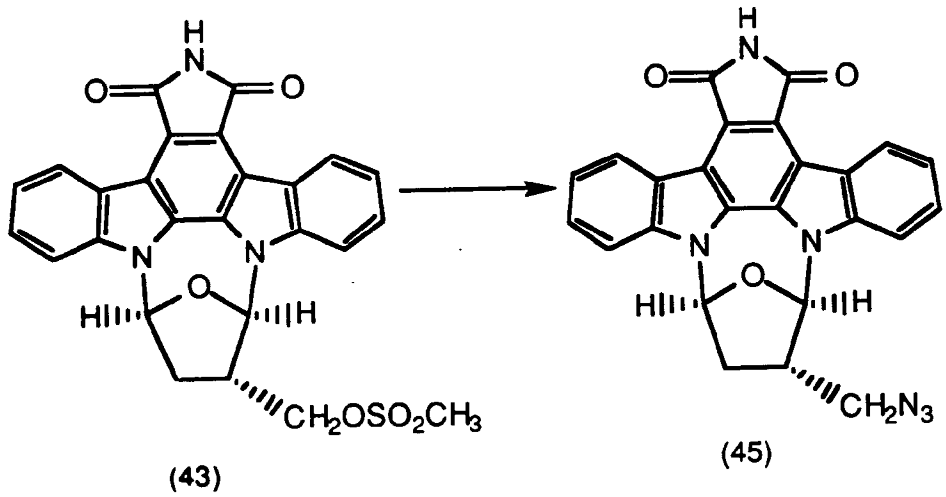

(43) (45)

A solution of the methanesulfonate (43) (0.15 gm; Example 40) and lithium azide (0.10 gm) in DMSO (15 mL) was heated at 60-70°C for 24 hours. The mixture was partitioned in ethyl acetate-water and the organic phase washed with water, dried and evaporated. The residue was triturated in ether, filtered and dried in high vacuum at 25° to give the azido compound (45) (0.12 gm) as a yellow, amorphous powder.
mp: decomposes at 200°C.
Mass Spectrum: m/e = 448 ($M^+$).

Example 43

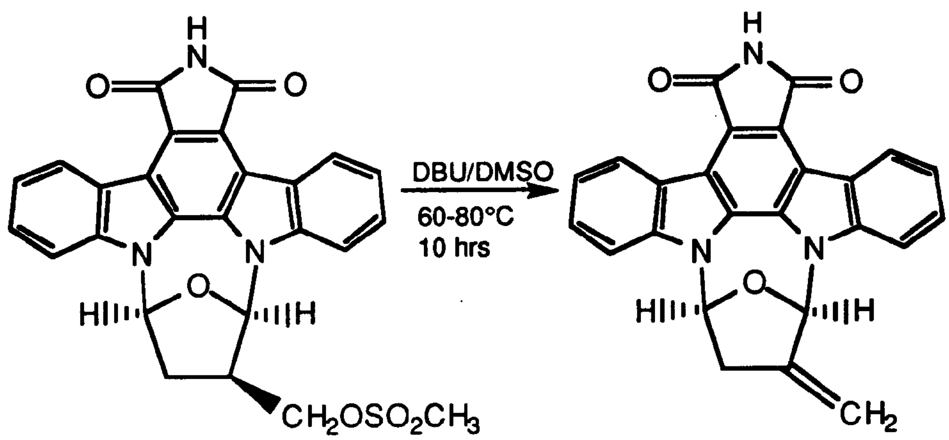


(42) (47)

A mixture of mesylate (42) [0.59g; 0.00118 mole] and DBU [0.36g, 2eq.] om 20 mL DMSO was heated to 60-80°C and stirred for 10 hours. After cooling the reaction was diluted with ethyl acetate (100mL) and washed with dilute HCl (2 x 25 mL: IN), water, and brine. The organic portion was dried ($MgSO_4$) and concentrated. The product (47) was isolated by silica-gel column chromatography (15% acetone/hexane).
Mass Spec.: (1[$M^+$] = 405.
mp: decomp. 250°C.

Example 44

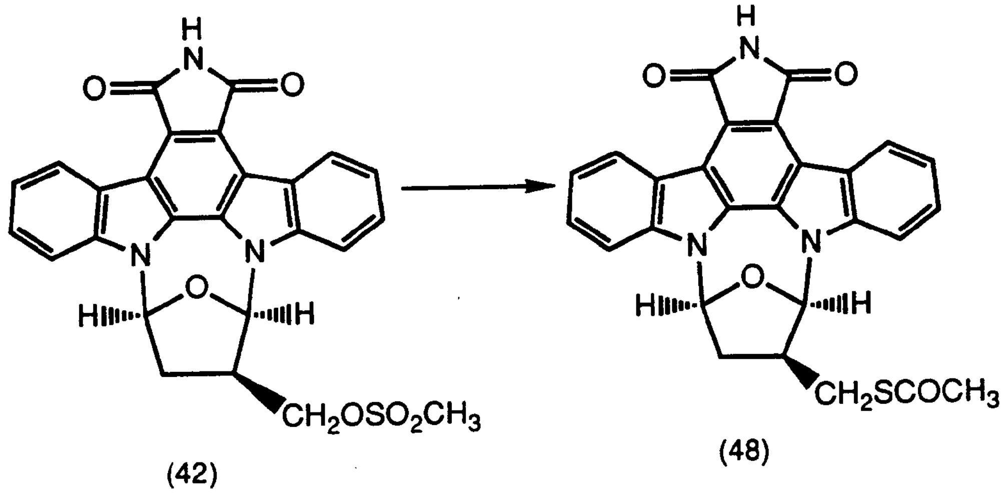

(42) (48)

Mesylate (42) (0.12g) and sodium thioacetate [0.046 g] in 15 mL THF was refluxed for 6 hours. The reaction was cooled and then diluted with 100 mL ethyl acetate and washed with $H_2O$ and brine. The organic phase was dried ($MgSO_4$), and concentrated to provide a crude residue which was subjected to silica-gel flash chromatography (20% acetone/hexane). The pure fractions were collected and the solvent removed to yield 0.09g of the thioacetate.
Mass Spec.: m/e = 481 (M+).
m.p.= 235°C - 237°C (with decomposition).

Example 45

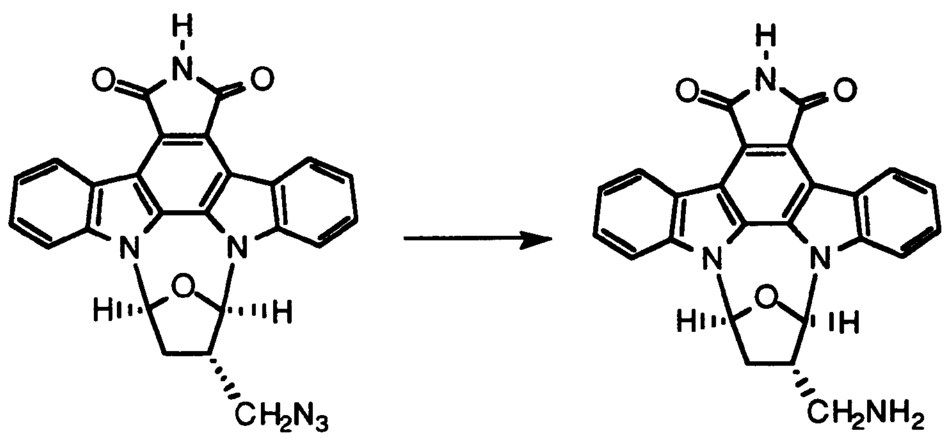

(45) (49)

A solution of the azide (0.38 g; Example 42) in DMF (15 mL) was shaken for 29 hours at room temperature with 20% palladium hydroxide on carbon (0.07 g) in hydrogen (60 psi). The mixture was diluted with ethyl acetate (100 mL), filtered, and the solution washed with 4 x 25 mL water, dried and evaporated. The residue was triturated in etherdichloromethane, and the solid filtered and dried at 25° and high vacuum to give the amino compound (49) (0.29 g) as a yellow powder
mp: 233-245° with decomposition
Mass Spectrum: m/e = 423 ($MH^+$).

Example 46

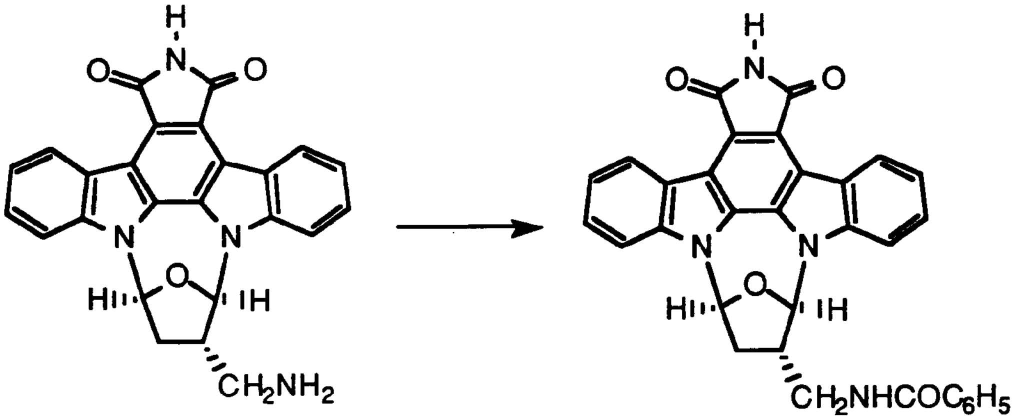

(49) (50)

A solution of the aminocompound (0.065 g; Example 45) in THF (5 mL) and pyridone (0.08 mL) was stirred for 10 minutes at room temperature with benzoyl chloride (0.042 g). After dilution with $CH_2Cl_2$ (50 mL), the solution was washed with water (3 x 50 mL), dried and evaporated, and the residue triturated in ether, filtered and dried at high vacuum to a yellow powder (0.032 g).
mp: 315-318° with decomposition.
Mass Spectrum: m/e = 526 ($M^+$).

Example 47

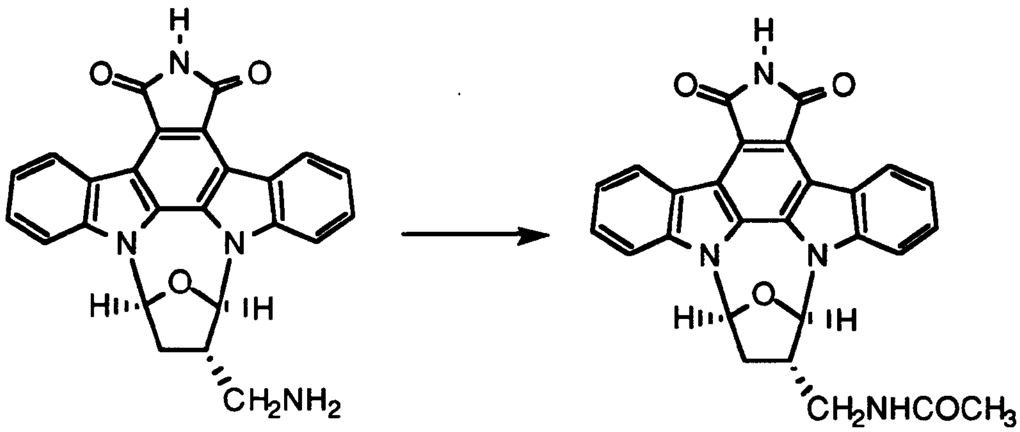

(49) (51)

A solution of the aminocompound (0.07 g; Example 45) in THF (5 mL) and acetic anhydride (0.1 mL) was stirred for 20 hours at room temperature, diluted with ethyl acetate (50 mL), washed with water, dried and evaporated. The residue was triturated in ether, filtered and dried at 25° and high vacuum to give the N-acetyl compound as a yellow powder (0.059 g).
mp: 320-327 with decomposition.
Mass Spectrum: m/e = 464 ($M^+$).

Example 48

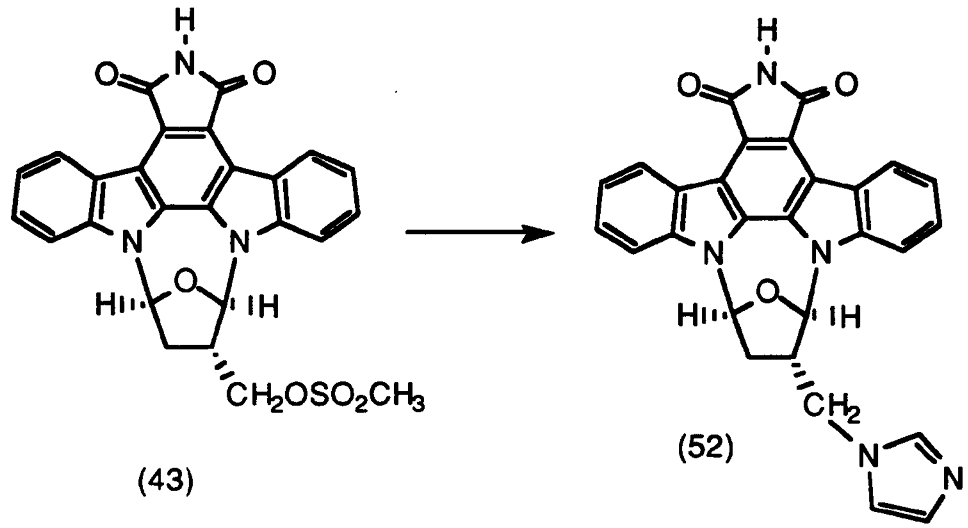

(43) (52)

A mixture of the mesylate (0.1 g; Example 40), imidazole (0.2 g) and DMF (1 mL) was heated for 20 hours at 90-100°, then worked up in ethyl acetate - $H_2O$, dried and evaporated. The crude product was chromatographed on silica, eluting initially with 5:1 $CH_2Cl_2$: acetone to remove a non-polar by-product, then with acetone. The product from the acetone eluates was further purified by chromatography on alumina, eluting with a gradient of 1% to 5% MeOH in $CH_2Cl_2$. The pure fractions were evaporated and dried at high vacuum to give the product as a yellow-orange powder (0.018 g).
mp: 300-310° with decomposition
Mass Spectrum: m/e = 474 ($MH^+$).

Example 49

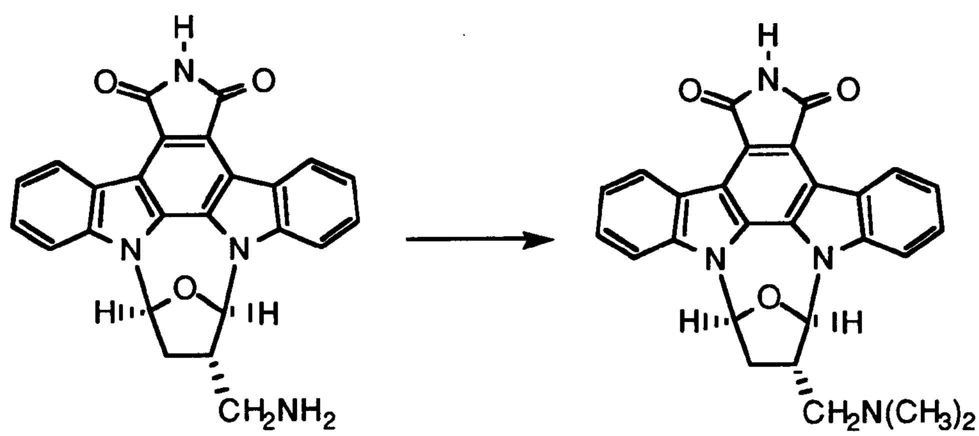

(49) (53)

The aminocompound (0.1 g; Example 45) and aqueous formaldehyde (1.5 mL) were stirred at room temperature for 3 hours in 1:1 MeOH-THF (10 mL) with acetic acid (0.08 g) and sodium cyanoborohydride (0.08 g). The reaction was diluted with EtOAc and washed with aqueous sodium bicarbonate, dried and evaporated. The resulting solid was stirred for 2 hours at room temperature in 1:1 MeOH-$CH_2Cl_2$ (20 mL) and potassium carbonate powder (0.8 g), then worked up in ethyl acetate - water. The product was isolated by chromatography on silica with 20:1 $CH_2Cl_2$ :
MeOH as a yellow powder (0.04 g).
mp: 204-209°.

Mass Spectrum: 451 ($MH^+$).

Example 50

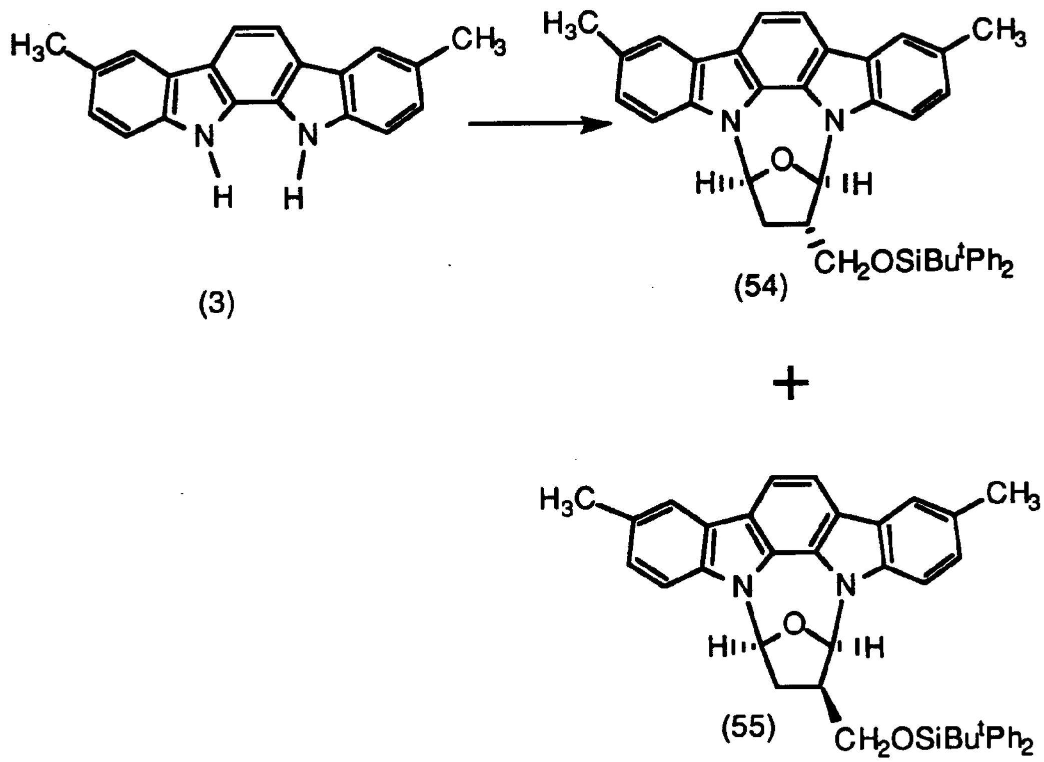

(3)

(54)

+

(55)

A mixture of 4,11-dimethylindolocarbazole (0.80 g), 2,5-dimethoxy-3-(tert-butyldiphenylsiloxymethyl)-tetrahydrofuran (3.0 g) and camphorsulfonic acid (0.5 g) in $CH_2Cl_2$ (20 mL) was stirred for 48 hours at room temperature, then diluted with hexanes (20 mL) and filtered through silica gel (10 g), washing with 1:1 $CH_2Cl_2$:hexanes. After evaporation, the crude product was chromatographed on flash-grade silica gel, eluting with a gradient from 4:1 to 1:1 hexanes:$CH_2Cl_2$, monitoring by thin layer chromatography with 1:1 $CH_2Cl_2$:hexanes. Appropriate pure fractions were evaporated to provide the less polar α-isomer (54) as a white foam (0.37 g) of indefinite melting point. As used herein $Bu^t$ means tert-butyl.
Mass Spectrum: m/e = 620($M^+$).
Later fractions gave the β-isomer (55) as a foam of indefinite melting point. Yield = 0.62g.
Mass Spectrum: m/e = 620($M^+$)

Example 51

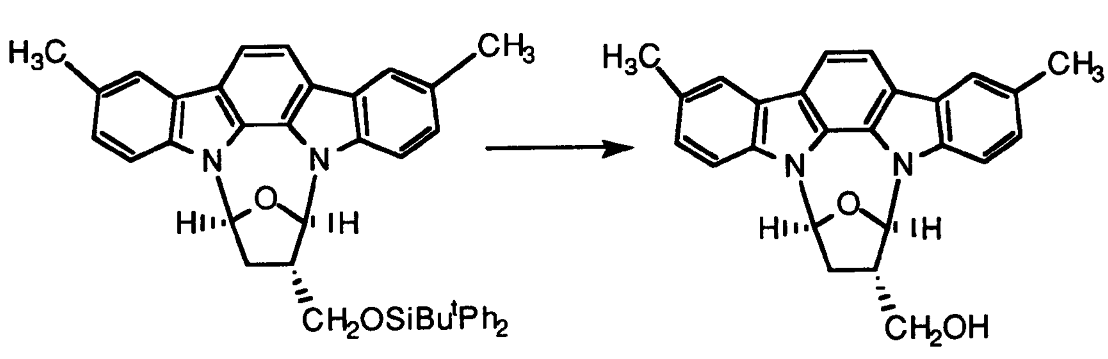

(54)

(56)

The silyl ether (0.37 g; Example 50) was stirred at room temperature for 1 hour in DMF (4 mL) with n-$Bu_4NF.3H_2O$ (0.21 g), then worked up in ethyl acetate, washing with water (3x). The solution was dried and evaporated, and the residue triturated thoroughly in ether-hexanes, filtered, washed with 1:1 ether-hexanes and dried at 25°/high vacuum to an off-white powder (0.21g). As used herein, n-Bu means n-butyl.
Mass Spectrum: m/e = 382 (M+)

Example 52

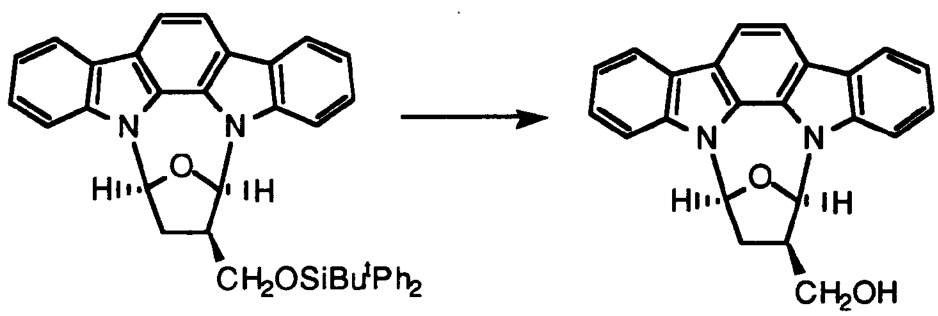

(55) (57)

Following the procedure of example 51, the corresponding β-isomer (0.62 g; Example 50) was desilylated to give the alcohol (57) (0.33g) as a white powder.
Mars Spectrum: m/e = 382 ($M^+$)

Example 53

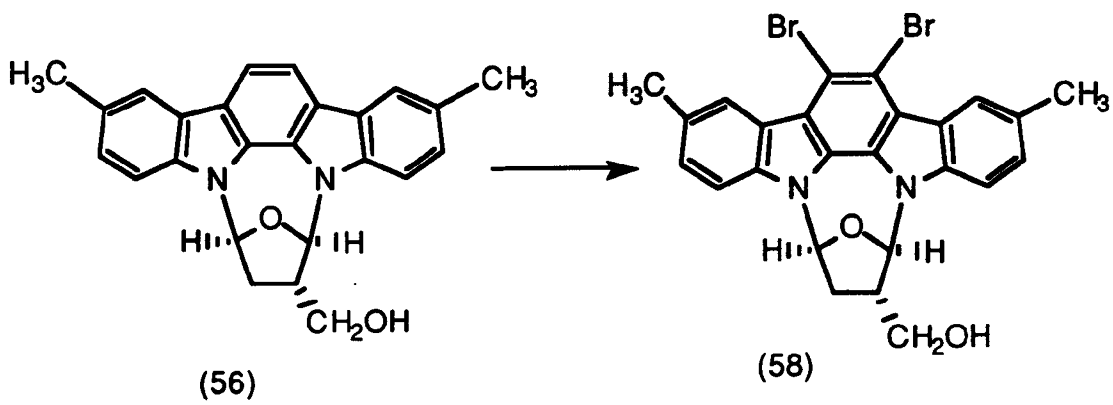

(56) (58)

The alcohol (56) (0.19 g; Example 51) in DMF (4 mL) was stirred at 0-5° and phenyltrimethylammonium tribromide PTAB (0.38 g) added in portions. After stirring without cooling for 2 hours, $H_2O$ (20 mL) was added and the solid was filtered, washed thoroughly with $H_2O$ and dried at 50°/high vacuum to a pale grey powder (0.23 g). This crude dibromocompound was used in the next step (Example 55) without further purification.

Example 54

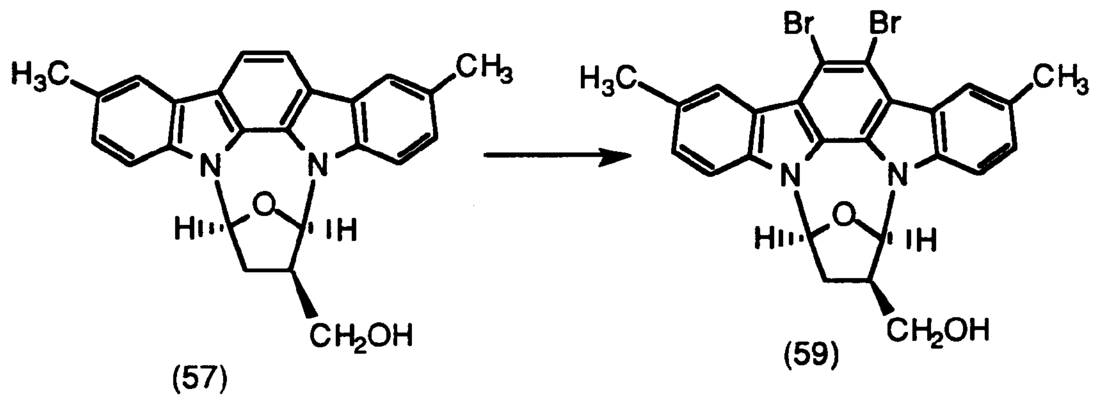

(57) (59)

Following the procedure of Example 53, the β-alcohol (57) (0.33 g; Example 52) was brominated with PTAB (0.65 g) in DMF to give the corresponding dibromocompound (59) (0.50 g) which was used in the next step (Example 56) without further purification.

Example 55

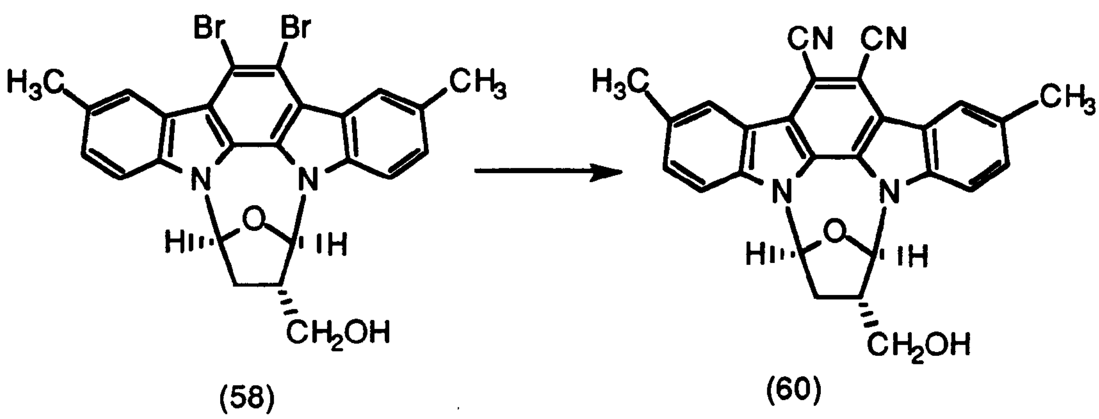

(58) (60)

A mixture of the crude dibromocompound (58) (0.22 g; Example 53), NaI (0.26 g), CuCN (0.45 g) and N,N-dimethylacetamide (DMAC) (5 mL) was heated at 180° for 5 hours, then cooled 10% aqueous ethylenediamine (50 mL) was added, and stirring continued for 1-2 hours. The solid was collected, washed with water and dried at high vacuum. This crude material was subjected to Soxhlet extraction with 10:1 $CH_2Cl_2$: acetone for 24 hours, and the solution was then evaporated. The residue was triturated in 1:1 $CH_2Cl_2$: ether, filtered off and dried to give the dicyanocompound (60) as a pale grey powder (0.11 g).
mp: >300° (decomposes)
Mass Spectrum: m/e = 432 ($M^+$).

Example 56

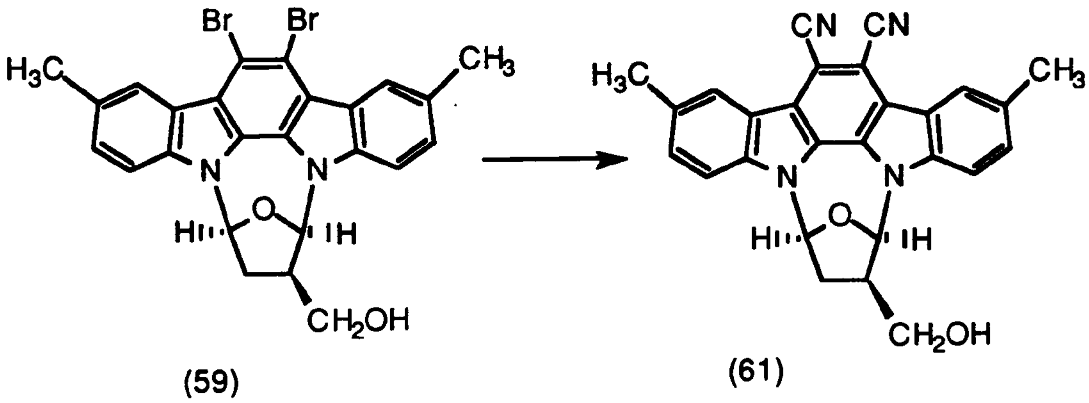

(59) (61)

Following the method of Example 55, the β-alcohol (59) (0.49 g; Example 54) was converted to the corresponding dicyanocompound (61 ), a pale brown powder (0.26 g).
mp: >300° (decomposes)
Mass Spectrum: m/e = 432 ($M^+$)

Example 57

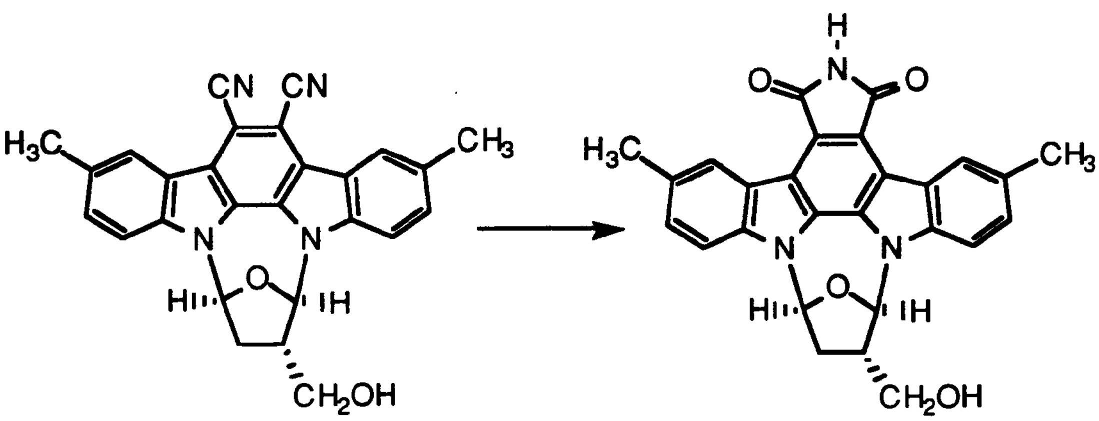

(60) (62)

The dicyanocompound (60) (0.11 g; Example 55) was heated at 70-80° for 1.5 hours in DMSO (10 mL) with KOH (0.2 g in 0.5 mL $H_2O$), with stirring. After cooling, trifluoroacetic acid (0.5 mL) was added, and stirring continued at room temperature for 2 hours. Water was added, and the product extracted with ethyl acetate. The organic phase was washed with $H_2O$ (3x), dried and evaporated, and the residue triturated in ether-$CH_2Cl_2$, filtered off and dried to give the imide (62) as a bright yellow powder (0.08 g).
mp: 208-212°
Mass Spectrum: m/e = 451 ($M^+$)

Example 58

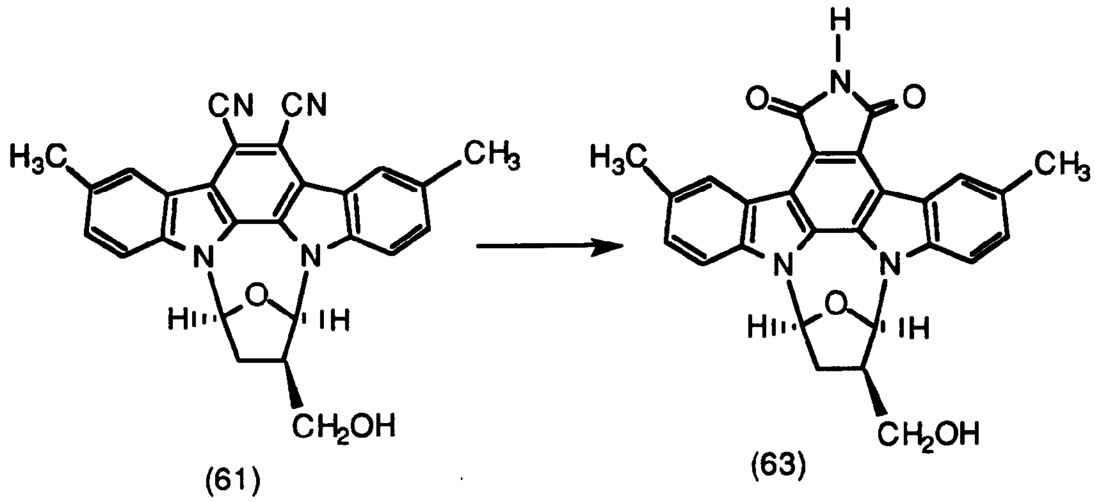

(61) (63)

Following the procedure of Example 57, the appropriate dicyanocompound (61 ) (0.26 g; Example 56) was converted to the corresponding imide (63), a yellow solid (0.16 g).
mp: 315-325° (decomposition)
Mass Spectrum: m/e = 451 ($M^+$).

Example 59

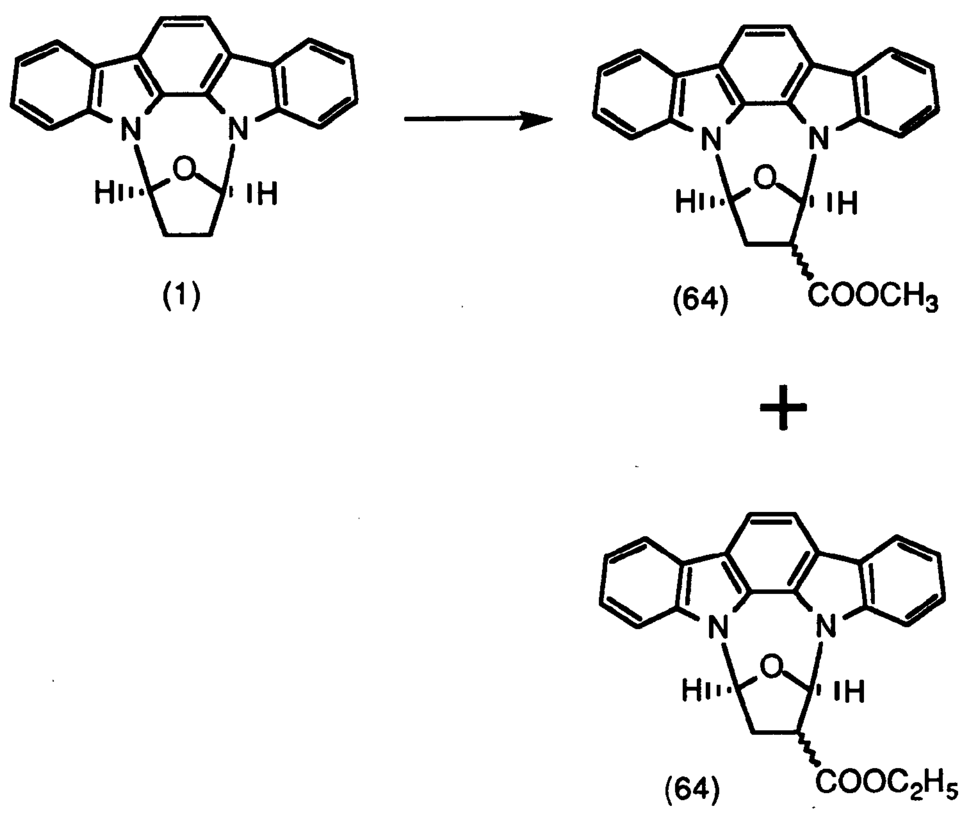

(1) (64) (64)

A mixture of indolocarbazole (1) (16.0 g), ethyl 2,5-dimethoxytetrahydro-3-furoate (29 g), dichloromethane (400 mL) and p-toluenesulfonic acid (5 g) was stirred for 60 hours at room temperature, then filtered through a 50 g pad of silica gel, washing with 300 mL of 4:1 $CH_2Cl_2$ hexanes. Evaporation gave a yellow oil, which was chromatographed on flash grade silica, with a gradient from 2:1 to 1:4 hexanes: $CH_2Cl_2$. The fractions containing

the non-polar ester mixtures were combined, evaporated and pumped at high vacuum to give a white foam, which was used directly in the next step (Example 60).

Example 60

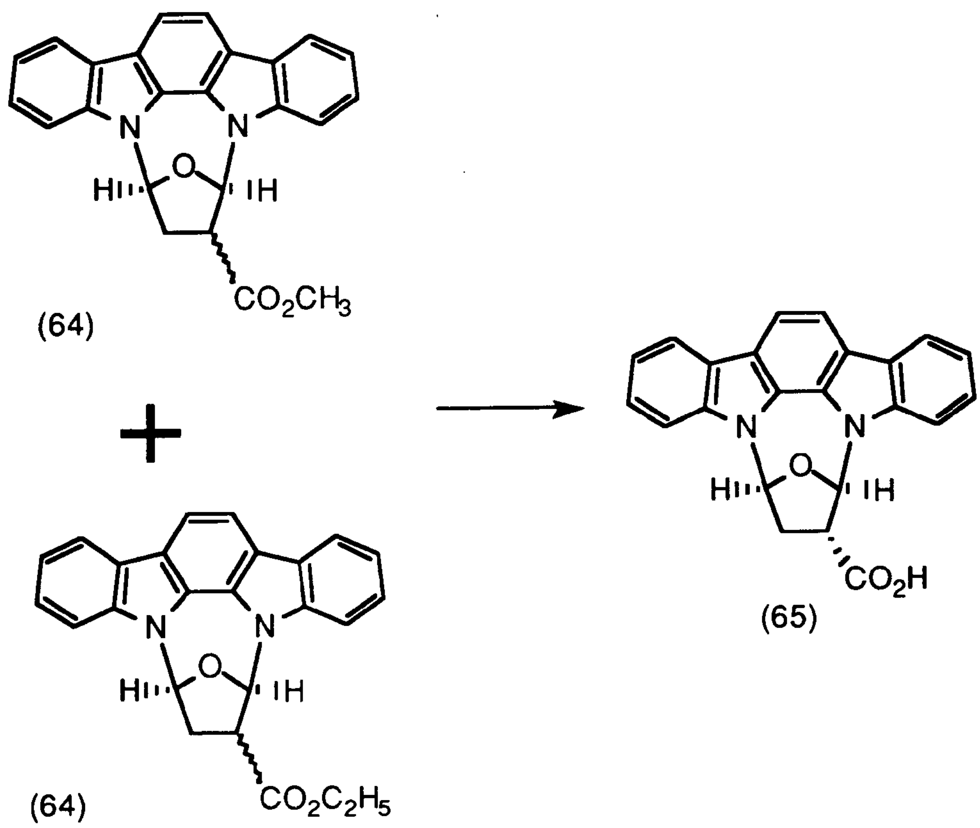
(64)
(64)
(65)

All of the ester mixture (64) (Example 59) was stirred in DMSO (200 mL) and methanol (150 mL). A solution of KOH (25 g) in $H_2O$ (40 mL) was added, and the mixture was heated on a steam bath for 1.5 hours, then diluted with iced water (1.25 L). With stirring, concentrated hydrochloric acid (50 mL) was added. After 0.5 hours, the solid was filtered, washed with several portions of hot water, and dried at 70° in high vacuum. This material was dissolved in 1:1 acetone-$CH_2Cl_2$, stirred with 25 g of activated carbon and filtered, washing with a little acetone. The solution was evaporated and the resulting solid triturated in 100 mL of 1:1 ether-hexanes, collected and dried at 70° to give the α-acid (65) as an off-white powder (14.9 g).
mp: 260-265° with decomposition.
Mass Spectrum: m/e = 368 (M)+

Example 61

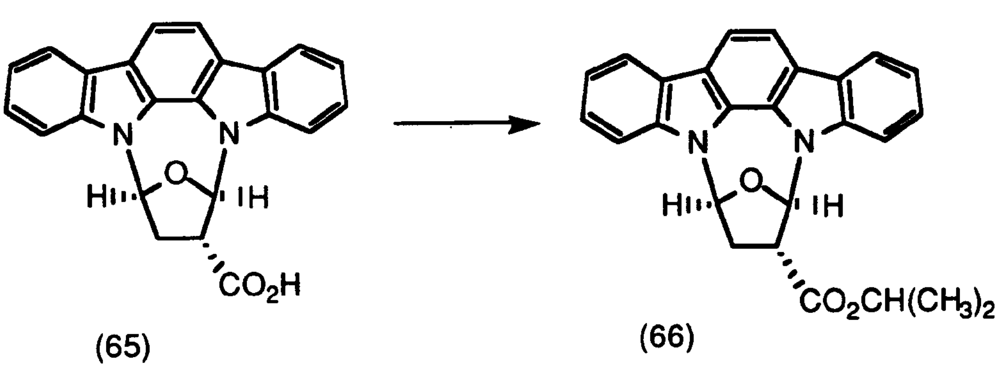
(65)
(66)

A mixture of the α-acid (65) (1.50 g; Example 60), 2-propanol (1.5 mL), 4-dimethylaminopyridine (0.1 g), N-hydroxybenzotriazole (0.1 g) and $CH_2Cl_2$ (40 mL) was stirred at room temperature and water-soluble carbodiimide (2.5 g) added in portions. After 4-5 hours, the mixture was washed with aqueous $NaHCO_3$ and water, dried and evaporated. The crude product was chromatographed on silica gel in 3:1 $CH_2Cl_2$: hexanes, and crystallized from ester-hexanes to give the 2-propylester (66) as a white solid (1.32 g).
mp: 170-173°
Mass Spectrum: m/e = 410 ($M^+$).

Example 62

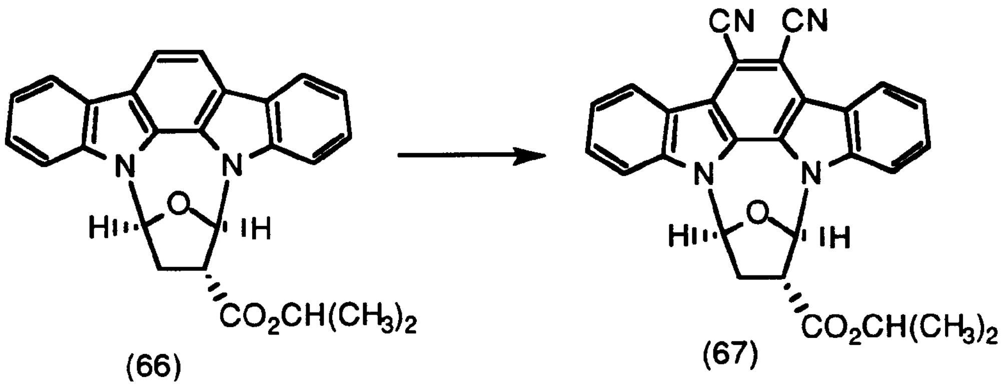

(66) (67)

A solution of the isopropylester (66) (0.82 g; Example 61) in DMF (20 mL) was stirred at room temperature and N-bromosuccinimide (0.72 g) was added in portions. The solution was stirred 20 hours at room temperature, then diluted gradually with $H_2O$ (70 mL). After 0.5 hours, the dibromocompound was filtered, washed with $H_2O$ and dried at 25°/high vacuum, then used in the next step. Yield = 1.08 g.

The foregoing product (1.0 g) was heated fro 8 hours at 170-180° in DMAC (25 mL) with NaI (0.4 g) and CuCN (1.5 g), then cooled and added to 150 mL of 10% aqueous ethylene diamine. After stirring for 1 hour, the crude product was filtered, washed ($H_2O$) and dried at high vacuum. This material was subjected to Soxhlet extraction with 10:1 $CH_2Cl_2$: acetone for 48 hours. The solution was evaporated and the residue triturated in 20 mL $CH_2Cl_2$, filtered and dried to give the dinitrile (67) as a cream powder (0.48 g).
mp: 298-303°, then decomposes
Mass Spectrum: m/e = 460 ($M^+$)

Example 63

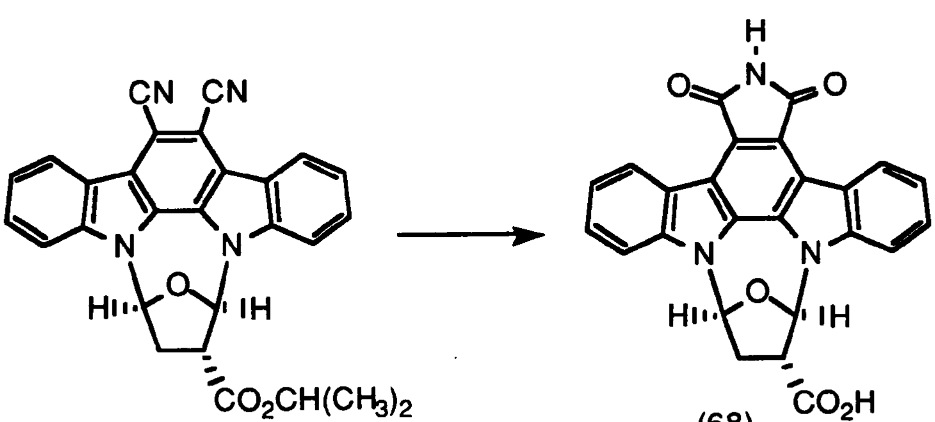

(67) (68)

A mixture of the dinitrile (67) (0.31 g; Example 62), $LiOH.H_2O$ (0.5 g), DMSO (10 mL) and 1:1 $MeOH-H_2O$ (4 mL) was heated at 70-80° with stirring for 1 hour. $H_2O$ (2 mL) was added, and the temperature of the heating bath gradually raised over ~1 hour to 150°, allowing methanol and some water to distill out.

The residual solution was cooled in ice and trifluoroacetic acid (1.8 mL) added, producing an orange red solution which subsequently deposited a yellow solid. After 2 hours at room temperature, $H_2O$ (20 mL) was

added and the product was filtered, washed well with water, then with 2 x 10 mL ether, and dried at 70°/high vacuum to give the acid (68) as an orange-yellow powder (0.27 g).
mp: 340-350° (decomposing)
Mass Spectrum: m/e = 438 and 437 ($MH^+$ and $M^+$)

Example 64

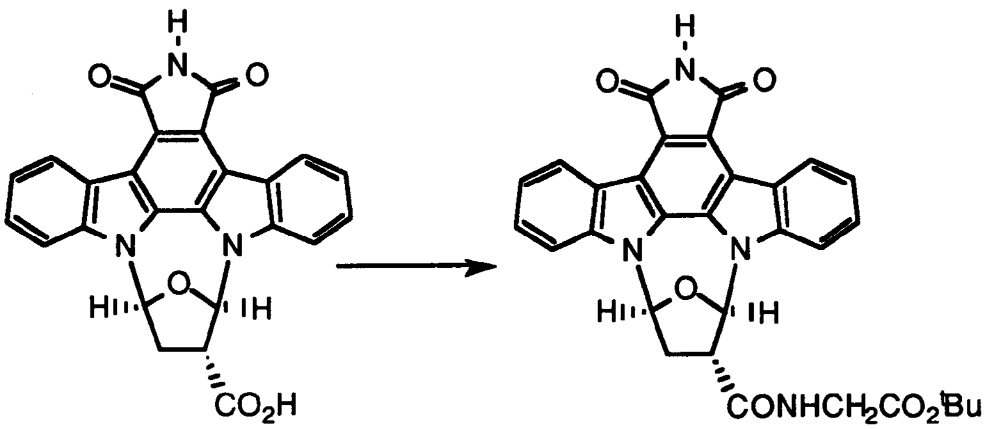

(68) (69)

A solution of the acid (68) (0.44 g; Example 63) in DMF (3 mL) was stirred for 2 hours at room temperature with $NEt_3$ (0.03 mL), N-hydroxy benzotriazole (0.027 g), glycine-t-butylester hydrochloride (0.034 g) and $H_2O$-soluble carbodiimide (0.05 g). The reaction was worked up in EtOAC-$H_2O$ and washed with aqueous $NaHCO_3$ then aqueous tartaric acid, dried and evaporated. The residue was triturated with ether to give the product (69) as an orange-yellow powder (0.037 g).
mp: decomposes above 250°
Mass Spectrum: m/e = 551 ($MH^+$).

Example 65

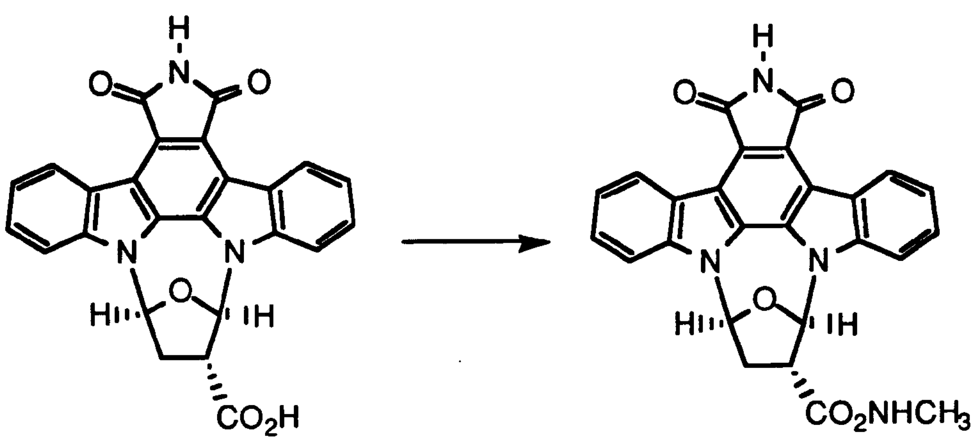

(70)

(68)

A mixture of the acid (68) (0.06 g; Example 63), DMF (4 mL), $MeNH_3^+Cl^-$ (0.1 g), $NEt_3$ (0.1 mL), N-hydroxybenzotriazole (0.06 g) and $1H_2O$-soluble carbodiimide (0.14 g) was stirred at room temperature for 20 hours. $H_2O$ (20 mL) was added slowly, and stirring continued for 1 hour. The product was collected, dried in air, and purified by column chromatography on silica gel with a gradient of 0-10% methanol in $CH_2Cl_2$. Pure fractions were evaporated to give the methylamide (70) as a yellow powder (0.049 g).
mp: >300° (decomposes)

Mass Spectrum: m/e = 450 ($M^+$).

Example 66

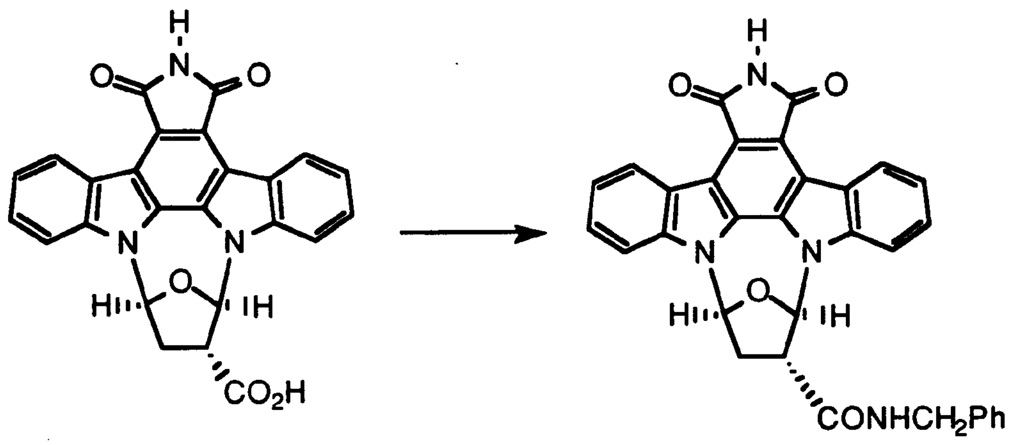

(68) (71)

The procedure of Example 65 was followed, replacing the methylamine hydrochloride with benzylamine hydrochloride. After isolation and chromatography in 2% MeOH-$CH_2Cl_2$, the benzylamide (71) was obtained as a yellow solid (0.034 g).
mp: 290-295°, with decomposition.
Mass Spectrum: m/e = 527 ($MH^+$)

Example 67

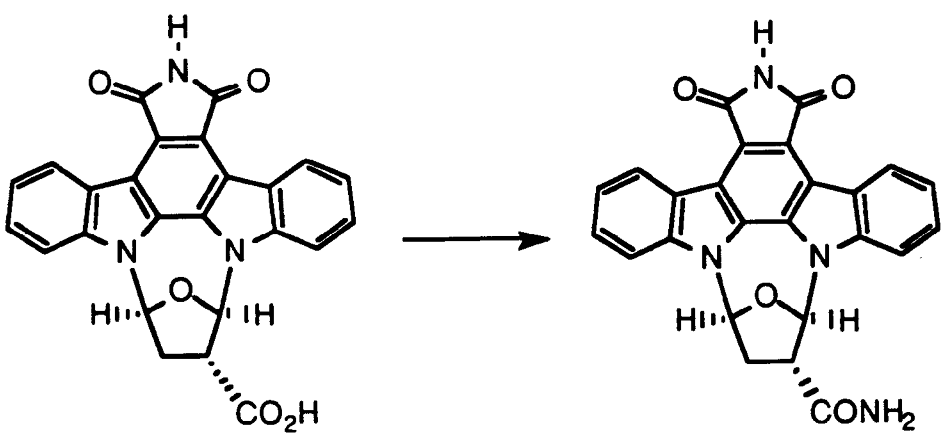

(68) (72)

The procedure of example 65 was followed, replacing the methylamine hydrochloride with ammonium chloride. After precipitation with $H_2O$, filtration and drying, the crude material was of satisfactory purity, and was insufficiently soluble in nonpolar solvents ($CH_2$, ethyl acetate) for chromatographic purification. The amide (72) was obtained as a bright yellow, amorphous solid (0.05 g).
mp: >350° (decomposes)
Mass Spectrum: m/e = 436 ($M^+$).

Example 68

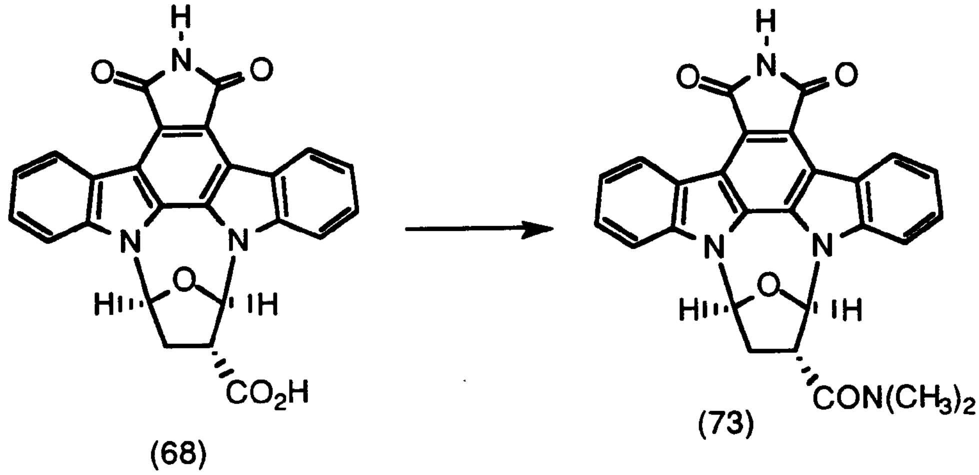

(68) (73)

The procedure of example 65 was followed, replacing the methylamine hydrochloride with dimethylamine hydrochloride. After chromatography on silica gel with 5% acetone-$CH_2Cl_2$, the dimethylamide (73) was obtained as a yellow powder (0.038 g).
mp: >350°
Mass Spectrum: m/e = 464 ($M^+$)

Example 69

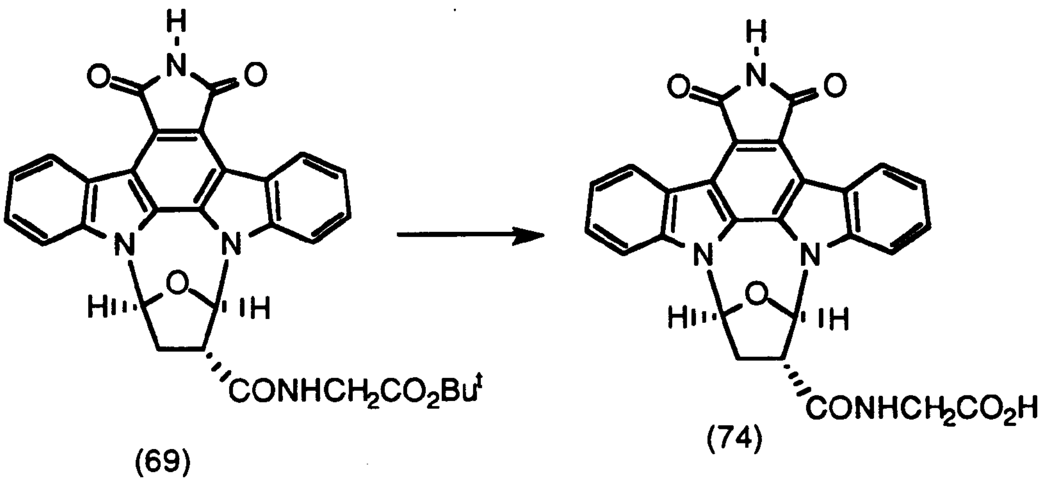

(69) (74)

The t-butylester (69) (0.025 g; Example 64) was stirred at room temperature for 2 hours in trifluoroacetic acid (2 mL). $H_2O$ (20 mL) was added. After stirring for 0.5 hours, the precipitate was collected, washed with water and ether, and dried at 50° in high vacuum to give the acid (74) as a deep orange powder (0.011 g).
mp: decomposes above 300°
Mass Spectrum: m/e = 495 ($MH^+$).

Example 70

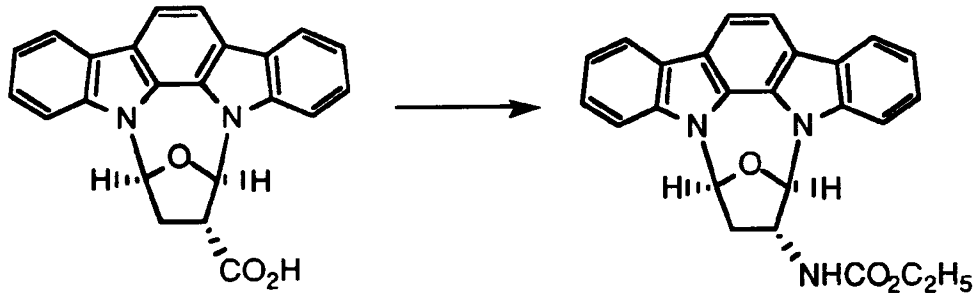

(65) (75)

A mixture of the α-acid (65) (1.85 g; Example 60), diisopropylethylamine (1.75 mL), dichloroethane (75 mL) and diphenyl phosphoryl azide (2.75 g) was refluxed for 0.5 hours at 90°-95°. Ethanol (5 mL) and dibutyltin acetate (0.2 g) were added, and refluxing continued for 1.5 hours. After cooling, the mixture was diluted with $CH_2Cl_2$ and washed with aq. HCl; (1 N) then 5% aqueous - $Na_2CO_3$ solution, dried and evaporated. The product was purified by flash chromatography with 3:1 $CH_2Cl_2$:hexanes. Pure fractions were evaporated and the product (75) tritiated in a little ether and dried at high vacuum to a white powder (1.72 g).
mp: 219-222°
Mass Spectrum: m/e = 411 ($M^+$).

Example 71

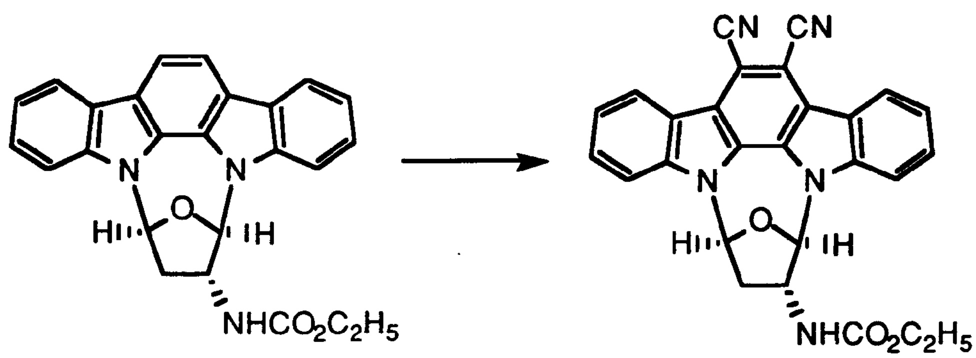

(75) (76)

A solution of the carbonate (75) (0.62 g; Example 70) in DMF (15 mL) was treated in portions with N-bromosuccinimide (0.55 g), and stirring continued at room temperature for 20 hours. $H_2O$ (50 mL) was slowly added, and stirring continued for 1 hour. The dibromocompound was collected, washed with water and dried for 1 hour at 80° in high vacuum, then used in the next step. Yield = 0.88 gm.

The foregoing product (0.87 g) was heated at 180° for 6.5 hours in DMAC (20 mL) with NaI (0.25 g) and CuCN (1.0 g), cooled, diluted with 200 mL of 50% aqueous ethylenediamine and extracted into $CH_2Cl_2$ (750 mL). The solution was washed with $H_2O$ and 0.1 N aqueous HCl, dried and evaporated, and residual DMAC was removed at high vacuum. The solid was stirred for 0.5 hours in 30 mL 1:1 $CH_2Cl_2$:ether, collected, washed with 5 mL $CH_2Cl_2$ and dried to give the dinitrile (76) as a pale brown solid (0.39 g).
mp: 320-325° decomposition
Mass Spectrum: m/e =461 ($M^+$)

Example 72

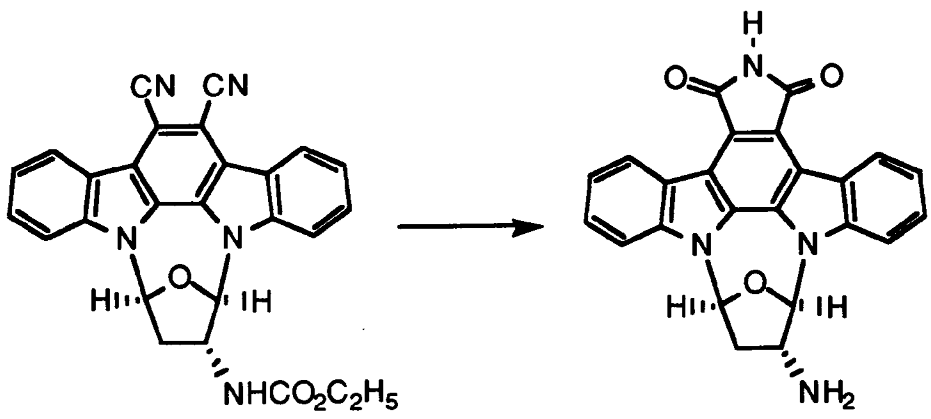

(76) (77)

A mixture of the dinitrile (76) (0.36 g; Example 71 ), DMSO (7 mL) and KOH in $H_2O$ (2 mL) and trifluoroacetic acid (TFA) (1-2 mL) were added, and the mixture stirred at 50° for 0.25 hours. A mixture of $H_2O$ (20 mL) and concentrated ammonia (4 mL) was added, stirring continued at room temperature for 1 hour, and the product filtered, washed thoroughly with that water and dried in high vacuum at 80° to give the amine (77) as a yellow powder (0.31 g).
mp: 320-325°, with decomposition.
Mass Spectrum: m/e = 409 ($MH^+$).

Example 73

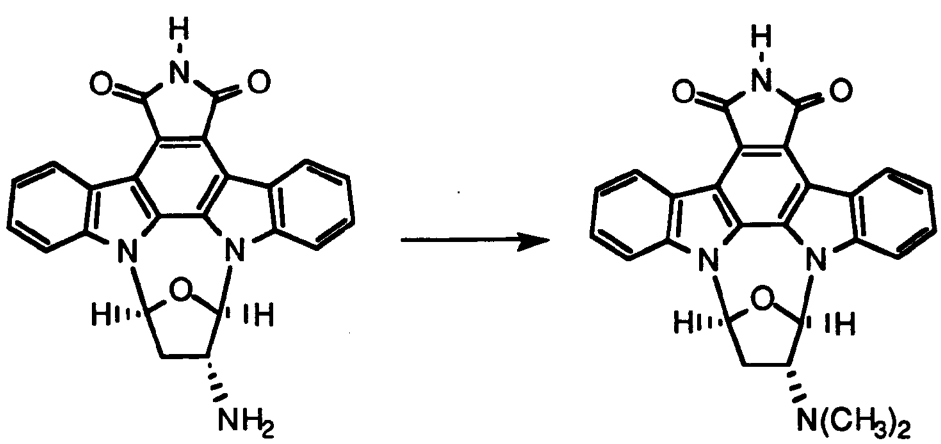

(77) (78)

A mixture of the primary aminocompound (77) (0.20 g; Example 72), MeOH (4 mL), THF (8 mL), HOAc (0.15 mL), 37% aqueous formaldehyde solution (0.2 mL) and $NaBH_3CN$ (0.1 g) was stirred for 20 hours at room temperature. The mixture was diluted with MeOH (15 mL) and stirred for 1 hour at room temperature with potassium carbonate (2 g). Water (150 mL) was added, and stirring at room temperature continued for 1.5 hours. The solid was filtered, washed with water and dried in high vacuum at 80° to give the dimethylaminocompound (78) as a yellow powder (0.21 g).
mp: 200-215° with decomposition.
Mass Spectrum: m/e = 437 ($MH^+$)

Example 74

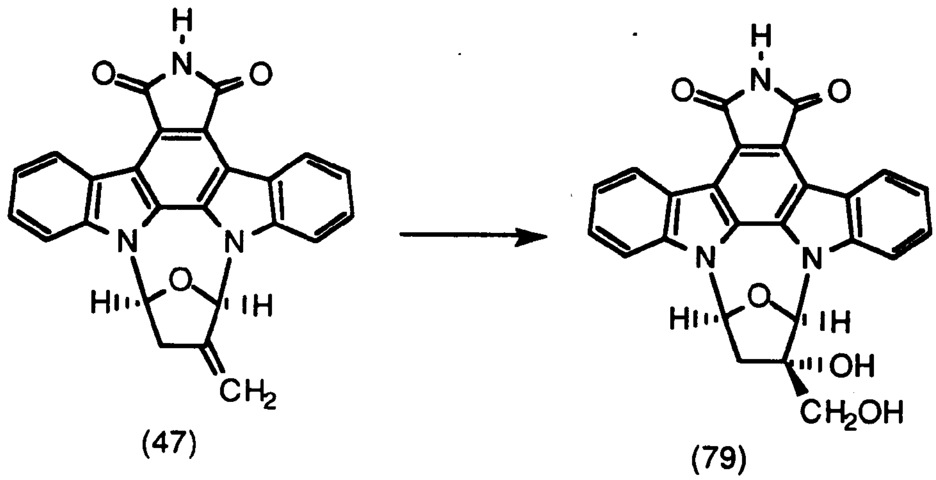

(47) (79)

To a 5 mL anhydrous THF solution containing olefin (47) (Example 43: 0.024 g) and pyridine (0.04 g), was added osmium tetroxide ($OSO_4$; 1 e.g., as 25% wt t-Butanol solution). The resulting mixture was stirred for 10 hours and then diluted with ethyl acetate (50 mL). The organics were washed with 1 x 25 mL aqueous sodium bisulfite and 2 x 25 mL water, dried ($MgSO_4$), and concentrated to yield a crude product which was subjected to silica-gel prep. plate chromotography to provide 0.012 g of diol (7 g).

Example 75

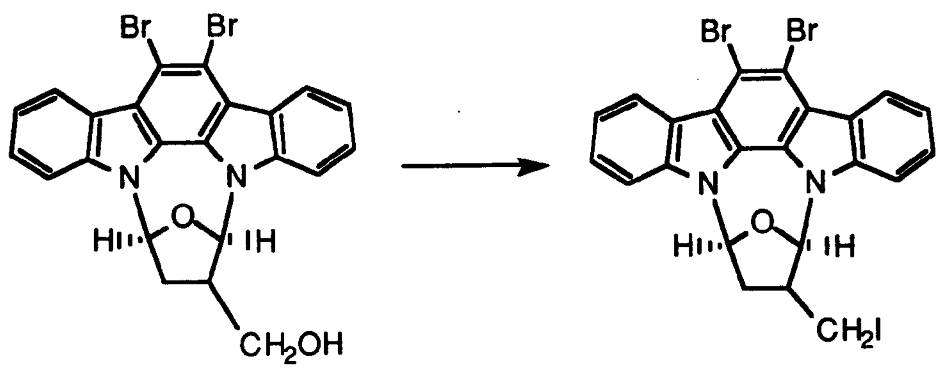

(18) (80)

N-Iodo succimimide (1.3 g) and triphenylphosphine (TPP) (1.68 g) were dissolved in anhydrous THF (50 mL) at 0°C. To this solution was added alcohol (18) (0.6 g, Ex. 13) and stirred for 6 hours at room temperature. The reaction was diluted with 100 mL ethyl acetate and washed (3 x 50 mL water) to remove succinimide. The organic portion was dried ($MgSO_4$) and concentrated to yield a solid. The solid was washed (2 x 30 mL hexane) to remove triphenyl phosphine. The resulting crude product was redissolved in 50 mL ethyl acetate and upon concentration the product (80) crystallized and leaving TPP oxide in the solution. The product was collected by filtration and dried to provide 1.2 g of pure product.

Mass Spec.: m/e = 621.9

Example 76

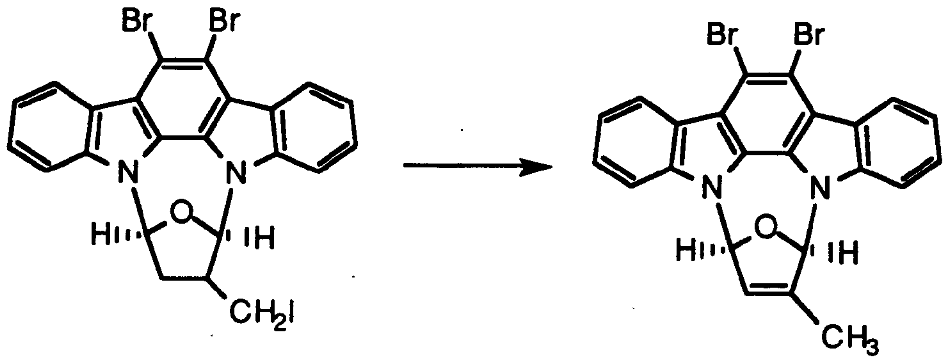

(80) (81)

To a solution of 5 mL anhydrous THF containing triethylene glycol mono methyl ether (0.052 g; 2 equivalents) at 0°C, was added NaH (60% oil dispersion; 0.014 g). The mixture was stirred for 10 minutes and then there was added 2 mL THF solution containing (80) (0.1 g). The reaction was stirred for 6 hours at room temperature and then quenched with 2 mL water, followed by dilution with ethyl acetate (25 mL). The organics were washed 2 x 10 mL water, dried ($MgSO_4$) and concentrated to yield crystalline product (0-07 g).
Mass Spec: EI m/e = 405.1 ($M^+$)

Example 77

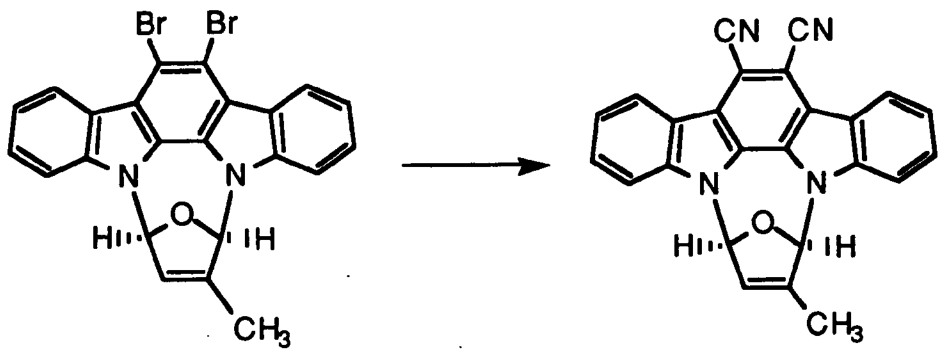

(81) (82)

The procedure from Example 16 was repeated on dibromide (81) (0.3 g) with CuCN (0.426 g) and NaI (0.213 g). Similar work up provided the dinitrile (82) which was taken to the next step. (Base-acid hydrolysis).

Example 78

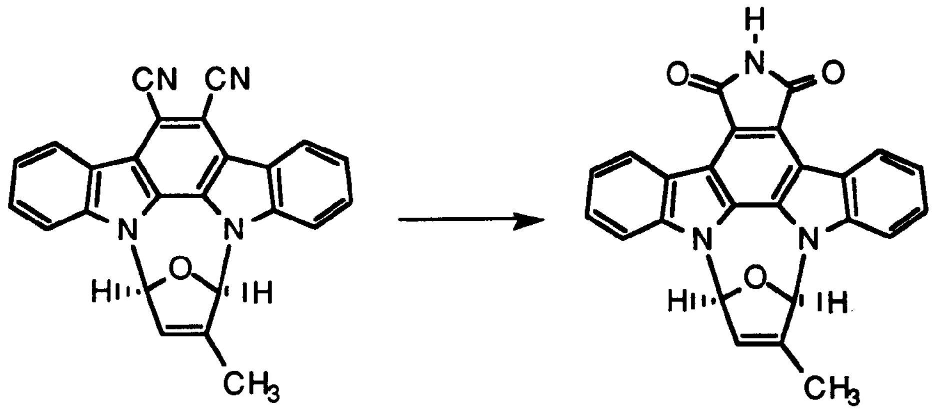

(82) (83)

Dinitrile (82) obtained from Example 77 was hydrolysed by employing the procedure from Example 22 using $KOH/H_2O$ (0.6 g/2 mL) followed by trifluoroacetic acid (0.9 mL). Similar work up/purification provided (83) (0.12 g).
Mass Spec: EI m/e = 405.1 ($M^+$).

Example 79

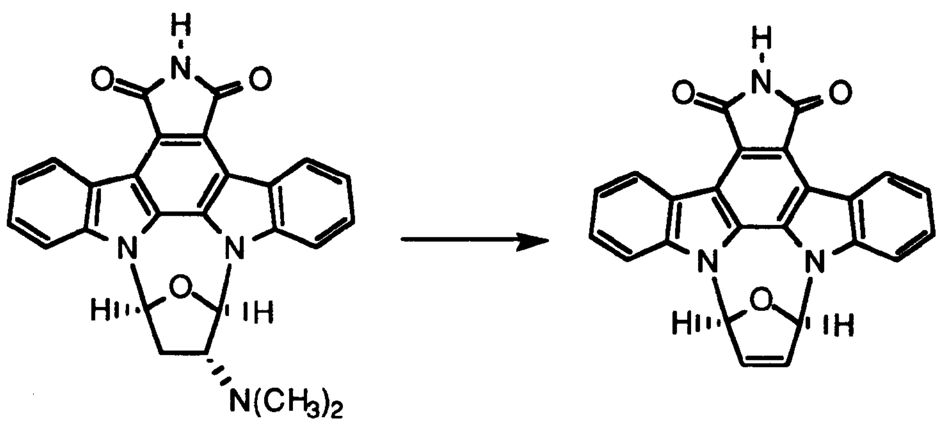

(78) (84)

A solution of the dimethyl amino compound (Example 73; 0.12 g) in DMF (5 mL) was stirred for 10 minutes with the addition of m-chloroperoxybenzoic acid (0.1 g). A mixture of $H_2O$ (30 mL) and concentrated ammonia (2 mL) was added and after further stirring, the intermediate N-oxide was filtered, washed with $H_2O$ and dried in high vacuum at room temperature.

This product was stirred at 25-30°C for 70 hours in DMSO (15 mL). The solution was diluted with ethyl acetate, washed (3 x $H_2O$), dried and evaporated, and the product chromatographed on silica gel, using 10% ether-$CH_2Cl_2$ to elute the non-polar product. The olefin (84) was obtained as a yellow powder (0.027 g).
mp: >300° (decomposes)
Mass Spectrum: m/e = 391 ($M^+$).

Example 80

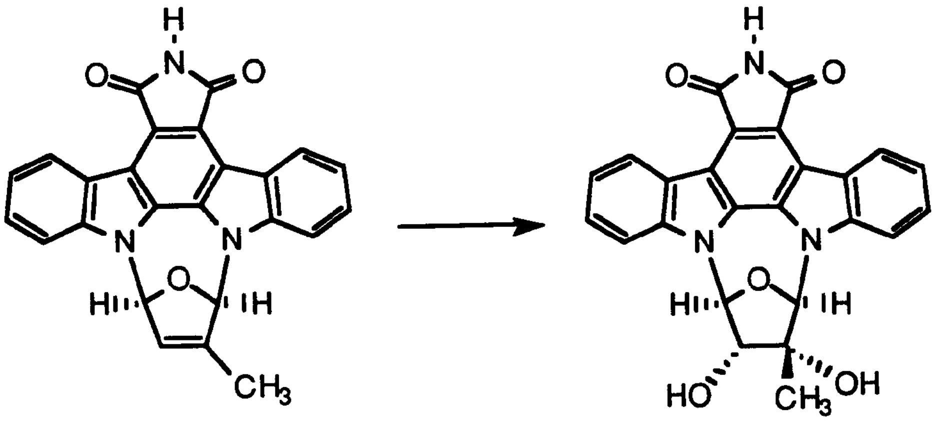

(83)

(85)

Treat the olefin (83) (Example 78) with osmium tetroxide according to the procedure of Example 74, workup and chromatograph to obtain the diol (85).

Example 81

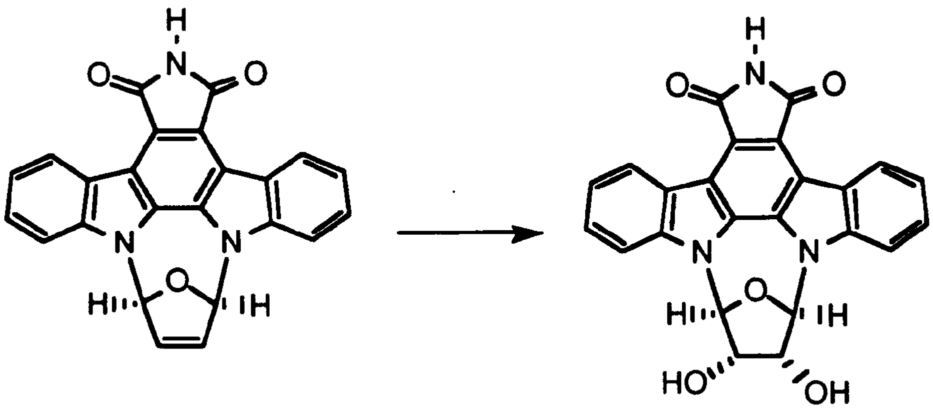

(84)

(86)

Following the same procedure as in Examples 80 and 74, oxidise the disubstituted olefin (84) (Example 79) to obtain the diol (86).

Example 82

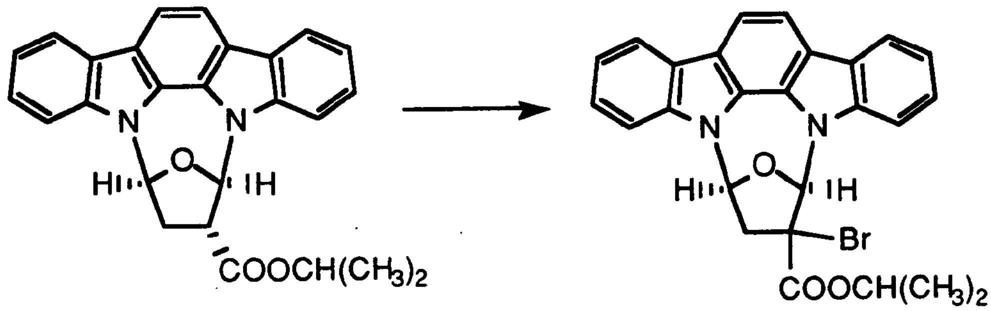

(66) (87)

To a dry THF solution of the ester (66) (Example 66), cooled to -70°, add lithium diisopropylamide in THF (1.5 equivalents). After 15 minutes at -70°, add carbon tetrabromide (5 equivalents) and keep the mixture for 2 hours at -70°. Work up in $H_2O$-ethyl acetate, dry, evaporate and chromatograph to obtain the bromoester (87) as a mixture of diastereoisomers.

Example 83

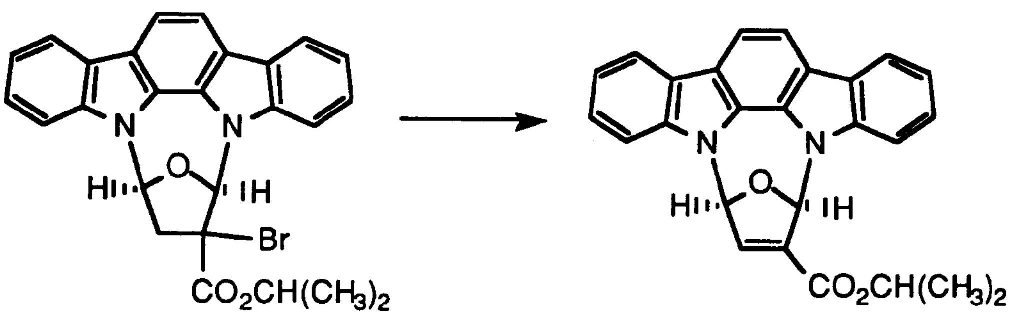

(87) (88)

Reflux a dry THF solution of the ester (87) (Example 82) with 5 equivalents of diazabicycloundecane (DBU) till thin layer chromatography indicates the elimination to be complete. Work up the reaction in ethyl acetate-aqueous tartaric acid, dry, evaporate and chromatograph on silica gel to obtain the olefin (88).

Example 84

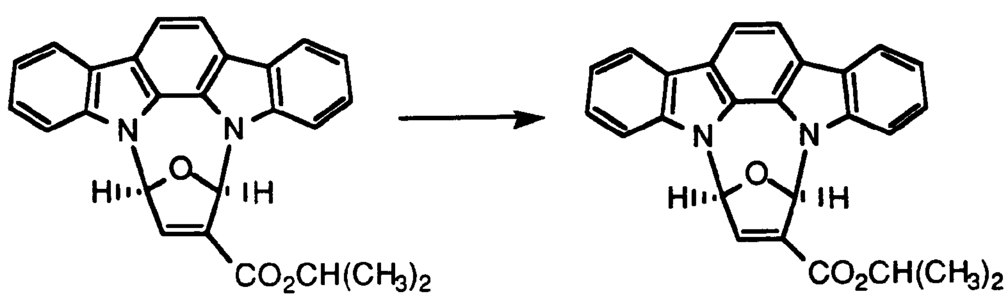

(88) (89)

Treat a dry THF solution of the ester (87) from Example 83 at room temperature with a solution containing lithium aluminum hydride (4 equivalents), and stir until reaction is complete. Workup by addition of excess ethyl

acetate, wash with water, dry, evaporate and isolate the alcohol (89) by silica gel chromatography.

Example 85

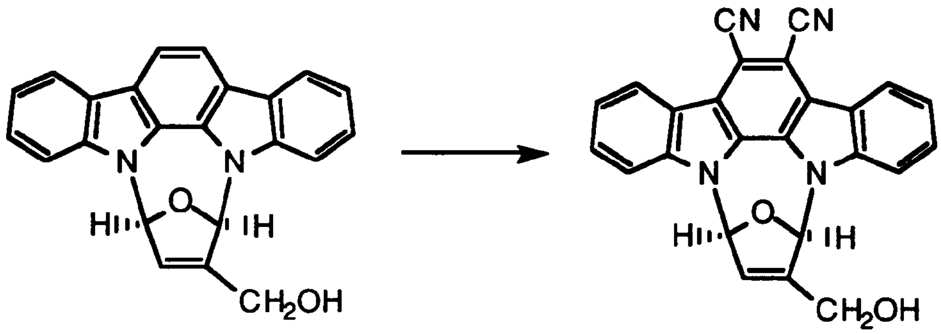

(89) (90)

Following the procedures described in Examples 15 and 16, dibrominate the alcohol 89 from Example 84 with two equivalents of N-bromosuccinimide, isolate the dibromo-compound and react it with cuprous cyanide and sodium iodide in hot dimethylacetamide to obtain the dicyanocompound (90).

Example 86

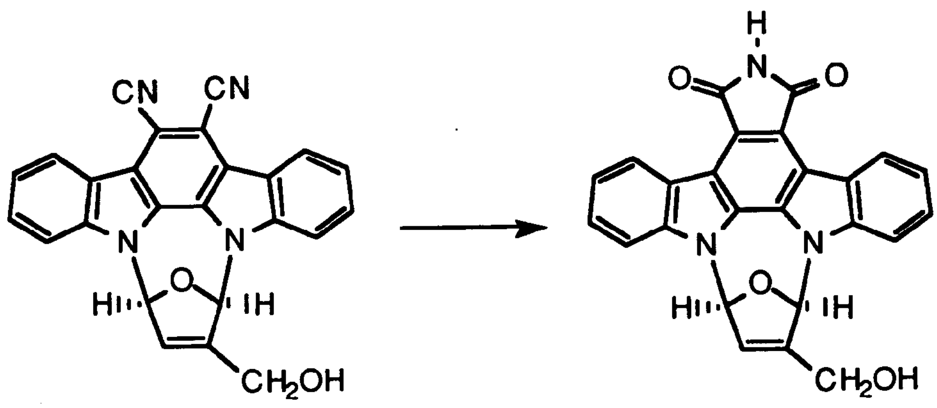

(90) (91)

Following the procedure described in Example 35, treat the dicyanocompound (90) from Example 85 with KOH-$H_2O$ in DMSO at 100°C, followed by aqueous trifluoroacetic acid at room temperature, to obtain the imide (91).

## Claims

1. A compound of the formula

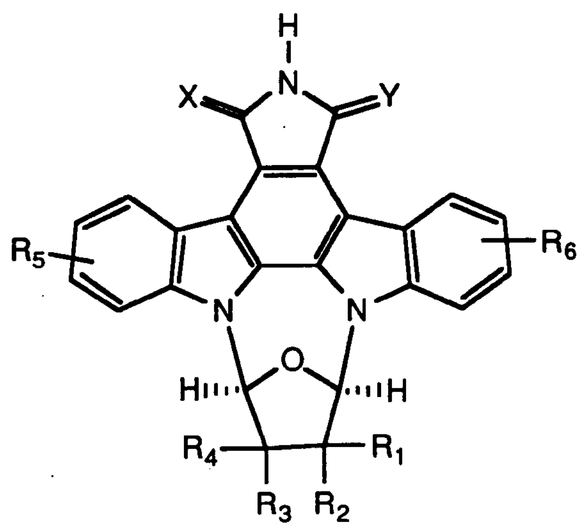

I

or a pharmaceutically acceptable salt thereof, wherein

X is O or S;

Y is O, NH, (H,H), (H,OH) or S; with the proviso that when X is S, Y is not NH, (H,H) or (H,OH);

$R_1$, $R_2$, $R_3$ and $R_4$ may be the same or different and each independently is H, $C_1$-$C_7$ alkyl, $C_1$-$C_7$ substituted alkyl, -CHO, CN, -CON($R_8$,$R_9$), -COO$R_7$, -CH=NO$R_8$, -CH=NNHCON$H_2$, F, Cl, Br, OH, $N_3$, -O$C_1$-$C_7$ alkyl, -O$C_1$-$C_7$ substituted alkyl, -OCO$R_9$, SH, S($C_1$-$C_7$)alkyl, S($C_1$-$C_7$)substituted alkyl, SCO$R_{10}$, S(O)$_n$$R_{11}$, $NH_2$, N($R_{12}$,$R_{13}$), and N( acyl, $R_{14}$);

$R_1$, and $R_2$ taken together may be O, NOH, NO$R_8$, =$CH_2$ or NNHCON$H_2$;

$R_3$, and $R_4$ taken together may be O, NOH, NO$R_8$, =$CH_2$ or NNHCON$H_2$;

$R_1$ and $R_4$ taken together may be a carbon-carbon bond with the proviso that $R_2$ and $R_3$ may be the same or different and each independently is selected from the group consisting of H, $C_1$-$C_7$ alkyl, $C_1$-$C_7$ substituted alkyl, -CHO, CN, -COO$R_7$, -CON($R_9$, $R_9$), -CH=NO$R_{10}$, and -CHN=NNHCON$H_2$;

$R_5$ and $R_6$ are independently H, $C_1$-$C_7$ alkyl, $C_1$-$C_7$ substituted alkyl, F, Cl, Br, OH, $N_3$, -O$C_1$-$C_7$ alkyl, -O$C_1$-$C_7$ substituted alkyl, -OCO$R_{15}$, SH, S($C_1$-$C_7$)alkyl, S($C_1$-$C_7$) substituted alkyl, SCO$R_{16}$, S(O)$_n$$R_{17}$, $NH_2$, N($R_{18}$,$R_{19}$), and N (acyl, $R_{20}$);

$R_7$, $R_8$, $R_9$, $R_{10}$, $R_{11}$, $R_{12}$, $R_{13}$, $R_{14}$, $R_{15}$, $R_{16}$, $R_{17}$, $R_{18}$, $R_{19}$, and $R_{20}$ may be the same or different and each independently is H alkyl or substituted alkyl; and

n is 1 or 2;

with the proviso that $R_1$ and $R_2$ may not both be directly attached to the 10-position via a heteroatom and $R_3$ and $R_4$ may not both be directly attached to the 11-position via a heteroatom ;

and with the still further proviso that the total number of carbon atoms for those $R_1$, $R_2$, $R_3$, and $R_4$ which are $C_1$-$C_7$ alkyl may not exceed 14;

and with the still further proviso that when X and Y are both O, then $R_1$, $R_2$, $R_3$, $R_4$, $R_6$, and $R_6$ cannot all be H.

2. A compound according to claim 1, wherein X and Y are both O.

3. A compound according to claim 1, wherein one of $R_1$, $R_2$, $R_3$ or $R_4$ is selected from the group consisting of OH, $N_3$, O$C_1$-$C_7$ alkyl, O$C_1$-$C_7$ substituted alkyl, OCO$R_9$, SH, S($C_1$-$C_7$)alkyl, S(O)$_n$$R_{11}$, $NH_2$, N($R_{12}$,$R_{13}$), and N (acyl, $R_{14}$).

4. A compound according to claim 1, wherein $R_1$ is $C_1$-$C_3$ alkyl substituted with OH, and $R_2$ is H; or wherein $R_2$ is $C_1$-$C_3$ alkyl substituted with OH, and $R_1$ is H; and $R_3$, $R_4$, $R_5$, and $R_6$ are H.

5. A compound according to claim 1, selected from the group consisting of

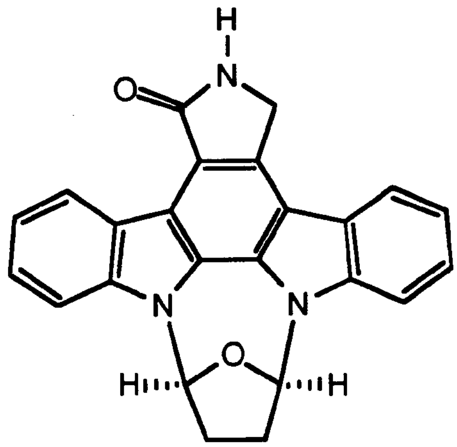

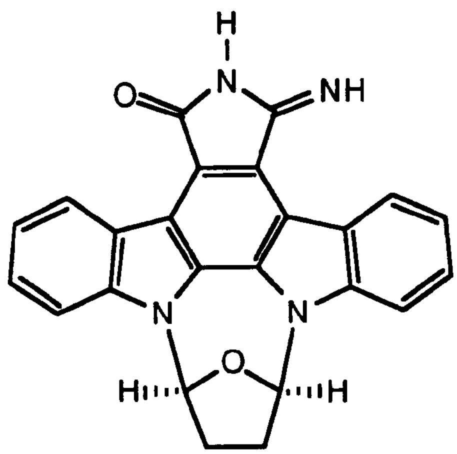

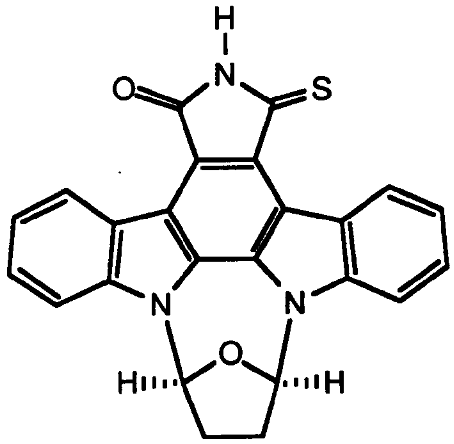

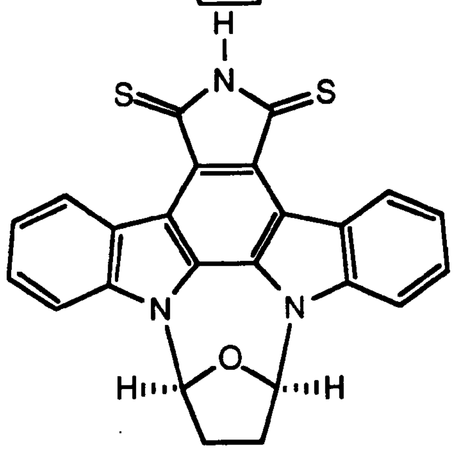

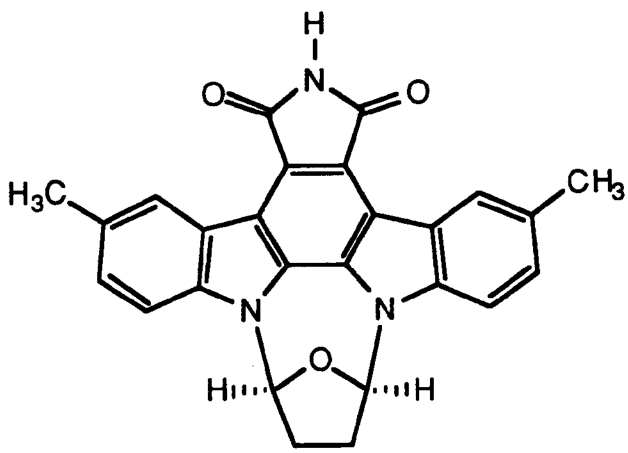

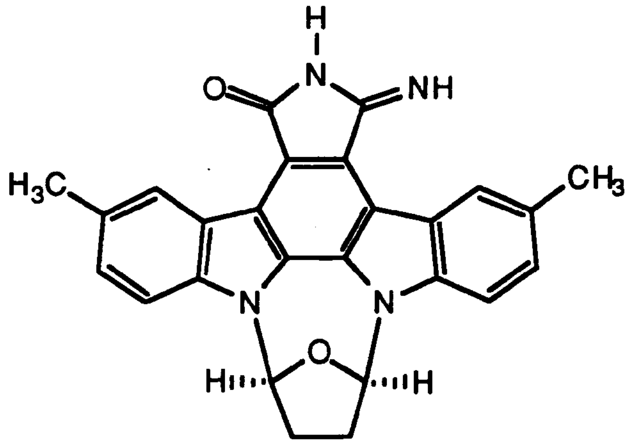

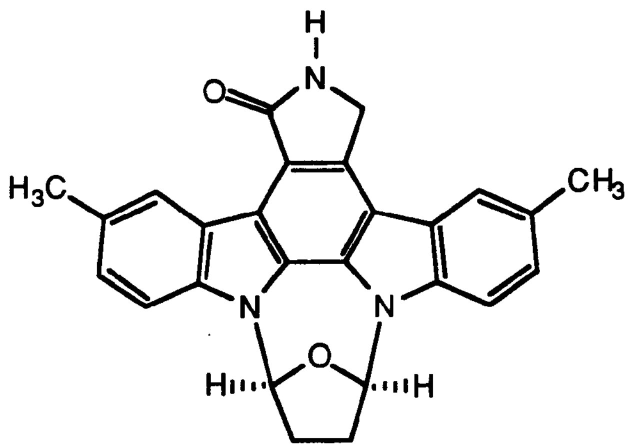

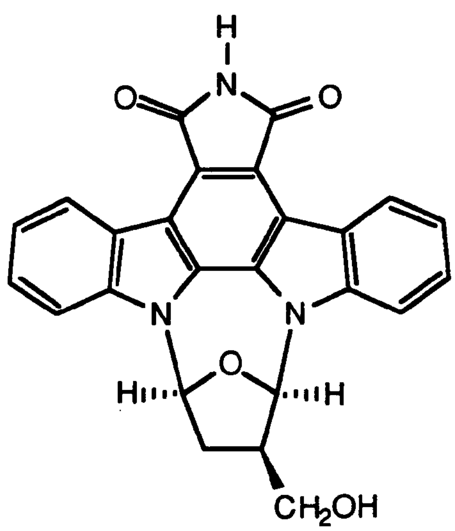

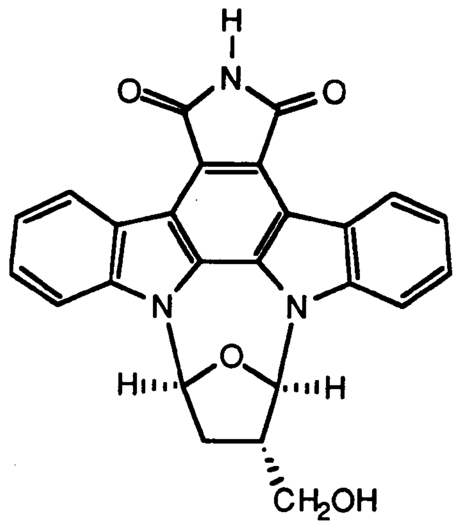

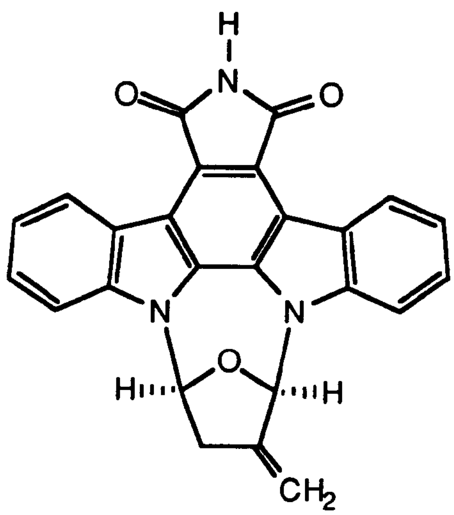

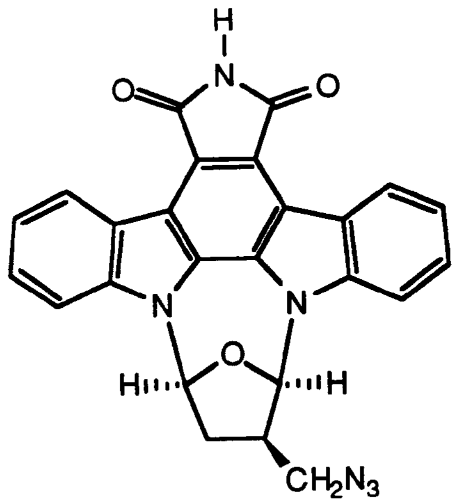

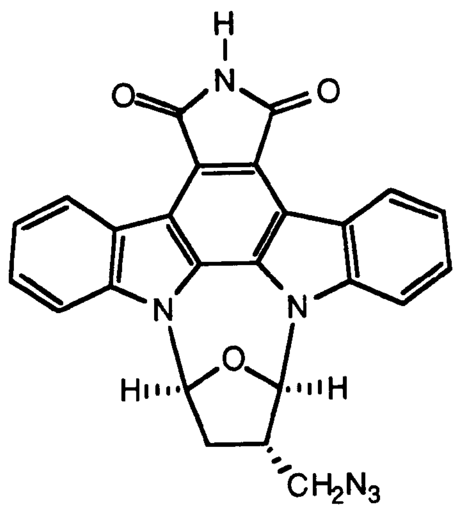

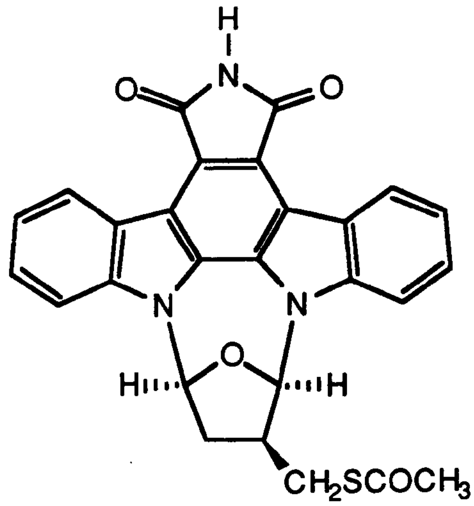

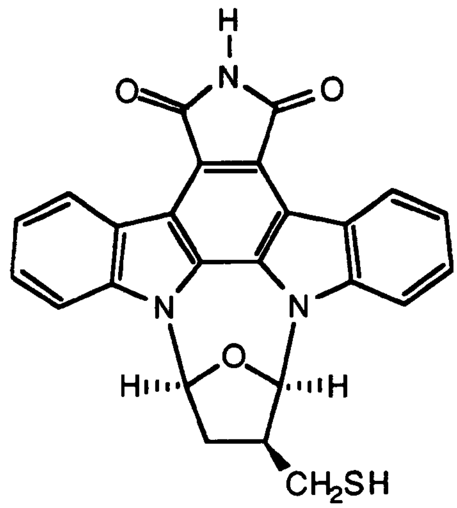

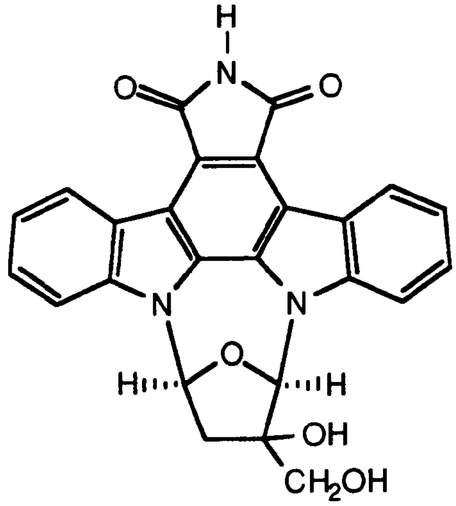

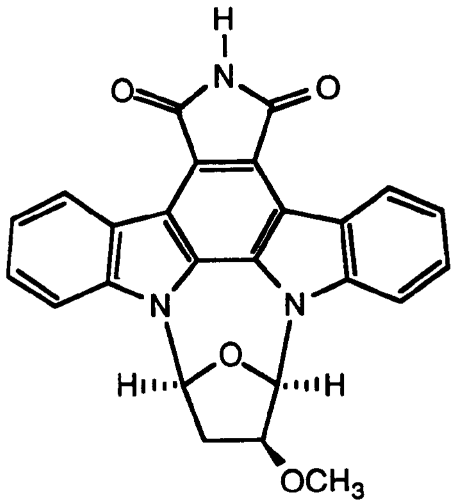

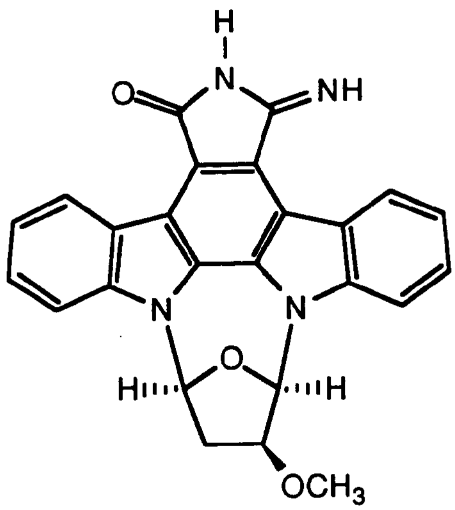

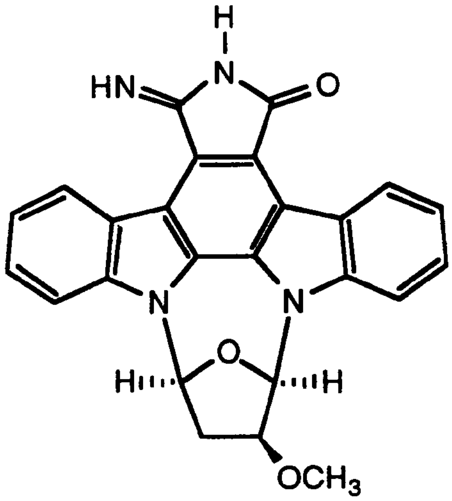

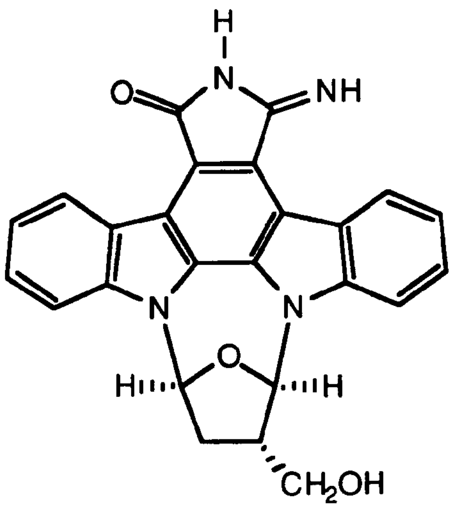

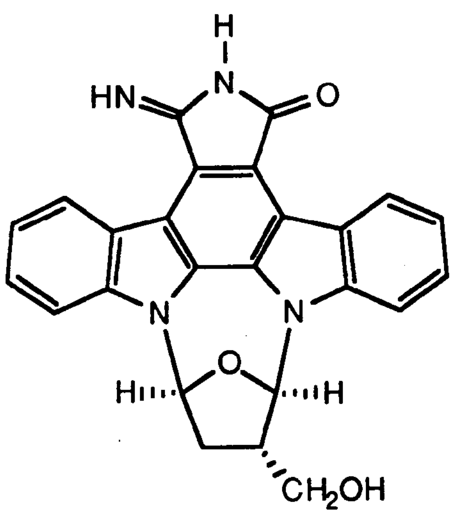

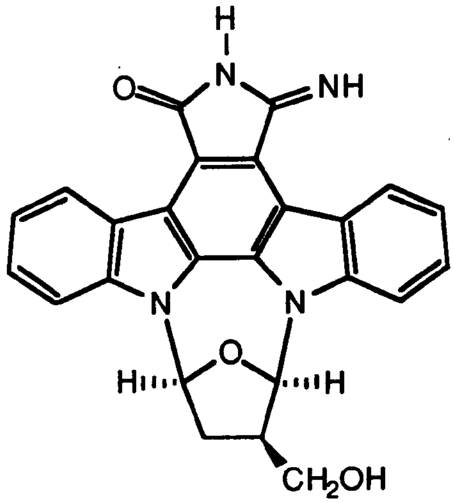

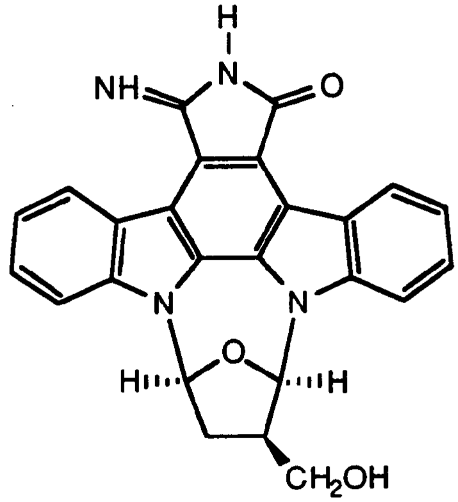

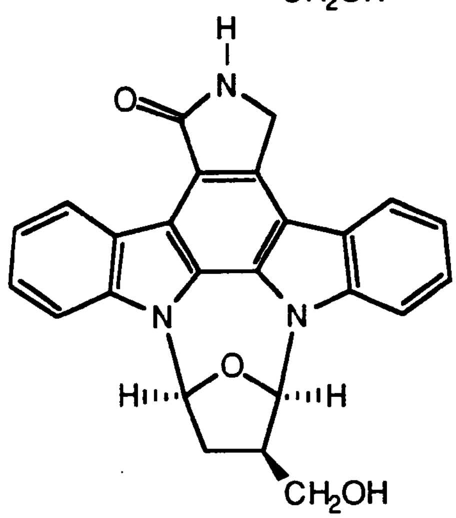

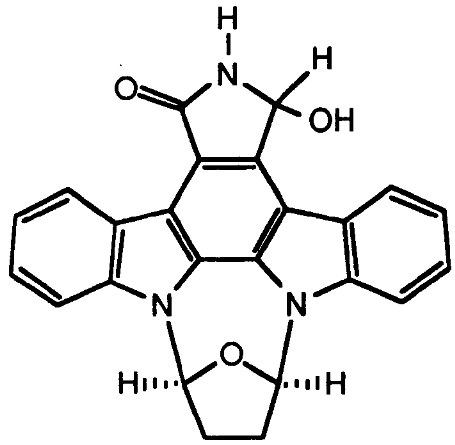

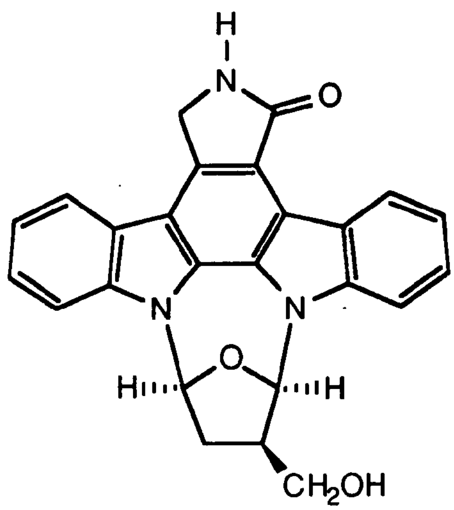

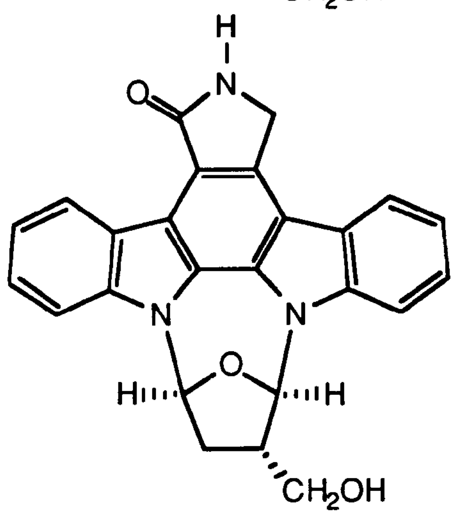

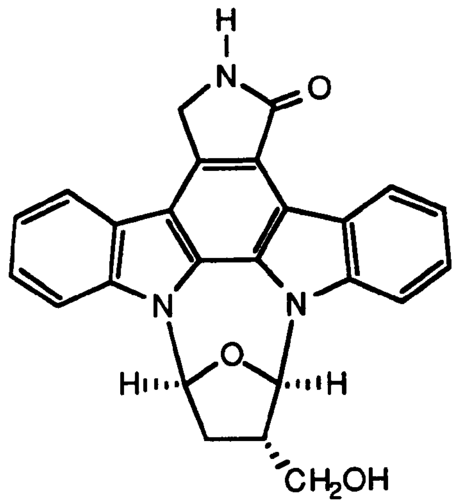

.

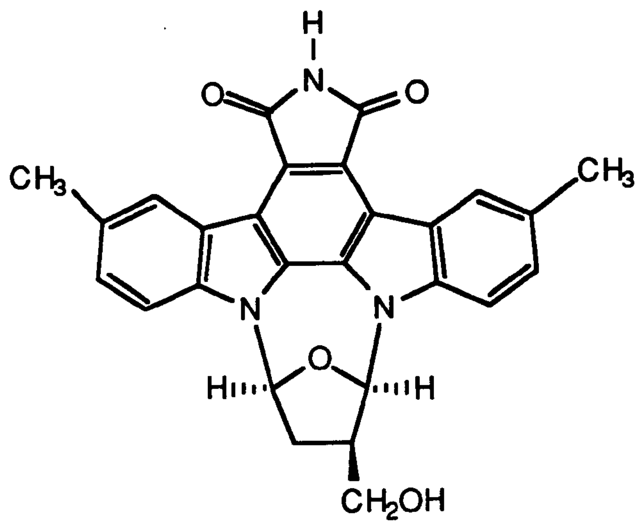

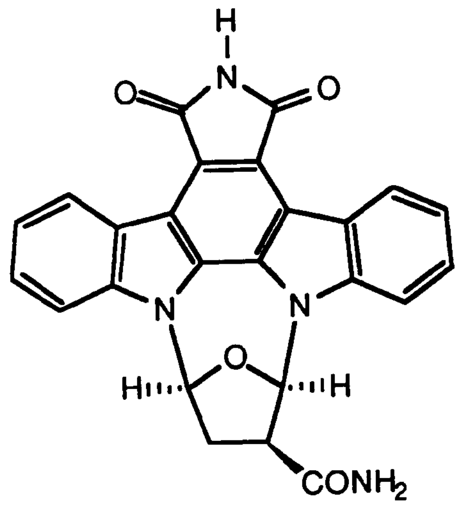

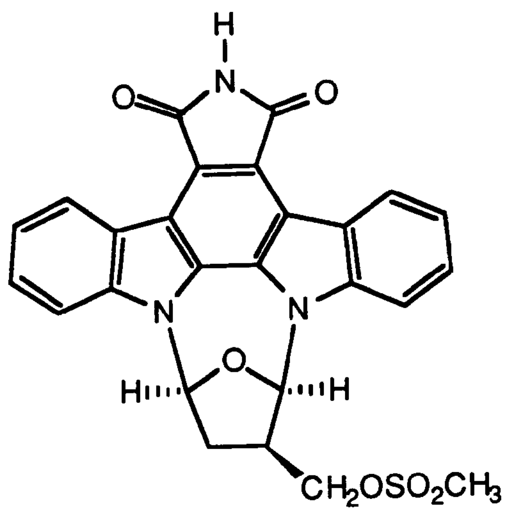

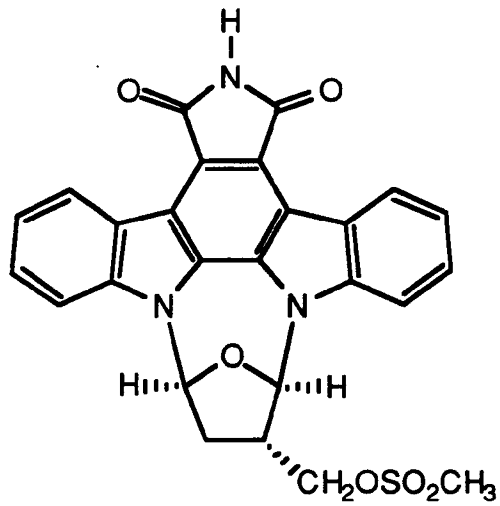

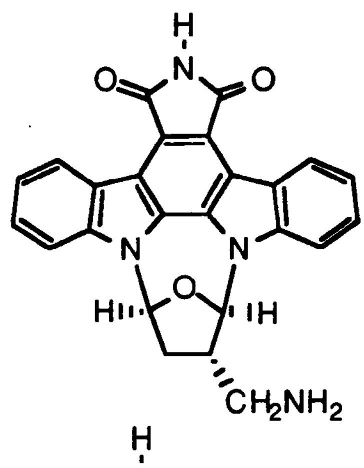

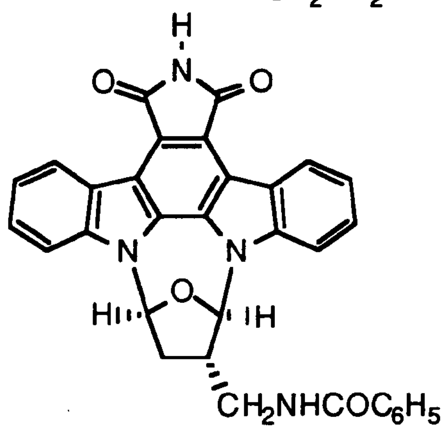

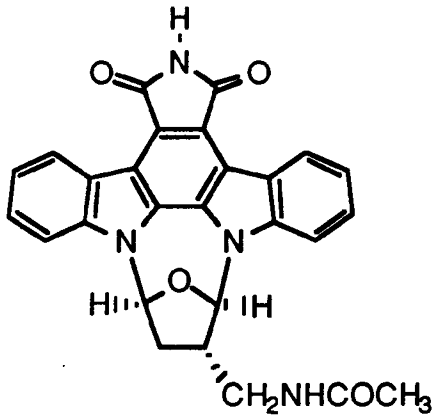

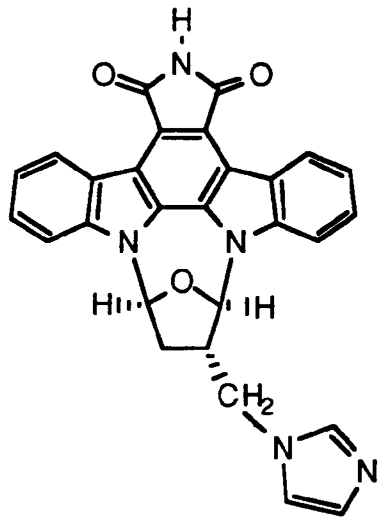

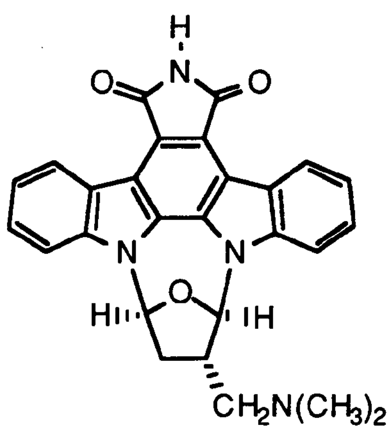

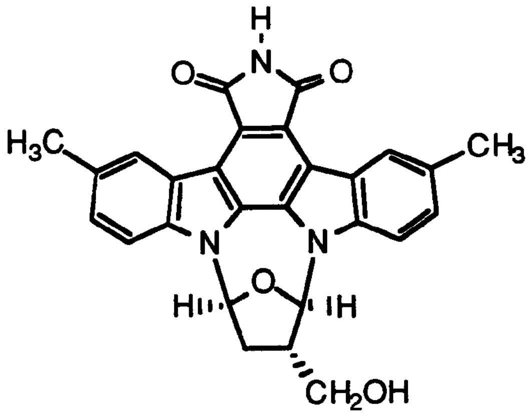

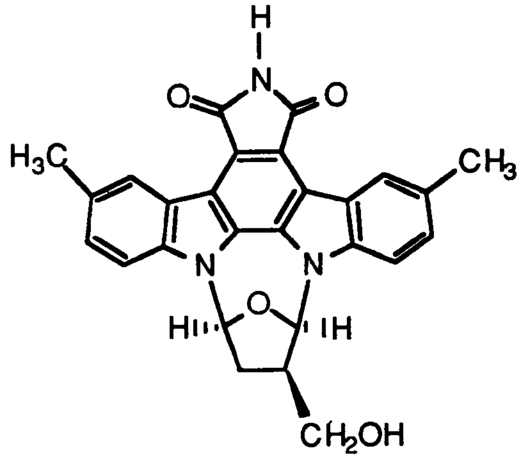

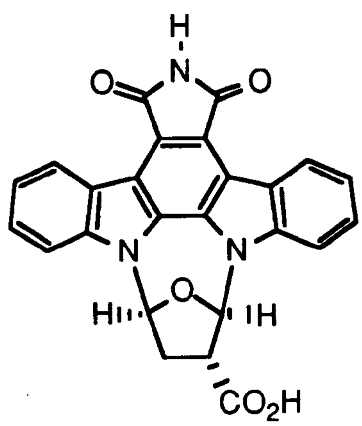

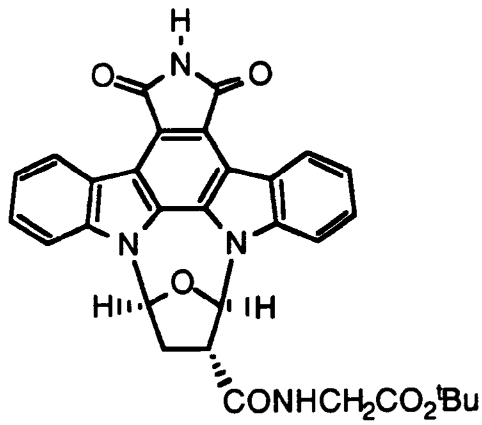

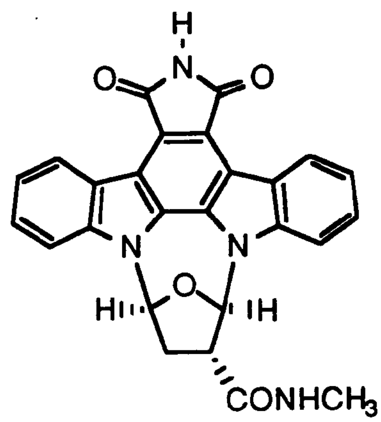

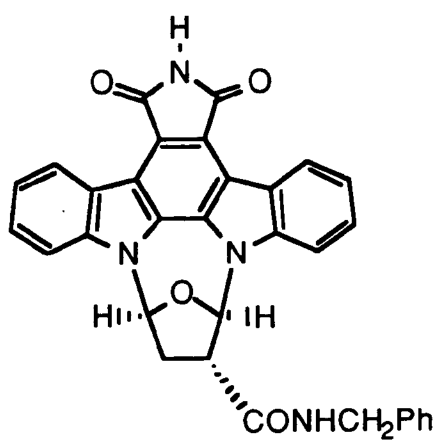

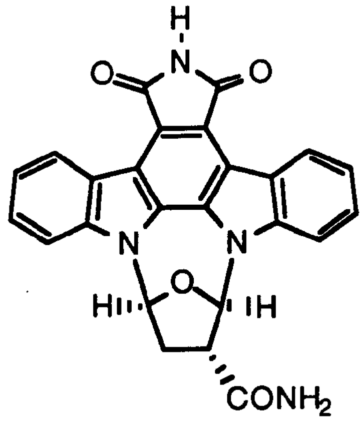

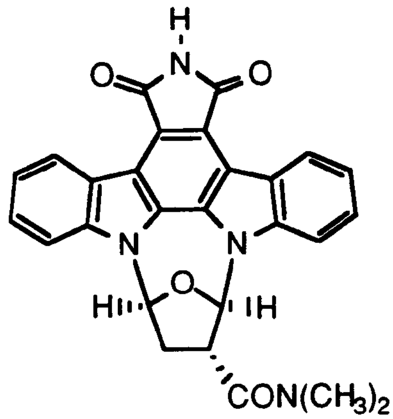

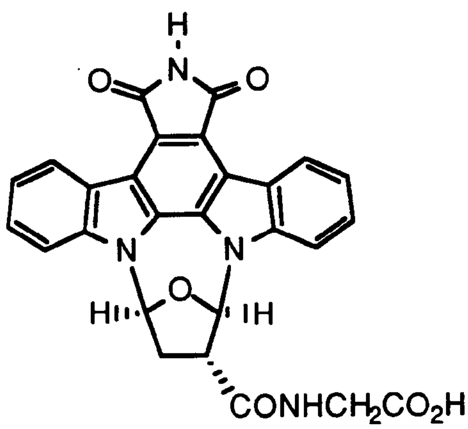

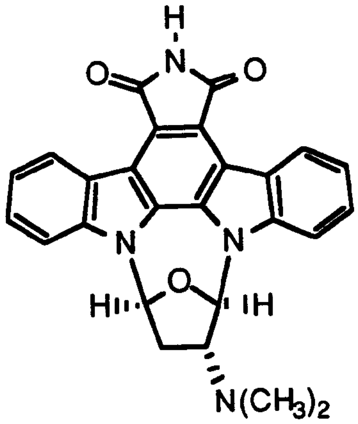

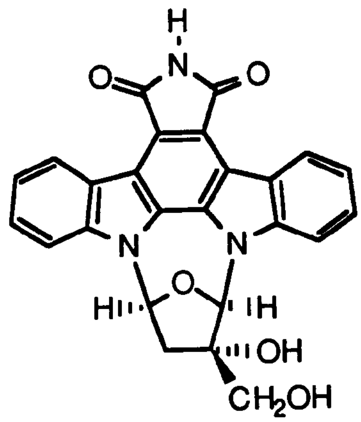

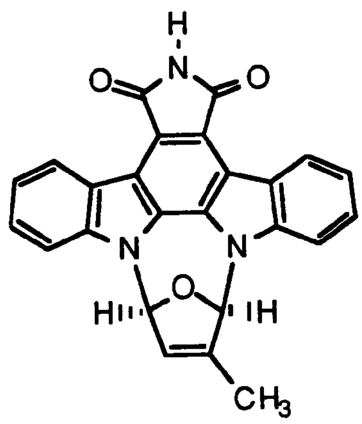

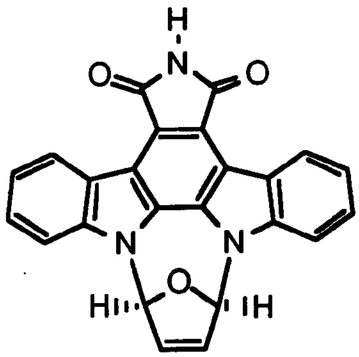

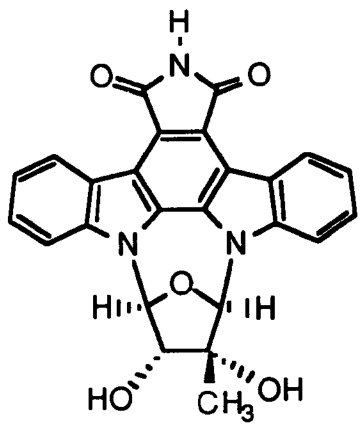

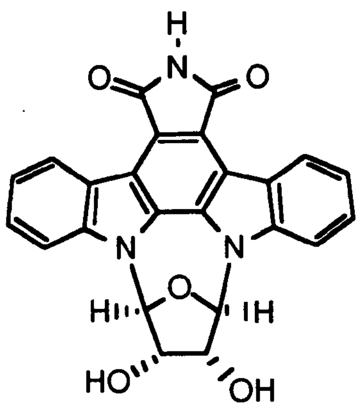

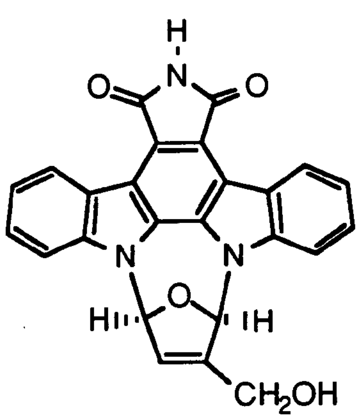

and

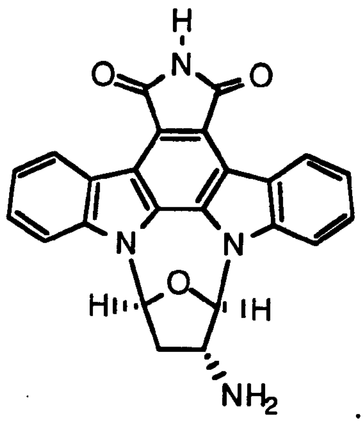

or a pharmaceutically acceptable salt thereof.

6. The compound according to claim 1

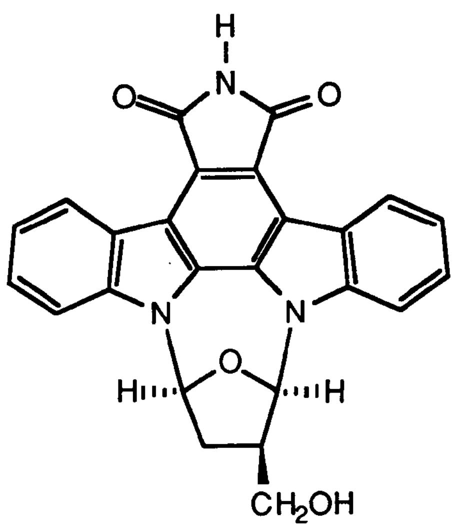

or a pharmaceutically acceptable salt thereof.

7. A pharmaceutical composition comprising a therapeutically effective amount of a compound as defined in claim 1 in combination with a pharmaceutically acceptable carrier.

8. A method for treating tumors in a mammal which comprises administering to a mammal in need of such treatment an anti-tumor effective amount of a compound according to claim 1.

9. A method for treating psoriasis in a mammal which comprises administering to a mammal in need of such treatment an anti-psoriatic effective amount of a compound according to claim 1.

10. A method for preparing a compound of formula I according to claim 1 which comprises carrying out the processes of Formula Schemes 2, 3, 4, 5, 6, or 7.

European Patent Office

# PARTIAL EUROPEAN SEARCH REPORT

which under Rule 45 of the European Patent Convention shall be considered, for the purposes of subsequent proceedings, as the European search report

Application Number

EP 92 30 3187

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 410 389 (GÖDECKE)<br>* Claims 1,4,5 *<br>--- | 1,7,8 | C 07 D 498/22<br>C 07 H 19/04<br>A 61 K 31/55 //<br>(C 07 D 498/22<br>C 07 D 307:00<br>C 07 D 273:00<br>C 07 D 209:00<br>C 07 D 209:00<br>C 07 D 209:00 ) |
| A | EP-A-0 303 697 (KYOWA HAKKO KOGYO)<br>* Claims 1-4 *<br>----- | 1,7 | |

TECHNICAL FIELDS SEARCHED (Int. Cl.5)

C 07 D
C 07 H
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claims 8,9 are directed to a method of treatment of (diagnostic method practised on) the human/animal body (Art. 52(4) EPC) the search has been carried out and based on the alleged effects of the compound/ composition.

| Place of search | Date of completion of the search | Examiner |
|---|---|---|
| THE HAGUE | 01-07-1992 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)